(19) 

Europäisches
Patentamt
European
Patent Office
Office européen
des brevets

(11) **EP 2 468 771 A1**

(12) **EUROPEAN PATENT APPLICATION**
published in accordance with Art. 153(4) EPC

(43) Date of publication:
**27.06.2012 Bulletin 2012/26**

(21) Application number: **10809728.8**

(22) Date of filing: **17.08.2010**

(51) Int Cl.:
*C07K 16/22* (2006.01)          *A61K 39/395* (2006.01)
*A61P 35/00* (2006.01)          *C12Q 1/02* (2006.01)

(86) International application number:
**PCT/JP2010/005074**

(87) International publication number:
**WO 2011/021381 (24.02.2011 Gazette 2011/08)**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **17.08.2009  PCT/JP2009/003915**

(71) Applicant: **Forerunner Pharma Research Co., Ltd.
Meguro-ku
Tokyo 153-0041 (JP)**

(72) Inventor: **KIMURA, Naoki
Tokyo 153-0041 (JP)**

(74) Representative: **Woods, Geoffrey Corlett et al
J A Kemp
14 South Square
Gray's Inn
London WC1R 5JJ (GB)**

(54) **PHARMACEUTICAL COMPOSITION CONTAINING ANTI-HB-EGF ANTIBODY AS ACTIVE INGREDIENT**

(57)     An anti-HB-EGF antibody having an internalizing activity is disclosed. A cytotoxic substance is preferably bound to the anti-HB-EGF antibody of the present invention. Also provided are an anti-cancer agent and a cell proliferation inhibitor, which comprise the antibody of the present invention as an active ingredient, a method of treating cancer and a method of diagnosing cancer, which comprise the administration of the antibody of the present invention. Cancers that can be treated by the anti-cancer agent of the present invention include pancreatic cancer, liver cancer, esophageal cancer, melanoma, colorectal cancer, gastric cancer, ovarian cancer, uterine cervical cancer, breast cancer, bladder cancer, brain tumors, and hematological cancers.

[Fig. 4]

**Description**

CROSS-REFERENCE TO RELATED APPLICATION

This application claims priority from International Application No. PCT/JP2009/003915 (filed on August 17, 2009), the contents of which are incorporated herein by reference.

TECHNICAL FIELD

The present invention relates to a method of treating cancer and to an anti-cancer agent.

BACKGROUND

Heparin-binding epidermal growth factor-like growth factor, or HB-EGF, is a growth factor belonging to the EGF ligand family. HB-EGF gene-null knockout mice exhibit very detrimental phenotypes, such as cardiac function failure accompanied by cardiohypertrophy, and quickly die after birth (Nonpatent Reference 1). This shows that HB-EGF makes a profound contribution to the formation of the heart during gestation. In the adult, on the other hand, its expression is distributed across a relatively broad range of tissues, e.g., the lung, heart, brain, and skeletal muscle (Nonpatent Reference 2), and HB-EGF has a very important role not just during gestation, but also in maintaining biological function in the adult (Nonpatent Reference 3).

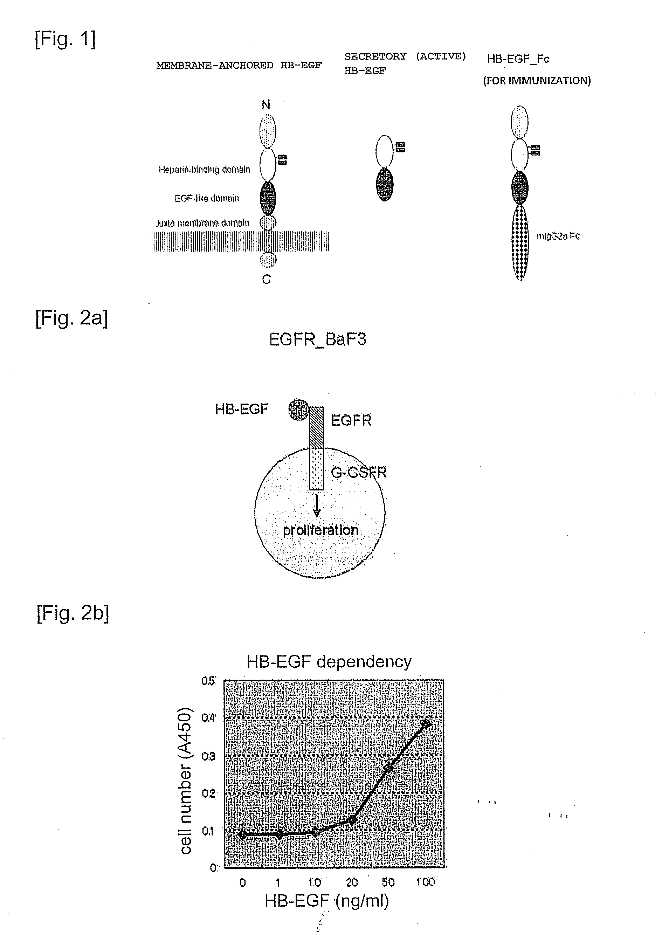

HB-EGF occurs as two different structures *in vivo*: a membrane-bound HB-EGF that is expressed on the cell surface of HB-EGF-expressing cells (designated below as proHB-EGF) and a secreted-form that occurs free from the cell (designated below as sHB-EGF or active-form HB-EGF). The structures of proHB-EGF and sHB-EGF are shown schematically in Figure 1. The proHB-EGF precursor protein is composed of 208 amino acids and is composed, considered from the N-terminal, of a signal peptide, propeptide, heparin-binding domain, EGF-like domain, juxtamembrane domain, transmembrane domain, and cytoplasmic domain. Cleavage of the signal peptide from the proHB-EGF precursor protein results in the expression of proHB-EGF as a type 1 transmembrane protein. Subsequently, proHB-EGF is subjected to protease digestion, known as ectodomain shedding, and sHB-EGF, composed of 73 to 87 amino acid residues, is released into the extracellular environment. This sHB-EGF is composed of just two domains, the heparin-binding domain and the EGF-like domain, and binds as an active ligand to the EGF receptor (Her1) and EGF receptor 4 (Her4). This results in the induction of proliferation, via the downstream ERK/MAPK signaling pathway, in a variety of cells, e.g., NIH3T3 cells, smooth muscle cells, epithelial cells, keratinocytes, renal tubule cells, and so forth (Nonpatent Reference 4). A substantial reduction in proliferation ability occurs with cells that express only proHB-EGF due to the introduction of mutation into the region that participates in ectodomain shedding. In addition, transgenic mice that express only proHB-EGF have the same phenotype as HB-EGF knockout mice. Based on these observations, the function of HB-EGF as a growth factor is thought to be borne mainly by the secreted form of HB-EGF (Nonpatent References 5 and 6).

proHB-EGF, on the other hand, is also known to have a unique function *in vivo* different from that of sHB-EGF. That is, proHB-EGF was initially known to function as a receptor for the diphtheria toxin (DT) (Nonpatent References 7 and 8). However, subsequent research demonstrated that proHB-EGF forms complexes at the cell surface with molecules such as DRAP27/CD9 and also integrin $\alpha_3\beta_1$ and heparin sulfate and participates in cell adhesion and migration. Operating through the EGF receptor (designated hereafter as EGFR) via a juxtacrine mechanism, proHB-EGF has also been shown to inhibit the growth of neighboring cells and to induce neighboring cell death. Thus, with regard to HB-EGF in its role as a ligand for EGFR, the membrane-bound proHB-EGF and secreted-form sHB-EGF are known to transmit diametrically opposite signals (Nonpatent References 5 and 8).

HB-EGF has a strong promoting activity on cell proliferation, cell movement, and infiltration in a variety of cell lines, for example, cancer cells. In addition, an increase in HB-EGF expression over that in normal tissue has been reported for a broad range of cancer types (e.g., pancreatic cancer, liver cancer, esophageal cancer, melanoma, colorectal cancer, gastric cancer, ovarian cancer, uterine cervical cancer, breast cancer, bladder cancer, and brain tumors), suggesting that HB-EGF is strongly implicated in cancer proliferation or malignant transformation (Nonpatent References 4 and 10).

Based on these findings, the inhibition of cancer cell growth via an inhibition of HB-EGF activity has therefore been pursued. The following effects, inter alia, have been reported for efforts to inhibit the action of HB-EGF using anti-HB-EGF neutralizing antibodies: an inhibition of DNA synthesis in 3T3 cells (Nonpatent Reference 11), an inhibition of keratinocyte growth (Nonpatent Reference 12), an inhibition of glioma cell growth (Nonpatent Reference 13), and an inhibition of DNA synthesis in myeloma cells (Nonpatent Reference 14).

Meanwhile the use of an attenuated diphtheria toxin (CRM197) that specifically binds to HB-EGF as an HB-EGF inhibitor has also been pursued. In fact, in a test of the efficacy in a mouse xenograft model (transplantation of an ovarian cancer cell line), the group receiving CRM197 presented a superior tumor shrinkage effect (Nonpatent Reference 15). In addition, clinical testing with CRM197 has also been carried out in cancer patients (Nonpatent Reference 16). Cancer treatments using anti-HB-EGF is disclosed in WO 2008/047914 and WO 2008/047925.

The references cited in this specification is listed below. The contents of these documents are herein incorporated by reference in their entirety. None of these documents is admitted as prior art to the present invention:

Patent Reference 1: WO 2008/047914
Patent Reference 2: WO 2008/047925

Nonpatent Reference 1: Iwamoto R, Yamazaki S, Asakura M et al., Heparin-binding EGF-like growth factor and ErbB signaling is essential for heart function. Proc. Natl. Acad. Sci. USA, 2003; 100:3221-6.
Nonpatent Reference 2: Abraham JA, Damm D, Bajardi A, Miller J, Klagsbrun M, Ezekowitz RA. Heparin-binding EGF-like growth factor: characterization of rat and mouse cDNA clones, protein domain conservation across species, and transcript expression in tissues. Biochem Biophys Res Commun, 1993; 190:125-33.
Nonpatent Reference 3: Karen M., Frontiers in Bioscience, 3, 288-299, 1998.
Nonpatent Reference 4: Raab G, Klagsbrun M. Heparin-binding EGF-like growth factor. Biochim Biophys Acta, 1997; 1333:F179-99.
Nonpatent Reference 5: Yamazaki S, Iwamoto R, Saeki K et al. Mice with defects in HB-EGF ectodomain shedding show severe developmental abnormalities. J Cell Biol, 2003; 163:469-75.
Nonpatent Reference 6: Ongusaha P., Cancer Res, (2004) 64, 5283-5290.
Nonpatent Reference 7: Iwamoto R., Higashiyama S., EMBO J. 13, 2322-2330 (1994).
Nonpatent Reference 8: Naglich JG., Metherall JE., Cell, 69, 1051-1061 (1992).
Nonpatent Reference 9: Iwamoto R, Handa K, Mekada E. Contact-dependent growth inhibition and apoptosis of epidermal growth factor (EGF) receptor-expressing cells by the membrane-anchored form of heparin-binding EGF-like growth factor. J. Biol. Chem. 1999; 274:25906-12.
Nonpatent Reference 10: Miyamoto S, Cancer Sci. 97, 341-347 (2006).
Nonpatent Reference 11: Blotnick S., Proc. Natl. Acad. Sci. USA, (1994) 91, 2890-2894.
Nonpatent Reference 12: Hashimoto K., J. Biol. Chem. (1994) 269, 20060-20066.
Nonpatent Reference 13: Mishima K., Act Neuropathol. (1998) 96, 322-328.
Nonpatent Reference 14: Wang YD. Oncogene, (2002) 21, 2584-2592.
Nonpatent Reference 15: Miyamoto S., Cancer Res. (2004) 64, 5720.
Nonpatent Reference 16: Buzzi S., Cancer Immunol Immunother, (2004) 53, 1041-1048.

DISCLOSURE OF THE INVENTION

An object of the invention is to provide an anti-HB-EGF antibody which is particularly useful as a medicament. More specifically, the object of the invention is to provide an anti-HB-EGF antibody which binds to a specific epitope of HB-EGF, a novel method for treating cancer with the use of the antibody, a novel cell proliferation inhibitor and an anticancer drug comprising the antibody.

The inventors have discovered that an anti-HB-EGF antibody which recognizes a specific epitope is particularly useful as a medicament. Based on the findings, the present inventors also discovered that anti-HB-EGF antibody is effective for the treatment of cancers in which HB-EGF expression is upregulated, most prominently ovarian cancer, and thereby achieved the present invention.

When the present inventors produced monoclonal antibody by immunizing mice with HB-EGF protein, they found that the obtained antibody had an internalizing activity. In addition, when a cytotoxic substance was bound to the obtained internalizing anti-HB-EGF antibody and the cell death-inducing activity was measured, a significant cell death-inducing activity was noted. Furthermore, when the ADCC activity and CDC activity of the obtained anti-HB-EGF antibody were measured, the anti-HB-EGF antibody was found to exhibit ADCC activity and/or CDC activity.

Thus, the present application provides a monoclonal antibody and a lower molecular weight antibody derivative selected from the following (1) to (19) as well as applications thereof:

(1) An anti-HB-EGF antibody selected from [1] and [2]:

[1] an antibody comprising
a heavy chain comprising the amino acid sequence of SEQ ID NO:1 as CDR1, the amino acid sequence of SEQ ID NO:2 as CDR2, and the amino acid sequence of SEQ ID NO:3 as CDR3, and

a light chain comprising the amino acid sequence of SEQ ID NO:4 as CDR1, the amino acid sequence of SEQ ID NO:5 as CDR2, and the amino acid sequence of SEQ ID NO:6 as CDR3; and

[2] an antibody which binds to the same epitope as an epitope to which the antibody set forth in [1] binds.

(2) The anti-HB-EGF antibody according to (1) having an internalizing activity.

(3) The anti-HB-EGF antibody according to (1) or (2) to which a cytotoxic substance is attached.

(4) The anti-HB-EGF antibody according to (1) or (2) having an ADCC activity or a CDC activity.

(5) The anti-HB-EGF antibody according to any one of (1) to (4), further having a neutralizing activity.

(6) The anti-HB-EGF antibody according to any one of (1) to (5), which comprises a marker attached.

(7) A pharmaceutical composition comprising the antibody according to any one of (1) to (6).

(8) A pharmaceutical composition comprising a cytotoxic substance attached to the antibody according to any one of (1) to (6).

(9) The pharmaceutical composition according to (7) or (8), which is a cell proliferation inhibitor.

(10) The pharmaceutical composition according to (9), which is an anti-cancer agent.

(11) The pharmaceutical composition according to (10), wherein the cancer is pancreatic cancer, liver cancer, esophageal cancer, melanoma, colorectal cancer, gastric cancer, ovarian cancer, uterine cervical cancer, breast cancer, bladder cancer, a brain tumor, or a hematological cancer.

(12) A method of delivering a cytotoxic substance into a cell by means of the anti-HB-EGF antibody according to any one of (1) to (6).

(13) A method of inhibiting cell proliferation with a cytotoxic substance attached to the anti-HB-EGF antibody according to any one of (1) to (6).

(14) The method according to (13), wherein the cell is a cancer cell.

(15) The method according to any one of (12) to (14), wherein the cytotoxic substance is a chemotherapeutic agent, a radioactive substance, or a toxic peptide.

(16) Use of the anti-HB-EGF antibody according to any one of (1) to (6) for transporting a cytotoxic substance into a cell.

(17) Use of the anti-HB-EGF antibody according to any one of (2) to (6) having an internalizing activity for inhibiting cell proliferation.

(18) The use according to (16) or (17), wherein the cell is a cancer cell.

(19) The use according to any one of (16) to (18), wherein a cytotoxic substance is attached to the anti-HB-EGF antibody.

(20) A method of producing a pharmaceutical composition comprising the steps of:

(a) providing the anti-HB-EGF antibody according to any one of (1) to (6);

(b) determining whether the antibody of (a) has an internalizing activity;

(c) selecting an antibody that has an internalizing activity; and

(d) attaching a cytotoxic substance to the antibody selected in (c).

(21) The method according to (20), wherein the pharmaceutical composition is an anti-cancer agent.

(22) A method of diagnosing cancer using an anti-HB-EGF antibody according to any one of (1) to (6).

(23) The method according to (22), comprising using an anti-HB-EGF antibody to which a labeling substance is attached.

(24) The method according to (22) or (23), wherein an intracellularly incorporated anti-HB-EGF antibody is detected.

BRIEF DESCRIPTION OF THE DRAWINGS

Figure 1 is a diagram that schematically depicts the structure of proHB-EGF, sHB-EGF, and the HB-EGF_Fc used as immunogen;

Figure 2a is a diagram that schematically depicts the influence of the binding of HB-EGF to the EGFR_Ba/F3 cell;

Figure 2b is a graph that shows the dependence of EGFR_Ba/F3 cell proliferation on the HB-EGF concentration;

Figure 3 is a graph that shows the neutralizing activity of HC-15 antibody on the HB-EGF-dependent growth of EGFR_Ba/F3 cells.

Figure 4 shows the sequences of the variable regions of the HC-15 antibody.

Figure 5 is a graph that shows the binding activity of antibodies HA-20, HB-20, and HC-15 to active-form HB-EGF;

Figure 6 shows histograms that show the binding activity of antibodies HA-20, HB-20, and HC-15 to proHB-EGF;

Figure 7 is a schematic illustration showing the inhibition of binding between HB-EGF and EGFR by HB-EGF antibody on a solid phase;

Figure 8 is a schematic illustration showing an ELISA-based analysis model for the EGFR/HB-EGF binding mode;

Figure 9 is a graph that shows the concentration curve for HB-EGF detected in the ELISA-based analysis model for the EGFR/HB-EGF binding mode;

Figure 10 is a graph that shows the inhibition of binding of HB-EGF to EGFR by antibodies HA-20, HB-20, and HC-15;

Figure 11 is a graph that compares the inhibition of the growth of EGFR_Ba/F3 cells by antibodies HA-20, HB-20, and HC-15;

Figure 12a is a graph that shows the inhibition of growth of the ovarian cancer cell line RMG-1 by the antibodies HA-20, HB-20, and HC-15 in a medium containing 8% FCS;

Figure 12b is a graph that shows the inhibition of growth of the ovarian cancer cell line RMG-1 by the antibodies HA-20, HB-20, and HC-15 in a medium containing 2% FCS;

Figure 13 is a schematic diagram of the process in which cell death is induced by the internalization into a cell of an antibody bound to antigen (complex of HB-EGF targeting antibody and saporin-labeled antibody);

Figure 14 is a graph that shows the internalization-mediated activity of the HA-20, HB-20, and HC-15 antibodies to induce cell death in SKOV-3 cells (original cell line) and HB-EGF_SKOV3 cells (high HB-EGF-expressing SKOV-3 cells);

Figure 15 is a histograms that show the binding activity of the HA-20, HB-20, and HC-15 antibodies for the HB-EGF on ES-2 cells;

Figure 16 is a graph that shows the internalization-mediated cell death inducing activity of HA-20, HB-20, and HC-15, on ES-2 ovarian cancer cells;

Figure 17 shows histograms of the FACS analysis of the expression of HB-EGF by ovarian cancer lines (RMG-1, MCAS);

Figure 18 is a diagram of the analysis of the neutralizing activity of the antibodies HA-20 and HC-15, to inhibit proliferation of RMG-1 cells in the soft agar colony formation assay;

Figure 19 is a diagram of the analysis of the internalization-mediated proliferation inhibiting activity of the antibodies HA-20 and HC-15 on RMG-1 ovarian cancer cells in the soft agar colony formation assay;

Figure 20 is a diagram of the analysis of the internalization-mediated proliferation inhibiting activity of the antibodies HA-20 and HC-15 on MCAS ovarian cancer cells in the soft agar colony formation assay;

Figure 21 shows histograms of the FACS analysis of the expression of HB-EGF by several hematological cancer cell lines;

Figure 22 shows graphs that show the internalization-mediated inhibition of proliferation by the HA-20 and HC-15 antibodies on several hematological cancer cell lines;

Figure 23a is a diagram of the analysis of the proliferation inhibiting activity exhibited by saporin-labeled HA-20 antibody (HA-SAP), saporin-labeled HC-15 antibody (HC-SAP), and saporin-labeled control antibody (IgG-SAP) on various solid cancer cell lines and normal human endothelial cells;

Figure 23b is a diagram of the analysis of the proliferation inhibiting activity exhibited by saporin-labeled HA-20 antibody (HA-SAP) and saporin-labeled HC-15 antibody (HC-SAP) on various hematological cancer cell lines;

Figure 24a is a graph of the FACS analysis of the binding activity exhibited by various anti-HB-EGF antibodies for HB-EGF overexpressed in Ba/F3 cells; the fluorescence intensity is expressed on the vertical axis as the G-mean value; and

Figure 24b shows the ADCC activity exhibited by various anti-HB-EGF antibodies on HB-EGF_Ba/F3 cells (upper) and the CDC activity exhibited by various anti-HB-EGF antibodies on HB-EGF_Ba/F3 cells (lower); the vertical axis shows the amount of chromium released from the cells due to ADCC-mediated or CDC-mediated cytotoxicity.

Figure 25 shows the results of ELISA analyses on the binding activities of chHC15 and chHE39 to secretory HB-EGF.

Figure 26 shows the results of FACS analyzing the binding activities of chHC15 and chHE39 to HB-EGF expressed on the cell membrane.

Figure 27a shows the results of analyzing the tumor inhibiting activity of chHE39 in vivo; the tumor volume was measured continuously over time in mice transplanted with an HB-EGF-expressing SKOV-3 cell line followed by administration of the antibody once per week.

Figure 27b shows the results of analyzing the tumor inhibiting activity of chHC15 in vivo; the tumor volume was measured continuously over time in mice transplanted with an HB-EGF-expressing SKOV-3 cell line followed by administration of the antibody once per week.

Figure 27c shows the results of analyzing the tumor inhibiting activity of chHE39 in vivo; the tumor volume was measured continuously over time in mice transplanted with an MCAS cell line followed by administration of the antibody once per week.

Figure 27d shows the results of analyzing the tumor inhibiting activity of chHC15 in vivo; the tumor volume was measured continuously over time in mice transplanted with an MCAS cell line followed by administration of the antibody once per week.

Figure 28a shows the results of analyzing the tumor inhibiting activity of chHC15 in an in vivo model; the tumor volume was measured over time in mice transplanted with the human pancreatic cancer cell line BxPC-3 followed by administration of the antibody once per week at a dose of 10 mg/kg.

Figure 28b shows the results of analyzing the tumor inhibiting activity of chHC15 in an in vivo model; the tumor volume was measured over time in mice transplanted with the human breast cancer cell line MDA-MB-231 (a cell line maintained in vivo) followed by administration of the antibody once per week at a dose of 10 mg/kg.

Figure 28c shows the results of analyzing the tumor inhibiting activity of chHC15 in an in vivo model; the tumor volume was measured over time in mice transplanted with the human breast cancer cell line MDA-MB-231 (a cell line maintained in vitro) followed by administration of the antibody once per week at a dose of 10 mg/kg.

## PREFERRED EMBODIMENT OF THE INVENTION

### The molecular forms of HB-EGF

HB-EGF is a growth factor that belongs to the EGF ligand family; the sequence of the gene encoding human HB-EGF is disclosed as GenBank accession number NM_001945 (SEQ ID NO: 11) and the amino acid sequence of HB-EGF is disclosed as GenBank accession number NP_001936 (SEQ ID NO: 12). Within the context of the present invention, "HB-EGF protein" is a term that encompasses both the full-length protein and fragments thereof. Within the context of the present invention, a "fragment" is a polypeptide that contains any region of the HB-EGF protein, wherein the fragment may not exhibit the functionality of the naturally occurring HB-EGF protein.

sHB-EGF, which is used herein as a specific embodiment of a fragment, is a molecule composed of 73 to 87 amino acid residues and is produced *in vivo* when the proHB-EGF expressed on the cell surface of an HB-EGF-expressing cell is subjected to protease cleavage in a process known as ectodomain shedding. Multiple sHB-EGF molecules are known; these sHB-EGF molecules have a structure in which the carboxyl terminal is the proline residue at position 149 in the proHB-EGF molecule, with the proHB-EGF molecule being composed of the 208 amino acids shown in SEQ ID NO: 12 while the amino terminal is the asparagine residue at position 63 of the proHB-EGF molecule, the arginine residue at position 73 of the proHB-EGF molecule, the valine residue at position 74 of the proHB-EGF molecule, or the serine residue at position 77 of the proHB-EGF molecule.

### The anti-HB-EGF antibody

The anti-HB-EGF antibody of the present invention is an antibody that binds to HB-EGF protein, but there are no limitations with regard to its origin (mouse, rat, human, and so forth), type (monoclonal antibody, polyclonal antibody), and configuration (engineered antibodies, low molecular weight antibodies, modified antibodies, and so forth).

The anti-HB-EGF antibody used in the present invention preferably binds specifically to HB-EGF. The anti-HB-EGF antibody used in the present invention is also preferably a monoclonal antibody.

Antibody that has an internalizing activity is a preferred embodiment of the antibody used in the present invention. The "antibody that has an internalizing activity" denotes antibody that is transported into the cell (into the cytoplasm, vesicles, other organelles, and so forth) upon binding to the HB-EGF on the cell surface.

The presence/absence of an internalizing activity by an antibody can be determined using methods known to those skilled in the art. For example, the internalizing activity can be determined by bringing a label-conjugated anti-HB-EGF antibody into contact with HB-EGF-expressing cells and checking the presence/absence of label incorporation into the cell, or by bringing a cytotoxin-conjugated anti-HB-EGF antibody into contact with HB-EGF-expressing cells and checking whether or not cell death has been induced in the HB-EGF-expressing cells. In more specific terms, the presence/absence of an internalizing activity by the antibody can be determined, for example, by the method described in the examples provided below.

In those instances in which the anti-HB-EGF antibody has an internalizing activity, the anti-HB-EGF antibody is preferably an antibody capable of binding proHB-EGF and more preferably is an antibody that binds to proHB-EGF more strongly than to sHB-EGF.

### The cytotoxic substance

Another preferred embodiment of the antibody used in the present invention is an antibody to which a cytotoxic substance is attached. Such a cytotoxic substance-conjugated antibody may be incorporated into a cell, resulting in the cytotoxic substance-mediated induction of the death of the cell that has incorporated the antibody. Accordingly, the cytotoxic substance-conjugated antibody preferably also has an internalizing activity.

The cytotoxic substance used in the present invention may be any substance that can induce cell death in a cell and may include toxins, radioactive substances, chemotherapeutic agents, and so forth. The cytotoxic substance used in the present invention encompasses prodrugs that undergo alteration *in vivo* to an active cytotoxic substance. The prodrug activation may proceed via an enzymatic alteration or a non-enzymatic alteration.

Within the context of the present invention, toxin denotes various cytotoxic proteins polypeptides, and so forth, of microbial, animal, or plant origin. The toxins used in the present invention may include the following: diphtheria toxin A chain (Langone J.J. et al., Methods in Enzymology, 93, 307-308, 1983), pseudomonas exotoxin *(*Mature Medicine, 2, 350-353, 1996), ricin A chain (Fulton R.J. et al., J. Biol. Chem., 261, 5314-5319, 1986; Sivam G., et al., Cancer Res., 47, 3169-3173, 1987; Cumber A.J. et al., J. Immunol. Methods, 135, 15-24, 1990; Wawrzynczak E.J. et al., Cancer Res., 50, 7519-7562, 1990; Gheeite V. et al., J. Immunol. Methods, 142, 223-230, 1991), deglycosylated ricin A chain (Thorpe P.E. et al., Cancer Res., 47, 5924-5931, 1987), abrin A chain (Wawrzynczak E.J. et al., Br. J. Cancer, 66, 361-366, 1992; Wawrzynczak E.J. et al., Cancer Res., 50, 7519-7562, 1990; Sivam G. et al., Cancer Res., 47, 3169-3173, 1987; Thorpe P.E. et al., Cancer Res., 47, 5924-5931, 1987), gelonin (Sivam G. et al., Cancer Res., 47, 3169-3173, 1987; Cumber A.J. et al., J. Immunol. Methods, 135, 15-24, 1990; Wawrzynczak E.J. et al., Cancer Res., 50, 7519-7562, 1990; Bolognesi A. et al., Clin. Exp.Immunol., 89, 341-346, 1992), PAP-s (pokeweed anti-viral protein from seeds; Bolognesi A. et al., Clin. Exp. Immunol., 89, 341-346, 1992), briodin (Bolognesi A. et al., Clin. Exp. Immunol., 89, 341-346, 1992), saporin (Bolognesi A. et al., Clin. Exp. Immunol., 89, 341-346, 1992), momordin (Cumber A.J. et al., J. Immunol. Methods, 135, 15-24, 1990; Wawrzynczak E.J. et al., Cancer Res., 50, 7519-7562, 1990; Bolognesi A. et al., Clin. Exp. Immunol., 89, 341-346, 1992), momorcochin (Bolognesi A. et al., Clin. Exp. Immunol., 89, 341-346, 1992), dianthin 32 (Bolognesi A. et al., Clin. Exp. Immunol., 89, 341-346, 1992), dianthin 30 (Stirpe F., Barbieri L., FEBS Letter, 195, 1-8, 1986), modeccin (Stirpe F., Barbieri L., FEBS Letter, 195, 1-8, 1986), viscumin (Stirpe F., Barbieri L., FEBS Letter, 195, 1-8, 1986), volkesin (Stirpe F., Barbieri L., FEBS Letter, 195, 1-8, 1986), dodecandrin (Stirpe F., Barbieri L., FEBS Letter, 195, 1-8, 1986), tritin (Stirpe F., Barbieri L., FEBS Letter, 195, 1-8, 1986), luffin (Stirpe F., Barbieri L., FEBS Letter, 195, 1-8, 1986), and trichokirin (Casellas P., et al., Eur. J. Biochem., 176, 581-588, 1988; Bolognesi A. et al., Clin. Exp. Immunol., 89, 341-346, 1992).

The radioactive substance in the present invention denotes a substance that contains a radioisotope. There are no particular limitations on the radioisotope and any radioisotope may be used. Examples of usable radioisotopes are $^{32}$P, $^{14}$C, $^{125}$I, $^{3}$H, $^{131}$I, $^{186}$Re, $^{188}$Re, and so forth.

The chemotherapeutic agent in the present invention denotes a cytotoxic substance other than the toxin and radioactive substance cited above and encompasses, for example, cytokines, antitumor agents, enzymes, and for forth. The chemotherapeutic agent used in the present invention is not particularly limited, but is preferably a low molecular weight chemotherapeutic agent. At low molecular weights, the chemotherapeutic agent is believed to have a low potential for interfering with antibody function even after the chemotherapeutic agent has been bound to the antibody. Within the context of the present invention, low molecular weight chemotherapeutic agents generally denote a molecular weight of 100 to 2000 and preferably a molecular weight of 200 to 1000. There are no particular limitations in the present invention on the chemotherapeutic agent, and examples of usable chemotherapeutic agents are as follows: melphalan (Rowland G.F. et al., Nature, 255, 487-488, 1975), cis-platinum (Hurwitz E. and Haimovich J., Method in Enzymology, 178, 369-375, 1986; Schechter B. et al., Int. J. Cancer, 48, 167-172, 1991), carboplatin (Ota Y. et al., Asia-Oceania J. Obstet. Gynaecol., 19, 449-457, 1993), mitomycin C (Noguchi A. et al., Bioconjugate Chem., 3, 132-137, 1992), adriamycin (doxorubicin) (Shih L.B. et al., Cancer Res., 51, 4192-4198, 1991; Zhu Z. et al., Cancer Immunol. Immunother., 40, 257-267, 1995; Trail P.A. et al., Science, 261, 212-215, 1993; Zhu Z. et al., Cancer Immunol. Immunother., 40, 257-267, 1995; Kondo Y. et al., Jpn. J. Cancer Res., 86, 1072-1079, 1995; Zhu Z. et al., Cancer Immunol. Immunother., 40, 257-267, 1995; Zhu Z. et al., Cancer Immunol. Immunother., 40, 257-267, 1995), daunorubicin (Dillman R.O. et al., Cancer Res., 48, 6097-6102, 1988; Hudecz F. et al., Bioconjugate Chem., 1, 197-204, 1990; Tukada Y. et al., J. Natl. Cancer Inst., 75, 721-729, 1984), bleomycin (Manabe Y. et al., Biochem. Biophys. Res. Commun., 115, 1009-1014, 1983), neocarzinostatin (Kitamura K. et al., Cancer Immunol. Immunother., 36, 177-184, 1993; Yamaguchi T. et al., Jpn. J. Cancer Res., 85, 167-171, 1994), methotrexate (Kralovec J. et al., Cancer Immunol. Immunother., 29, 293-302, 1989; Kulkarni P.N. et al., Cancer Res., 41, 2700-2706, 1981; Shin L.B. et al., Int. J. Cancer, 41, 832-839, 1988; Gamett M.C. et al., Int. J. Cancer, 31, 661-670, 1983), 5-fluorouridine (Shin L.B. Int. J. Cancer, 46, 1101-1106, 1990), 5-fluoro-2'-deoxyuridine (Goerlach A. et al., Bioconjugate Chem., 2, 96-101, 1991), cytosine arabinoside (Hurwitz E. et al., J. Med. Chem., 28, 137-140, 1985), aminopterin (Kanellos J. et al., Immunol. Cell. Biol., 65, 483-493, 1987), vincristine (Johnson J.R. et al., Br. J. Cancer, 42, 17, 1980), vindesine (Johnson J.R. et al., Br. J. Cancer, 44, 472-475, 1981), interleukin 2 (IL-2), tumor necrosis factor α (TNFa), interferon (INF), carboxypeptidase, alkaline phosphatase, β-lactamase, and cytidine deaminase.

The present invention may use a single cytotoxic substance or a combination of two or more cytotoxic substances.

The aforementioned cytotoxic substances can be bound or conjugated to the anti-HB-EGF antibody by covalent bond or noncovalent bond. Methods for producing antibody conjugated with these cytotoxic substances are known.

The cytotoxic substance may be directly bound to the anti-HB-EGF antibody through, for example, linking groups present on these species themselves, or may be indirectly bound to the anti-HB-EGF antibody through another substance, for example, a linker or intermediary support. The linking group in the case of direct bonding between the anti-HB-EGF antibody and the cytotoxic substance include the disulfide bond, which is based on the utilization of SH groups. In specific terms, an intramolecular disulfide bond in the Fc region of the antibody can be reduced with a reducing agent such as, for example, dithiothreitol; a disulfide bond in the cytotoxic substance can be similarly reduced; and the two species can then be linked to each other by a disulfide bond. The formation of the disulfide bond between the two species may be promoted by preliminary activating either the antibody or the cytotoxic substance with an activation promoter, for example, Ellman's reagent. Examples of other methods for implementing direct bonding between the anti-HB-EGF antibody and the cytotoxic substance are as follows: methods that use a Schiff base, carbodiimide methods, active ester methods (N-hydroxysuccinimide method), methods that use a mixed anhydride, and methods that use the diazo reaction.

Binding between the anti-HB-EGF antibody and cytotoxic substance can also occur by indirect binding through another substance. There are no particular limitations on the other substances employed for indirect binding, and the other substance may include compounds that have at least two groups - comprising a single type or a combination of two or more types - selected from the amino group, carboxyl group, mercapto group, and so forth, and may also include peptide linkers and compounds that have the ability to bind to the anti-HB-EGF antibody. The following are examples of compounds that have at least two groups - comprising a single type or a combination of two or more types - selected from the amino group, carboxyl group, mercapto group, and so forth: N-succinimidyl 3-(2-pyridyldithio)propionate (SPDP; Wawrzynczak E.J. et al., Cancer Res., 50, 7519-7562, 1990; Thorpe P.E. et al., Cancer Res., 47, 5924-5931, 1987), succinimidyl 6-3-[2-pyridyldithio]propionamido)hexanoate (LC-SPDP; Hermanson G.T., BIOCONJUGATE Techniques, 230-232, 1996), sulfosuccinimidyl 6-(3-[2-pyridyldithio]propionamido)hexanoate (sulfo-LC-SPDP; Hermanson G.T., BIOCONJUGATE Techniques, 230-232, 1996), N-succinimidyl 3-(2-pyridyldithio)butyrate (SPDB; Wawrzynczak E.J. et al., Br. J. Cancer, 66, 361-366, 1992), succinimidyloxycarbonyl-α-(2-pyridyldithio)toluene (SMPT; Thorpe P.E. et al., Cancer Res., 47, 5924-5931, 1987), succinimidyl 6-(a-methyl-[2-pyridyldito]toluamide)hexanoate (LC-SMPT; Hermanson G.T., BIOCONJUGATE Techniques, 232-235, 1996), sulfosuccinimidyl 6-(α-methyl-[2-pyridyldithio]toluamide)hexanoate (sulfo-LC-SMPT; Hermanson G.T., BIOCONJUGATE Techniques, 232-235, 1996), succinimidyl-4-(p-maleimidophenyl)butyrate (SMPB; Hermanson G.T., BIOCONJUGATE Techniques, 242-243, 1996), sulfo-succinimidyl-4-(p-maleimidophenyl)butyrate (sulfo-SMPB; Hermanson G.T., BIOCONJUGATE Techniques, 242-243, 1996), m-maleimidobenzoyl-N-hydroxysuccinimide ester (MBS; Hermanson G.T., BIOCONJUGATE Techniques, 237-238, 1996), m-maleimidobenzoyl-N-hydroxysulfosuccinimide ester (sulfo-MBS; Hermanson G.T., BIOCONJUGATE Techniques, 237-238, 1996), S-acetyl mercaptosuccinic anhydride (SAMSA; Casellas P. et al., Eur. J. Biochem., 176, 581-588, 1988), dimethyl 3,3'-dithiobispropionimidate (DTBP; Casellas P. et al., Eur. J. Biochem., 176, 581-588, 1988), 2-iminothiolane (Thorpe P.E. et al., Cancer Res., 47, 5924-5931, 1987), and so forth.

Examples of other substances that can be used to bind the cytotoxic substance to the anti-HB-EGF antibody are peptides, antibodies, poly-L-glutamic acid (PGA), carboxymethyl dextran, dextran, aminodextran, avidin-biotin, cis-aconitic acid, glutamic acid dihydrazide, human serum albumin (HSA), and so forth.

Moreover, a cytotoxic protein can also be attached to the antibody by genetic engineering techniques. As a specific example, DNA encoding a cytotoxic peptide as described above can be fused in-frame with DNA encoding the anti-HB-EGF antibody and a recombinant vector can be constructed by incorporation into an expression vector. The vector can be transfected into a suitable host cell, and the resulting transformed cells can be cultured to express the incorporated DNA and obtain a fusion protein comprising the anti-HB-EGF antibody to which the toxic peptide is attached. In those instances where a fusion protein with an antibody is prepared, the drug protein or protein toxin is generally positioned on the C-terminal side of the antibody. In addition, a peptide linker can be interposed between the antibody and the drug protein or protein toxin.

The neutralizing activity

The anti-HB-EGF antibody used in the present invention may have a neutralizing activity.

A neutralizing activity generally refers to the ability to inhibit the biological activity of a ligand that exhibits biological activity on a cell, with an agonist being an example of such a ligand. Thus, a substance that has a neutralizing activity denotes a substance that binds to such a ligand - or to the receptor that binds the ligand - and thereby inhibits binding by the ligand or by the receptor. The receptor prevented from binding with the ligand as a consequence of the neutralizing activity is then unable to manifest the biological activity that proceeds through the receptor. An antibody that exhibits such a neutralizing activity is generally known as a neutralizing antibody. The neutralizing activity of a particular test substance can be measured by comparing the biological activity in the presence of the ligand and the test substance with the biological activity in the presence of the ligand and the absence of the test substance.

The EGF receptor is considered to be the principal receptor for the HB-EGF described herein. In this case, a dimer is formed due to binding by the ligand and a tyrosine kinase, which is its own domain within the cell, is thereby activated. The activated tyrosine kinase causes the formation by autophosphorylation of phosphorylated tyrosine-containing peptide, with which various signal transduction accessory molecules associate. These are principally PLCγ (phospholipase Cy), Shc, Grb2, and so forth. Among these accessory molecules, the former two are additionally phosphorylated by the tyrosine kinase of the EGF receptor. The principal pathway in signal transduction from the EGF receptor is a pathway in which phosphorylation is transduced in the sequence Shc, Grb2, Sos, Ras, Raf/MAPK kinase/MAP kinase. A pathway from PLCγ to PKC, which is a secondary pathway, is additionally thought to be present. This intracellular signal cascade is different in each cell type, and therefore a suitable target molecule can be established for each desired target cell. There is no limitation to the factors cited above. The neutralizing activity can be evaluated by measuring *in vivo* signal activation. Commercially available kits for measuring *in vivo* signal activation can be suitably used (for example, the protein kinase C activation measurement system from GE Healthcare Biosciences).

*In vivo* signal activation can also be detected by focusing on the induction of transcription for a target gene that is present downstream in the *in vivo* signal cascade. Changes in the transcription activity for a target gene can be detected using the reporter assay concept. In specific terms, a reporter gene (e.g., green fluorescence protein (GFP) or luciferase) can be disposed downstream from the transcription factor or promoter region of the target gene, and by measuring the reporter activity the change in transcription activity can be measured in terms of the reporter activity.

In addition, since signal transduction through the EGF receptor generally acts in the direction of promoting cell growth, the neutralizing activity can be evaluated by measuring the growth activity of a target cell.

Antibody that possesses both a neutralizing activity and an internalizing activity can be a very effective anti-cancer agent for cancers that strongly express HB-EGF.

The ADCC activity and/or CDC activity

The anti-HB-EGF antibody used in the present invention may have an antibody-dependent cell-mediated cytotoxicity (ADCC) and/or a complement-dependent cytotoxicity (CDC).

In the present invention, the CDC activity refers to a cell-destroying activity due to the complement system. The ADCC activity, on the other hand, refers to an activity in which a specific antibody attaches to a cell surface antigen on a target cell, an Fcγ receptor-presenting cell (immune cell and so forth) binds through its Fcγ receptor to the Fc region of the antigen-bound antibody, and the target cell is then attacked.

Known methods can be used in the present invention to measure whether an antibody exhibits ADCC activity and whether an antibody exhibits CDC activity (For example, Current Protocols in Immunology. Chapter 7: Immunologic Studies in Humans. Editor: John E. Coligan et al., John Wiley & Sons, Inc. (1993), and so forth).

In specific terms, effector cells, a complement solution, and target cells are first prepared.

(1) Preparation of the effector cells

The spleen is removed from, for example, CBA/N mice, and the splenocytes are separated in RPMI1640 medium (Invitrogen Corporation). The effector cells can then be prepared by washing with the same medium containing 10% fetal bovine serum (FBS, HyClone) and subsequently adjusting the cell concentration to $5 \times 10^6$/mL.

(2) Preparation of the complement solution

The complement solution can be prepared by diluting baby rabbit complement (Cedarlane Laboratories Ltd.) 10 times with medium containing 10% FBS (Invitrogen Corporation).

(3) Preparation of the target cells

Cells that express HB-EGF protein are cultured with 0.2 mCi $^{51}$Cr sodium chromate (GE Healthcare Biosciences) for 1 hour at 37°C on DMEM medium containing 10% FBS in order to radiolabel these target cells. For example, cancer cells (e.g., ovarian cancer cells) or cells transformed with an HB-EGF protein-encoding gene can be used as the HB-EGF protein-expressing cells. After radiolabeling, the cells are washed 3 times with RPMI1640 medium containing 10% FBS and the target cells are prepared by adjusting the cell concentration to $2 \times 10^5$/mL.

The ADCC activity and CDC activity can be measured by the following methods. In order to measure the ADCC activity, 50 μL target cells and 50 μL anti-HB-EGF antibody are added to a 96-well U-bottom plate (Becton, Dickinson and Company) and a reacted for 15 minutes on ice. Then 100 μL effector cells is added and incubated for 4 hours in a $CO_2$ incubator. A final antibody concentration of 0 or 10 μg/mL is employed. After incubation, 100 μL of the supernatant is recovered and the radioactivity is measured with a gamma counter (COBRA II AUTO-GAMMA, MODEL D5005, Packard Instrument Company). Using the obtained values, the cytotoxic activity (%) can be calculated from the formula (A-C)/(B-C) x 100 where A is the radioactivity (cpm) in the particular sample, B is the radioactivity (cpm) in a sample to which 1% NP-40 (Nacalai Tesque, Inc.) has been added, and C is the radioactivity (cpm) of a sample containing only the target cells.

When, on the other hand, the CDC activity is to be measured, 50 μL target cells and 50 μL anti-HB-EGF antibody are added to a 96-well flat-bottom plate (Becton, Dickinson and Company) and a reacted for 15 minutes on ice. This is followed by the addition of 100 μL complement solution and incubation for 4 hours in a $CO_2$ incubator. A final antibody concentration of 0 or 3 μg/mL is employed. After incubation, 100 μL supernatant is recovered and the radioactivity is measured with a gamma counter. The cytotoxic activity can be calculated in the same manner as for measurement of the ADCC activity.

Antibody that possesses both an internalizing activity and an ADCC activity and/or a CDC activity can be a very effective anti-cancer agent for cancers that strongly express HB-EGF. In addition, antibody that possesses both a neutralizing activity and an ADCC activity and/or a CDC activity can be a very effective anti-cancer agent for cancers that strongly express HB-EGF. Moreover, antibody that possesses an internalizing activity plus a neutralizing activity plus an ADCC activity and/or a CDC activity can be a very effective anti-cancer agent for cancers that strongly express HB-EGF.

Antibody production

Monoclonal anti-HB-EGF antibody according to the present invention can be obtained using known means. Monoclonal antibody of mammalian origin is particularly preferred for the anti-HB-EGF antibody of the present invention. The monoclonal antibody of mammalian origin encompasses, inter alia, monoclonal antibody produced by a hybridoma and monoclonal antibody produced by a host that has been transformed by genetic engineering techniques with an expression vector that comprises the antibody gene.

Monoclonal antibody-producing hybridomas can be prepared using known technology, for example, as described in the following. First an animal is immunized with HB-EGF protein as the sensitizing antigen according to the usual immunization methods. Immune cells obtained from the immunized animal are fused with a known partner cell by the usual cell fusion techniques to obtain hybridomas. Using the usual screening techniques, these hybridomas can be subjected to the selection of hybridomas that produce anti-HB-EGF antibody by screening for cells that produce the desired antibody.

In specific terms, monoclonal antibody production can be carried out, for example, as follows. First, the HB-EGF protein used as the sensitizing antigen for antibody acquisition can be obtained by the expression of an HB-EGF gene. The base sequence of the human HB-EGF gene is disclosed, for example, as GenBank accession number NM_001945 (SEQ ID NO: 11). Thus, the gene sequence encoding HB-EGF is inserted into a known expression vector and a suitable host cell is then transformed with the expression vector; the desired human HB-EGF protein can subsequently be purified from within the host cells or from the culture supernatant. Purified natural HB-EGF protein can also be used in the same manner. The protein may be purified using one or a combination of the usual chromatographic techniques, e.g., ion chromatography, affinity chromatography, and so forth, using a single run or a plurality of runs. The immunogen used in the present invention can also be a fusion protein as obtained by fusion of a desired partial polypeptide from the HB-EGF protein with a different polypeptide. For example, a peptide tag or the Fc fragment from the antibody can be used to produce the fusion protein that will be used as the immunogen. A vector that expresses the fusion protein can be prepared by in-frame fusion of the genes encoding the desired two or more polypeptide fragments and insertion of the fused gene into an expression vector as described above. Methods for producing fusion proteins are described in Molecular Cloning 2nd Edt. (Sambrook, J. et al., Molecular Cloning 2nd Edt., 9.47-9.58, Cold Spring Harbor Laboratory Press, 1989).

The HB-EGF protein purified in the described manner can be employed as the sensitizing antigen used to immunize a mammal. A partial peptide from HB-EGF can also be used as the sensitizing antigen. For example, the following peptides can be used as the sensitizing antigen:

peptide obtained from the amino acid sequence for human HB-EGF by chemical synthesis;
peptide obtained by incorporating a portion of the human HB-EGF gene into an expression vector and expressing same; and
peptide obtained by degradation of human HB-EGF protein with a protein degrading enzyme.

There are no limitations on the HB-EGF region used as the partial peptide or on the size of the partial peptide. A preferred region can be selected from the amino acid sequence constituting the extracellular domain of HB-EGF (positions 22 to 149 in the amino acid sequence of SEQ ID NO: 12). The number of amino acids making up the peptide that will be used as the sensitizing antigen is preferably at least 3, for example, at least 5 or at least 6. More specifically, a peptide of 8 to 50 residues and preferably 10 to 30 residues can be used as the sensitizing antigen.

There are no particular limitations on the mammal that may be immunized by the sensitizing antigen described above. In order to obtain monoclonal antibody by cell fusion techniques, the immunized animal is preferably selected considering the compatibility with the partner cell that will be used in cell fusion. Rodents are generally preferred as the immunized animal. Specifically, the mouse, rat, hamster, or rabbit can be used as the immunized animal. Monkeys can also be used as the immunized animal.

The animal as described above can be immunized with the sensitizing antigen according to known methods. For example, as a general method, the mammal can be immunized by subcutaneous or intraperitoneal injection of the sensitizing antigen. In specific terms, the sensitizing antigen may be administered to the mammal a plurality times on a 4 to 21 day schedule. The sensitizing antigen is used diluted to a suitable dilution factor with, for example, phosphate-buffered saline (PBS) or physiological saline. The sensitizing antigen may also be administered in combination with an adjuvant. For example, the sensitizing antigen can be prepared by mixing and emulsification with Freund's complete adjuvant. A suitable carrier can also be used in immunization with the sensitizing antigen. Particularly in those instances in which a low molecular weight partial peptide is used as the sensitizing antigen, immunization is desirably effected with the sensitizing peptide antigen conjugated with a protein carrier, e.g., albumin, keyhole limpet hemocyanin, and so forth.

After the mammal is immunized in the described manner and a desired rise in the serum antibody titer is observed, immune cells are collected from the mammal and are submitted to cell fusion. Splenocytes in particular are preferred immune cells.

Mammalian myeloma cells are used as the cells for fusion with the above-described immune cells. The myeloma cells are preferably provided with a suitable selection marker to support screening. The selection marker denotes a trait that can appear (or that cannot appear) under specific culture conditions. Known selection markers include hypoxanthine-guanine-phosphoribosyltransferase deficiency (abbreviated below as HGPRT deficiency) and thymidine kinase deficiency (abbreviated below as TK deficiency). Cells that are HGPRT- or TK-deficient exhibit hypoxanthine-aminopterin-thymidine sensitivity (abbreviated below as HAT sensitivity). HAT-sensitive cells are unable to undergo DNA synthesis on an HAT selection medium and die; however, when fused with a normal cell, DNA synthesis can continue using the salvage pathway of the normal cell and growth can also occur on HAT selection medium.

HGPRT-deficient cells can be selected on a medium containing 6-thioguanine or 8-azaguanine (8AG), while TK-deficient cells can be selected on a medium containing 5'-bromodeoxyuridine. Normal cells incorporate these pyrimidine analogues into their DNA and die, while cells deficient in these enzymes do not incorporate these pyrimidine analogs and are able to survive on the selection medium. Another selection marker, known as G418 resistance, imparts resistance to 2-deoxystreptamine-type antibiotics (gentamycin analogues) based on the neomycin resistance gene. Various myeloma cells suitable for cell fusion are known. For example, the following myeloma cells can be employed: P3 (P3x63Ag8.653) (J. Immunol. (1979) 123, 1548-1550), P3x63Ag8U.1 (Current Topics in Microbiology and Immunology (1978) 81, 1-7), NS-1 (Kohler, G. and Milstein, C. Eur. J. Immunol. (1976) 6, 511-519), MPC-11 (Margulies, D.H. et al., Cell (1976) 8, 405-415), SP2/0 (Shulman, M. et al., Nature (1978) 276, 269-270), FO (de St. Groth, S. F. et al., J. Immunol. Methods (1980) 35, 1-21), S194 (Trowbridge, I.S. J. Exp. Med. (1978) 148, 313-323), and R210 (Galfre, G. et al., Nature (1979) 277, 131-133).

Cell fusion between the above-described immune cells and myeloma cells can be carried out according to known methods, for example, according to the method of Kohler and Milstein (Kohler, G. and Milstein, C., Methods Enzymol. (1981) 73, 3-46).

More specifically, cell fusion can be carried out, for example, in the usual nutrient culture fluids in the presence of a cell fusion promoter. For example, polyethylene glycol (PEG) or Sendai virus (HVJ) can be used as the fusion promoter. As desired, an auxiliary such as dimethyl sulfoxide can be added in order to boost the fusion efficiency.

The ratio between the immune cells and the myeloma cells can be freely selected. For example, the immune cells are preferably used at from 1X to 10X with respect to the myeloma cells. The culture fluid used for cell fusion can be, for example, RPMI1640 culture medium or MEM culture medium, which are very suitable for the growth of the previously cited myeloma cell lines, or the usual culture media used for this type of cell culture. A serum supplement such as fetal calf serum (FCS) can also be added to the culture medium.

The desired fused cells (hybridomas) are formed by cell fusion by thoroughly mixing prescribed quantities of the immune cells and myeloma cells in a culture fluid as described above and admixing a PEG solution that has been preheated to about 37°C. For example, PEG with an average molecular weight of 1000 to 6000 can be added to the cell fusion process at a concentration generally from 30 to 60% (w/v). Then, the cell fusion agents and so forth that are undesirable for hybridoma growth are removed by repeating the process of adding a suitable culture fluid as described above, centrifuging, and removing the supernatant.

The hybridomas obtained in the described manner can be selected by using a selection medium adapted to the selection markers exhibited by the myeloma used for cell fusion. For example, HGPRT- or TK-deficient cells can be selected by culture on HAT medium (medium containing hypoxanthine, aminopterin, and thymidine). Thus, when HAT-sensitive myeloma cells are used for cell fusion, cells resulting from cell fusion with the normal cells can selectively grow on the HAT medium. Culture on the HAT medium is continued for a period of time sufficient for cells (unfused cells) other than the desired hybridomas to die. In specific terms, the desired hybridomas can be selected generally by culture for from several days to several weeks. The usual limit dilution process can be used for screening and monocloning of hybridomas that produce the desired antibody. Or, antibody that recognizes HB-EGF can also be produced by the method described in WO 03/104453.

Screening for and monocloning the desired antibody can be suitably carried out by a screening procedure based on known antigen-antibody reactions. For example, an antigen may be bound to a carrier (e.g., beads of, for example, polystyrene, or a commercial 96-well microtiter plate) and then reacted with hybridoma culture supernatant. Then, after the carrier has been washed, the cells are reacted with, for example, an enzyme-labeled secondary antibody. If the desired sensitizing antigen-reactive antibody was present in the culture supernatant, the secondary antibody will bind to the carrier through the antibody. The presence/absence of the desired antibody in the culture supernatant can finally be established by detection of the secondary antibody that is bound to the carrier. A hybridoma that produces the desired antigen-binding antibody can be cloned, for example, by the limit dilution method. Here, substantially the same HB-EGF protein is suitably used as the antigen, including those used for immunization. For example, an oligopeptide comprising the extracellular domain of HB-EGF - or comprising a partial amino acid sequence from that region - can be used as the antigen.

In addition to the above-described method of producing a hybridoma by immunizing a nonhuman animal with antigen, the desired antibody can also be obtained by the antigenic sensitization of human lymphocytes. In specific terms, human lymphocytes are first sensitized in vitro with HB-EGF protein. The immunosensitized lymphocytes are then fused with a suitable fusion partner. For example, myeloma cells of human origin having a permanent cell division ability can be used as the fusion partner (refer to Japanese Patent Publication No. H 1-59878). The anti-HB-EGF antibody obtained by this method is a human antibody that has the activity to bind to HB-EGF protein.

Human anti-HB-EGF antibody can also be obtained by administering HB-EGF protein as antigen to a transgenic animal that has the entire human antibody gene repertoire. Antibody-producing cells from the immunized animal can be immortalized by cell fusion with a suitable fusion partner or by a treatment such as infection with the Epstein-Barr virus. Human antibody to the HB-EGF protein can be isolated from the resulting immortalized cells (refer to International Publications WO 94/25585, WO 93/12227, WO 92/03918, and WO 94/02602). Moreover, cells that produce antibody having the desired reaction specificity can also be cloned by cloning the immortalized cells. When a transgenic animal is employed as the immunized animal, the animal's immune system recognizes human HB-EGF as foreign. This makes it possible to readily obtain human antibody directed against human HB-EGF. The monoclonal antibody-producing hybridoma constructed in the described manner can be subcultured in the usual culture media. Long-term storage of the hybridoma in liquid nitrogen is also possible.

The aforementioned hybridoma can be cultured according to the usual methods and the desired monoclonal antibody can be obtained from the resulting culture supernatant. Or, the hybridoma can be injected to a mammal compatible with the cells and monoclonal antibody can be obtained from the ascites fluid of the mammal. The former method is well suited for the production of high-purity antibody.

The present invention can also use antibody encoded by an antibody gene that has been cloned from an antibody-producing cell. Antibody expression can be achieved by incorporating the cloned antibody gene into a suitable vector followed by transfection into a host. Methods have already been established for isolating the antibody gene and inserting it into a vector and for transforming the host cell (refer, for example, to Vandamme, A.M. et al., Eur. J. Biochem. (1990) 192, 767-775).

For example, cDNA encoding the variable region (V region) of the anti-HB-EGF antibody can be obtained from a hybridoma cell that produces anti-HB-EGF antibody. The total RNA is typically first extracted from the hybridoma. Methods that can be used to extract the mRNA from cells are, for example, the guanidine ultracentrifugal method (Chirgwin, J. M. et al., Biochemistry (1979) 18, 5294-5299) and the AGPC method (Chomczynski, P. et al., Anal. Biochem. (1987) 162, 156-159).

The extracted mRNA can be purified using, for example, an mRNA Purification Kit (GE Healthcare Biosciences). Or, kits for the direct extraction of the total mRNA from cells are also commercially available, such as the QuickPrep mRNA Purification Kit (GE Healthcare Biosciences). Kits such as these can also be used to obtain the total mRNA from hybridomas. cDNA encoding the antibody V region can be synthesized from the obtained mRNA using a reverse transcriptase. The cDNA can be synthesized with, for example, an AMV Reverse Transcriptase First-Strand cDNA Synthesis Kit (Seikagaku Corporation). In addition, a 5'-Ampli FINDER RACE Kit (Clontech) and the PCR-based 5'-RACE method (Frohman, M.A. et al., Proc. Natl. Acad. Sci. USA (1988) 85, 8998-9002; Belyavsky, A., et al., Nucleic Acids Res. (1989) 17, 2919-2932) can be used to synthesize and amplify the cDNA. Moreover, suitable restriction enzyme sites, infra, can be introduced at both ends of the cDNA in such a cDNA synthesis procedure.

The target cDNA fragment is purified from the obtained PCR product and is then ligated with vector DNA; the recombinant vector fabricated in this manner is transfected into, for example, *E. coli,* and colonies are selected; and the desired recombinant vector can be prepared from the *E. coli* that has exhibited colony formation. In addition, known methods, for example, the dideoxynucleotide chain termination method, can be used to ascertain whether the recombinant vector has the base sequence of the target cDNA.

In order to obtain a gene that encodes the variable region, PCR using variable region gene amplification primers can also be employed. First, cDNA is synthesized using extracted mRNA as the template in order to obtain a cDNA library. A commercially available kit is conveniently used to synthesize the cDNA library. In actuality, the amount of mRNA obtained from only a small number of cells will be quite small, and thus its direct purification provides a low yield. Accordingly, purification is generally carried out after the addition of carrier RNA that clearly does not contain the antibody gene. Or, in those cases in which a certain amount of RNA can be extracted, it may be possible to achieve an efficient extraction even with only the RNA from the antibody-producing cells. For example, in some cases it may not be necessary to add carrier RNA to RNA extraction from at least 10 or at least 30 and preferably at least 50 antibody-producing cells.

Employing the obtained cDNA library as a template, the antibody gene can be amplified by PCR. Primers for the PCR-based amplification of antibody genes are known. For example, primers for the amplification of human antibody genes can be designed based on the information in the literature (for example, J. Mol. Biol. (1991) 222, 581-597). These primers have a base sequence that varies with the immunoglobulin subclass. Thus, when a cDNA library of unknown subclass is employed as the template, PCR is carried out considering all of the possibilities.

In specific terms, when the goal is, for example, the acquisition of genes encoding human IgG, primers can be used that have the ability to amplify genes encoding $\gamma 1$ to $\gamma 5$ for the heavy chain and the $\kappa$ chain and $\lambda$ chain for the light chain. In order to amplify the IgG variable region gene, a primer that anneals to the region corresponding to the hinge region is ordinarily used for the 3'-side primer. On the other hand, a primer adapted for each subclass can be used for the 5'-side primer.

The PCR products based on gene amplification primers for each heavy chain and light chain subclass are made as respective independent libraries. Using the libraries thus synthesized, immunoglobulin comprising a heavy chain plus light chain combination can be reconstructed. The desired antibody may be screened using as an indicator the binding activity of the reconstructed immunoglobulin for HB-EGF.

Binding by the antibody of the present invention to HB-EGF is more preferably specific binding. Screening for antibody that binds HB-EGF can be carried out, for example, by the following steps:

(1) bringing HB-EGF into contact with antibody comprising a V region encoded by cDNA obtained from a hybridoma;
(2) detecting binding between the HB-EGF and the antibody; and
(3) selecting antibody that binds to the HB-EGF.

Methods of detecting binding between an antibody and HB-EGF are known. In specific terms, the test antibody may be reacted with HB-EGF that has been immobilized on a carrier and then reacted with a labeled antibody that recognizes the antibody. When, after washing, the labeled antibody can be detected on the carrier as an indicator of binding of the test antibody to the HB-EGF. A fluorescent substance such as FITC or an enzymatic protein such as peroxidase or $\beta$-galactoside can be used for the label. HB-EGF-expressing cells in immobilized form can also be used to evaluate the antibody's binding activity.

Panning using a phage vector can also be employed as a method of antibody screening using binding activity as the indicator. Screening using a phage vector is advantageous when as described above the antibody genes are obtained as heavy chain subclass and light chain subclass libraries. The genes encoding the heavy chain and light chain variable regions can be made into a single-chain Fv (scFv) by linking with a suitable linker sequence. The scFv-encoding gene may be inserted into a phage vector to obtain a phage that expresses scFv on its surface. The phage is brought into contact with the target antigen, and the recovery of phage that was bound to the antigen enables the recovery of DNA coding for scFv that has the desired binding activity. scFv having the desired binding activity can be enriched by repeating this process as necessary.

In the present invention, antibody-encoding polynucleotide may encode the full length of the antibody or may encode a portion of the antibody. This portion of the antibody may be any portion of the antibody molecule. Antibody fragment is a term used below in some instances to indicate a portion of an antibody. Preferred antibody fragments in the present invention comprise the complementarity determination region (CDR). A more preferred antibody fragment in the present invention comprises all of the three CDRs that constitute the variable region.

Once the cDNA encoding the V region of the target anti-HB-EGF antibody has been obtained, cDNA is digested by restriction enzymes that recognize the restriction enzyme sites that have been inserted at both ends of the cDNA. Preferred restriction enzymes will recognize and digest base sequences that have a low potential of occurrence in the base sequence constituting the antibody gene. In order to insert 1 copy of the digestion fragment in the correct direction in the vector, a restriction enzyme that provides cohesive ends is preferred. An antibody expression vector can be obtained by inserting the cDNA encoding the anti-HB-EGF antibody V region, digested as described in the preceding, into a suitable expression vector. At this point, a chimeric antibody can be obtained through the in-frame fusion of a gene encoding the antibody constant region (C region) with the aforementioned V region-encoding gene. Here, chimeric antibody refers to a product having different origins for the constant region and variable region. Accordingly, in the context of the present invention "chimeric antibody" also encompasses human-human allochimeric antibodies in addition to heterochimeric antibodies such as mouse-human. A chimeric antibody expression vector can also be constructed by inserting the aforementioned V region gene into an expression vector that already carries the constant region.

In specific terms, for example, a restriction enzyme recognition sequence for a restriction enzyme used to digest the aforementioned V region gene can be disposed in advance on the 5' side of an expression vector that holds the DNA coding for the desired antibody constant region (C region). Digestion of the two with the same restriction enzyme combination and in-frame fusion results in the construction of a chimeric antibody expression vector.

In order to produce the anti-HB-EGF antibody of the present invention, the antibody gene can be incorporated in the expression vector in such a manner that expression occurs under control by an expression control region. Expression control regions for antibody expression include, for example, enhancers and promoters. Recombinant cells that express DNA coding for anti-HB-EGF antibody can then be obtained by transforming suitable host cells with the expression vector under consideration.

For expression of the antibody gene, the DNA coding for the antibody heavy chain (H chain) and the DNA coding for the antibody light chain (L chain) can be incorporated in separate expression vectors. An antibody molecule provided with H and L chains can be expressed by simultaneously transforming (co-transfect) the same host cell with the vector incorporating the H chain and the vector incorporating the L chain. Or, DNA encoding the H chain and L chain may be incorporated in a single expression vector and the host cell may then be transformed (International Publication WO 94/11523).

Numerous host/expression vector combinations are known for antibody production by isolating temporarily the antibody gene and transfecting a suitable host. Any of these expression systems may be applied to the present invention. Animal cells, plant cells, or fungal cells can be used when eukaryotic cells are used as the host. Specific examples of animal cells that can be used in the present invention are mammalian cells (e.g., CHO, COS, myeloma, baby hamster kidney (BHK), Hela, Vero, and for so forth), amphibian cells (e.g., *Xenopus laevis* oocytes and so forth), and insect cells (e.g., sf9, sf21, Tn5, and so forth).

In the case of plant cells, antibody gene expression systems based on cells from genus *Nicotiana,* e.g., *Nicotiana tabacum* and so forth, are known. Callus-cultured cells can be used for plant cell transformation.

The following, for example, can be used as the fungal cells: yeast (e.g., *Saccharomyces* such as *Saccharomyces cerevisiae, Pichia* such as *Pichia pastoris,* and so forth), and filamentous fungi (e.g., *Aspergillus* such as *Aspergillus niger*) .

Antibody gene expression systems using prokaryotes are also known. Taking bacteria as an example, bacteria such as *E. coli, Bacillus subtilis,* and so forth, can be used in the present invention.

When a mammalian cell is used, an expression vector can be constructed by functionally ligating an effective, commonly used promoter, the antibody gene that is to be expressed, and a polyA signal downstream at the 3'-terminal of the antibody gene. An example of a promoter/enhancer is the human cytomegalovirus immediate early promoter/enhancer.

Other promoter/enhancers that can be used to express the antibody of the present invention are, for example, viral promoter/enhancers and promoter/enhancers that originate in mammalian cells, such as human elongation factor 1α (HEF1α). Specific examples of viruses that can provide usable promoter/enhancers are retroviruses, polyoma viruses, adenoviruses, and simian virus 40 (SV40).

The SV40 promoter/enhancer can be used according to the method of Mulligan et al. (Nature (1979) 277, 108). In addition, the HEF1α promoter/enhancer can be readily utilized for the desired gene expression according to the method of Mizushima et al. (Nucleic Acids Res. (1990) 18, 5322).

In the case of *E. coli,* expression of the gene under consideration can be achieved by functionally ligating an effective, commonly used promoter, a signal sequence for antibody secretion, and the antibody gene that is to be expressed. The promoter can be, for example, the lacZ promoter or the araB promoter. The lacZ promoter can be used according to the method of Ward et al. (Nature (1989) 341, 544-546; FASEBJ. (1992) 6, 2422-2427). Or, the araB promoter can be used for the desired gene expression according to the method of Better et al. (Science (1988) 240, 1041-1043).

With regard to the signal sequence for antibody secretion, the pelB signal sequence (Lei, S.P. et al., J. Bacteriol. (1987) 169, 4379) may be used in the case of production in the *E. coli* periplasm. After the antibody produced in the periplasm has been isolated, the antibody structure can be reorganized (refolded) - by the use of a protein denaturant such as the guanidine hydrochloride and urea - so as to exhibit the desired binding activity.

The origin of replication inserted into the expression vector can be, for example, an origin of replication originating in SV40, polyoma virus, adenovirus, bovine papilloma virus (BPV), and so forth. In addition, a selection marker can be inserted in the expression vector for amplification of the gene copy number in the host cell system. In specific terms, usable selection markers are the aminoglycoside transferase (APH) gene, the thymidine kinase (TK) gene, the *E. coli* xanthine-guanine phosphoribosyltransferase (Ecogpt) gene, the dihydrofolate reductase (dhfr) gene, and so forth.

The target antibody can be produced by transfecting the expression vector under consideration into a host cell and culturing the transformed host cell *in vitro* or *in vivo.* Host cell culture can be carried out according to known methods.

For example, DMEM, MEM, RPMI1640, or IMDM can be used as the culture medium; a serum supplement such as fetal calf serum (FCS) can also be added.

The antibody expressed and produced as described above can be purified by the usual methods known for use for protein purification; a single such method can be used or suitable combinations of these methods can be used. The antibody can be isolated and purified using suitable selections and combinations of, for example, an affinity column (for example, a protein A column), column chromatography, filtration, ultrafiltration, salting out, dialysis, and so forth (Antibodies: A Laboratory Manual. Ed Harlow, David Lane, Cold Spring Harbor Laboratory, 1988).

In addition to host cells as described in the preceding, transgenic animals can also be used to produce recombinant antibodies. That is, the antibody under consideration can be obtained from an animal into which a gene encoding the target antibody has been introduced. For example, a fused gene can be fabricated by the in-frame insertion of the antibody gene within a gene coding for a protein that is natively produced in milk. For example, goat β-casein can be used as the protein secreted into milk. A DNA fragment containing the fused gene that incorporates the antibody gene may be injected into a goat embryo and the injected embryo may be introduced into a female goat. The desired antibody can be obtained as a fusion protein with the milk protein from the milk produced by the transgenic goat (or its offspring) born from the embryo-implanted goat. In addition, hormones can be used as appropriate on the transgenic goat in order to increase the amount of milk containing the desired antibody that is produced from the transgenic goat (Ebert, K.M. et al., Bio/Technology (1994) 12, 699-702).

C regions originating in animal antibodies can be used as the C region of the recombinant antibody of the present invention. The mouse antibody H chain C regions designated Cγ1, Cγ2a, Cγ2b, Cγ3, Cμ, Cδ, Cα1, Cα2, and Cε can be used, and the L chain C regions designated as Cκ and Cλ can be used. Animal antibodies from, for example, the rat, rabbit, goat, sheep, camel, monkey, and so forth, can be used as animal antibodies other than mouse antibodies. These sequences are known. The C region can be modified in order to improve the antibody or improve the stability of its production. When the antibody will be administered to humans, an artificially engineered genetically recombinant antibody can be made in the present invention with the goal, for example, of lowering the foreign antigenicity in the human. Such a genetically recombinant antibody includes, for example, chimeric antibodies and humanized antibodies.

These engineered antibodies can be produced using known methods. A chimeric antibody denotes an antibody in which a variable region is ligated to a constant region that has a different origin from the variable region. For example, an antibody having a heavy chain variable region and a light chain variable region from a mouse antibody and a heavy chain constant region and light chain constant region from a human antibody is a mouse-human-heterochimeric antibody. A recombinant vector that expresses chimeric antibody can be constructed by ligating DNA that encodes mouse antibody variable region to DNA that encodes human antibody constant region and incorporating it into an expression vector. A recombinant cell transformed by the vector is then cultured to bring about expression of the incorporated DNA, and the produced chimeric antibody in the culture medium can then be recovered. The C region of human antibody is used for the C region of chimeric antibodies and humanized antibodies. With regard to the H chain, for example, Cγ1, Cγ2, Cγ3, Cγ4, Cμ, Cδ, Cα1, Cα2, and Cε can be used for the C region. For the L chain, Cκ and Cλ can be used for the C region. The amino acid sequences of these C regions are known, as are the base sequences that code for these amino acid sequences. In addition, the human antibody C region can be modified in order to improve the antibody itself or improve the stability of antibody production.

Chimeric antibodies are generally constructed from the V regions of antibodies of nonhuman animal origin and the C regions of antibodies of human origin. In contrast, a humanized antibody is constructed of complementarity determining regions (CDRs) from antibody of nonhuman animal origin, framework regions (FRs) from antibody of human origin, and C regions from antibody of human origin. Humanized antibodies are useful as active ingredients in therapeutic agents of the present invention with the goal of lowering the antigenicity in the human body.

The variable region of an antibody is typically constructed of three CDRs sandwiched in four FRs. The CDRs are regions that substantially determine the binding specificity of an antibody. The amino acid sequences of CDRs are richly diverse. The amino acid sequences that form the FRs, on the other hand, frequently exhibit high homology even between antibodies that have different binding specificities. Due to this, the binding specificity of a certain antibody can typically be grafted into another antibody by CDR grafting.

Humanized antibodies are also known as reshaped human antibodies. In specific terms, for example, humanized antibodies are known in which the CDRs from a nonhuman animal antibody, such as a mouse antibody, have been grafted into a human antibody. General genetic recombination techniques for obtaining humanized antibodies are also known.

In specific terms, for example, overlap extension PCR is known as a method for grafting mouse antibody CDRs into human FRs. In overlap extension PCR, a base sequence encoding the mouse antibody CDR to be grafted is added to a primer for the synthesis of human antibody FR. Primers are prepared for each of the four FRs. The selection of human FR that exhibits a high homology with mouse FR is generally advantageous for maintenance of CDR function in the grafting of mouse CDR to human FR. Thus, the use is generally preferred of human FR that has an amino acid sequence that exhibits high homology with the amino acid sequence of the FR adjacent to the mouse CDR to be grafted.

In addition, the base sequences that are ligated are designed so as to join with each other in-frame. The human FRs are synthesized separately using primers for each. In this way, products are obtained in which DNA encoding mouse CDR is appended to each FR. The base sequences encoding the mouse CDR in each product are designed so as to overlap with each other. Then, the overlapping CDR regions of the products synthesized with the human antibody gene as a template are annealed to each other and a complementary chain synthesis reaction is carried out. This reaction results in ligation of the human FRs via the mouse CDR sequences.

Finally, the variable region gene comprising four FRs ligated with three CDRs is submitted to full length amplification by annealing, at its 5' end and 3' end, primers to which suitable restriction enzyme recognition sequences have been added. An expression vector for human-type antibody can be constructed by inserting the DNA obtained as described above and DNA encoding a human antibody C region into an expression vector in such a manner that they are fused in-frame. The thus-formulated vector is inserted into a host and a recombinant cell is established; the recombinant cell is cultured to express the DNA encoding the humanized antibody; and humanized antibody is thereby produced in the culture medium of the cultured cells (refer to European paten Publication EP 239,400 and International Publication WO 96/02576).

Human antibody FRs that when ligated across CDRs enable the CDRs to form high-quality antigen binding sites, can be suitably selected by qualitatively or quantitatively measuring and evaluating the binding activity to antigen by humanized antibody that has been constructed as described in the preceding. Amino acid substitution can also be carried on the FRs as necessary so as to enable the CDRs of the reshaped human antibody to form well-adapted antigen binding sites. For example, mutations in the amino acid sequence can be introduced into an FR using the PCR methodology used to graft mouse CDRs onto human FRs. In specific terms, partial base sequence mutations can be introduced in the primers that are annealed to the FR. Base sequence mutations are then introduced into the FR synthesized using such primers. A mutated FR sequence having the desired properties can be selected by measurement and evaluation, by the methods described above, of the antigen binding activity of the mutated, amino acid-substituted antibody (Sato, K. et al., Cancer Res., 1993, 53, 851-856).

Methods for obtaining human antibodies are also known. For example, human lymphocytes can be sensitized *in vitro* with a desired antigen or with cells that express a desired antigen. The desired human antibody capable of binding to the antigen can then be obtained by fusing the sensitized lymphocytes with human myeloma cells (refer to Japanese Patent Publication No. H 1-59878). For example, U266 can be used for the human myeloma cell employed as the fusion partner.

A desired human antibody can also be obtained by immunizing a transgenic animal having the entire human antibody gene repertoire with a desired antigen (refer to International Publications WO 93/12227, WO 92/03918, WO 94/02602, WO 94/25585, WO 96/34096, and WO 96/33735). Technology for obtaining human antibodies by panning using a human antibody library are also known. For example, the human antibody V region can be expressed as a single chain antibody (scFv) on the surface of a phage by the phage display method and phage that binds to an antigen can be selected. The DNA sequence that codes for the V region of human antibody that binds the antigen can then be established by analysis of the genes of the selected phage. Once the DNA sequence of the antigen-binding scFv has been established, the V region sequence can be in-frame fused with a sequence for the desired human antibody C region, after which an expression vector can be constructed by insertion in an appropriate expression vector. The expression vector can be transfected into an appropriate expression cell as described above and human antibody can be obtained by expression of the gene coding for the human antibody. These methods are already known (International Publications WO 92/01047, WO 92/20791, WO 93/06213, WO 93/11236, WO 93/19172, WO 95/01438, and WO 95/15388).

Insofar as binding to the HB-EGF protein occurs, the antibody according to the present invention encompasses not only bivalent antibody as typified by IgG, but also polyvalent antibody as typified by IgM and monovalent antibody. Polyvalent antibody according to the present invention includes polyvalent antibody in which all the antigen binding sites are the same and polyvalent antibody in which some or all of the antigen binding sites are different. Antibody according to the present invention is not limited to the full length antibody molecule, but includes low molecular weight antibody and modifications thereof, insofar as these can bind to the HB-EGF protein.

Low molecular weight antibody encompasses antibody fragments generated by the deletion of a portion of the whole antibody (for example, whole IgG). A partial deletion of the antibody molecule is permissible as long as the ability to bind to the HB-EGF antigen is present. The antibody fragment used in the present invention preferably comprises either the heavy chain variable region (VH) or the light chain variable region (VL) or both. The amino acid sequence of the VH or VL can comprise substitutions, deletions, additions, and/or insertions. Moreover, a portion of either the VH or VL or of both can also be deleted, insofar as the ability to bind the HB-EGF antigen remains present. The variable region may also be chimerized or humanized. Specific examples of antibody fragments are Fab, Fab', F(ab')2, and Fv. Specific examples of low molecular weight antibodies are Fab, Fab', F(ab')2, Fv, scFv (single chain Fv), diabody, and sc(Fv)2 (single chain (Fv)2). Multimers of these antibodies (e.g., dimers, trimers, tetramers, polymers) are also encompassed by the low molecular weight antibodies of the present invention.

The antibody fragments can be obtained by the enzymatic treatment of an antibody to produce antibody fragments. For example, papain, pepsin, plasmin, and so forth, are known as enzymes that produce antibody fragments. Or, a gene encoding such an antibody fragment can be constructed and inserted into an expression vector followed by expression by a suitable host cell (refer, for example, to Co, M.S. et al., J. Immunol. (1994) 152, 2968-2976; Better, M. & Horwitz, A.H. Methods in Enzymology (1989) 178, 476-496; Plueckthun, A. & Skerra, A. Methods in Enzymology (1989) 178, 476-496; Lamoyi, E. Methods in Enzymology (1989) 121, 652-663; Rousseaux, J. et al., Methods in Enzymology (1989) 121, 663-669; and Bird, R.E. et al., TIBTECH (1991) 9, 132-137).

A digestive enzyme cleaves specific antibody fragment sites to yield antibody fragments with specific structures as described below. Any portion of the antibody can be deleted when genetic engineering techniques are applied to these enzymatically generated antibody fragments.

papain digestion: F(ab)2 or Fab

pepsin digestion: F(ab')2 or Fab'

plasmin digestion: Facb

Diabody designates a bivalent antibody fragment that is constructed by gene fusion (Holliger, P. et al., Proc. Natl. Acad. Sci. USA 90, 6444-6448 (1993), EP 404,097, WO 93/11161, and so forth). A diabody is a dimer built up from two polypeptide chains. In general, each of the polypeptide chains constituting a diabody is a VL and a VH ligated by a linker into one and the same chain. The linker for a diabody is generally sufficiently short that the VL and VH are unable to bind to one another. In specific terms, for example, about five amino acid residues make up the linker. Due to this, the VL and VH coded on the same polypeptide chain are unable to form a single chain variable region fragment and form a dimer with a separate single chain variable region fragment. Thus a diabody has two antigen binding sites.

scFv is obtained by ligating the H chain V region of an antibody to the L chain V region. The H chain V region and L chain V region in scFv are ligated to each other by a linker and preferably a peptide linker (Huston, J.S. et al., Proc. Natl. Acad. Sci. USA 85, 5879-5883 (1988)). The H chain V region and L chain V region in the scFv may originate from any antibody described herein. There are no particular limitations on the peptide linker that links the V regions. For example, any single peptide chain having from about 3 to 25 residues can be used as the linker.

The V regions can be linked, for example, using the PCR techniques described in the preceding. In order to link the V regions by PCR, DNA coding for all or a desired portion of the amino acid sequence from the DNA sequence coding for the H chain or H chain V region of the antibody and DNA coding for all or a desired portion of the amino acid sequence from the DNA sequence coding for the L chain or L chain V region of the antibody are first used as templates.

The DNA encoding the H chain V region and the DNA encoding the L chain V region are each amplified by PCR using pairs of primers that have sequences that correspond to the sequences at the two ends of the DNA to be amplified. DNA coding for the peptide linker region is then prepared. The peptide linker-encoding DNA can also be synthesized using PCR. A base sequence that can join with each of the separately synthesized V region amplification products is added in advance to the 5' side of the primers used. A PCR reaction is then run using assembly PCR primers and each of the DNAs for [H chain V region DNA]-[peptide linker DNA]-[L chain V region DNA]. The assembly PCR primers are a combination of a primer that anneals to the 5' side of the [H chain V region DNA] and a primer that anneals to the 3' side of the [L chain V region DNA]. That is, the assembly PCR primers form a primer set that can amplify DNA that encodes the full length sequence of the scFv that is to be synthesized. On the other hand, base sequences that can join with each V region DNA are added to the [peptide linker DNA]. As a result, these DNAs are joined and, in addition, the full length of the scFv is finally produced as an amplification product by the assembly PCR primers. Once the scFv-encoding DNA has been produced, an expression vector containing the DNA as well as recombinant cells transformed by the expression vector can be obtained by the usual methods. In addition, the recombinant cells thus obtained can be cultured and scFv can be obtained through expression of the scFv-encoding DNA.

sc(Fv)2 is a low molecular weight antibody in which two VHs and two VLs are ligated by, for example, a linker, into a single chain (Hudson et al., J. Immunol. Methods, 231, 177-189 (1999)). sc(Fv)2 can be prepared, for example, by joining scFv's with a linker.

This is preferably an antibody that characteristically has the two VHs and the two VLs lined up in the sequence, considered from the N-terminal side of the single chain polypeptide, VH, VL, VH, VL ([VH]linker-[VL]linker-[VH]linker-[VL]).

The sequence of the two VHs and the two VLs is not particularly limited to the arrangement cited above and they may be aligned in any sequence. The following sequences can be provided as examples.

[VL]linker-[VH]linker-[VH]linker-[VL]

[VH]linker-[VL]linker-[VL]linker-[VH]

[VH]linker-[VH]linker-[VL]linker-[VL]

[VL]linker-[VL]linker-[VH]linker-[VH]

[VL]linker-[VH]linker-[VL]linker-[VH]

The linker connecting the variable regions of the antibody can be, for example, any peptide linker that can be inserted by genetic engineering or a synthetic compound linker, for example, as disclosed in Protein Engineering, 9(3), 299-305 (1996). Peptide linkers are preferred in the present invention. The length of the peptide linker is not particularly limited and can be selected as appropriate by those skilled in the art in view of the intended application. In general, from 1 to 100 amino acid residues, preferably from 3 to 50 amino acid residues, more preferably from 5 to 30 amino acid residues, and particularly preferably from 12 to 18 amino acid residues (for example, 15 amino acid residues) are in the peptide linker.

The amino acid sequence of the peptide linker can be any sequence that does not interfere with the binding action of the scFv.

Alternatively, the V regions can also be joined using a synthetic chemical linker (chemical crosslinking agent). Those crosslinking agents typically used to crosslink, for example, peptide compounds, can be used in the present invention. The following, for example, can be used: N-hydroxysuccinimide (NHS), disuccinimidyl suberate (DSS), bis (sulfosuccinimidyl) suberate (BS3), dithiobis(succinimidylpropionate) (DSP), dithiobis(sulfosuccinimidylpropionate) (DTSSP), ethylene glycol bis(succinimidylsuccinate) (EGS), ethylene glycol bis(sulfosuccinimidylsuccinate) (sulfo-EGS), disuccinimidyl tartrate (DST), disulfosuccinimidyl tartrate (sulfo-DST), bis[2-(succinimidooxycarbonyloxy)ethyl]sulfone (BSOCOES), bis[2-(sulfosuccinimidooxycarbonyloxy)ethyl]sulfone (sulfo-BSOCOES), and so forth.

Three linkers are ordinarily required when ligating four antibody variable regions. Linkers in plurality may be the same as each other or different linkers may be used. Diabody and sc(Fv)2 are preferred low molecular weight antibodies for the present invention. To obtain such low molecular weight antibodies, an antibody may be treated with an enzyme (for example, papain, pepsin, and so forth) to produce antibody fragments, or DNA encoding these antibody fragments may be constructed and inserted into an expression vector followed by expression in a suitable host cell (refer, for example, to Co, M.S. et al., J. Immunol. (1994) 152, 2968-2976; Better, M. and Horwitz, A.H. Methods Enzymol. (1989) 178, 476-496; Plueckthun, A. and Skerra, A. Methods Enzymol. (1989) 178, 497-515; Lamoyi, E. Methods Enzymol. (1986) 121, 652-663; Rousseaux, J. et al., Methods Enzymol. (1986) 121, 663-669; and Bird, R.E. and Walker, B.W. Trends Biotechnol. (1991) 9, 132-137).

In addition, the antibody of the present invention can also be used in the form of a modified antibody to which various molecules, for example, polyethylene glycol (PEG) and so forth, are attached. These modified antibodies can be obtained by chemical modification on the antibody according to the present invention. Antibody modification methods have already been established in the art.

The antibody of the present invention may also be a bispecific antibody. A bispecific antibody is an antibody that has, within the same antibody molecule, variable regions that recognize different epitopes, wherein these epitopes may be present in different molecules or may be present in a single molecule. Thus, in the context of the present invention, a bispecific antibody can have antigen binding sites that recognize different epitopes on the HB-EGF molecule. With such a bispecific antibody, two antibody molecules can bind to one HB-EGF molecule. Therefore a stronger cytotoxicity can be expected. These antibodies are also encompassed by the "antibody" according to the present invention.

The present invention also encompasses bispecific antibody that recognizes an antigen other than HB-EGF. For example, the present invention encompasses bispecific antibody that recognizes an antigen different from HB-EGF, wherein the antigen is specifically expressed on the cell surface of cancer cells that are likewise targets for HB-EGF.

Methods of producing bispecific antibodies are known. For example, a bispecific antibody can be produced by joining two antibodies that recognize different antigens. Each of the joined antibodies may be a half-molecule that has an H chain and an L chain or may be a quarter-molecule that has only an H chain. Or, a fused cell that produces bispecific antibody can also be produced by fusing hybridomas that produce different monoclonal antibodies. Bispecific antibodies can additionally be produced by genetic engineering techniques.

The antibody of the present invention may also be an antibody having engineered sugar chains. It is known that antibody cytotoxicity can be enhanced by engineering the sugar chains on an antibody.

The following are examples of antibodies that have engineered sugar chains: glycosylation-engineered antibodies (e.g., WO 99/54342), antibodies in which the fucose present in the sugar chain has been deleted (e.g., WO 00/61739, WO 02/3140, WO 2006/067847, WO 2006/067913), and antibodies bearing a sugar chain that has bisecting GlcNAc (e.g., WO 02/79255).

Fucose-negative antibody is an example of a preferred sugar chain-engineered antibody of the present invention. The sugar chain linked to an antibody can be an N-glycoside linked sugar chain, which is linked to the side-chain N atom of an asparagine in the antibody molecule, or can be an O-glycoside linked sugar chain, which is linked to the side-chain hydroxyl group of a serine or threonine in the antibody molecule; however, in the present invention, the presence/absence of fucose is an issue involving N-glycoside linked sugar chains.

In the context of the present invention, a fucose-negative antibody indicates that the fucose has been deleted in at least 20%, preferably at least 50%, more preferably at least 70%, and even more preferably at least 90% of the N-glycoside linked sugar chains, based on the N-glycoside linked sugar chains on the antibodies in the particular composition.

Fucose-negative antibody can be prepared by methods known to those skilled in the art; for example, it can be produced by expressing the antibody protein in a host cell that has little or no ability to add $\alpha$-1,6 core fucose. The host cell that has little or no ability to add fucose may include, but not limited to, YB2/3HL.P2.G11.16Ag.20 rat myeloma cells (abbreviated as YB2/0 cells, preserved as ATCC CRL 1662), FTVIII knock out CHO cells (WO 02/31140), Lecl3 cells (WO 03/035835), and fucose transporter-negative cells (e.g., WO 2006/067847, WO 2006/067913).

Sugar chain may be analyzed by methods known to those skilled in the art. For example, the sugar chains can be released from an antibody by the action of, for example, N-Glycosidase F (Roche) on the antibody. Then the sample is desalted by solid-phase extraction using a cellulose cartridge (Shimizu Y. et al., Carbohydrate Research 332 (2001), 381-388) followed by concentration to dryness and fluorescent labeling with 2-aminopyridine (Kondo A. et al., Agricultural and Biological Chemistry 54:8 (1990), 2169-2170). After the reagent has been removed from the obtained PA-labeled sugar chain by solid-phase extraction using a cellulose cartridge, the purified PA-labeled sugar chain is obtained by concentrating on centrifuge, and measured by reverse-phase HPLC analysis using an ODS column. In addition, after the PA-labeled sugar chain is prepared, two-dimensional mapping can also be carried out, in which reverse-phase HPLC analysis with an ODS column is combined with normal-phase HPLC analysis with an amine column.

Examples of antibodies which may be used in the present invention include the antibodies of [1] and [2] below:

[1] an antibody comprising a heavy chain variable region having the amino acid sequence of SEQ ID NO:1 as CDR1, the amino acid sequence of SEQ ID NO:2 as CDR2, and the amino acid sequence of SEQ ID NO:3 as CDR3, and a light chain variable region having the amino acid sequence of SEQ ID NO:4 as CDR1, the amino acid sequence of SEQ ID NO:5 as CDR2, and the amino acid sequence of SEQ ID NO:6 as CDR3; and
[2] an antibody which binds to the same epitope as the epitope to which the antibody set forth in [1] binds.

Illustrative examples of heavy chains having the amino acid sequence of SEQ ID NO:1 as CDR1, the amino acid sequence of SEQ ID NO:2 as CDR2 and the amino acid sequence of SEQ ID NO:3 as CDR3 may include, but not limited to, a heavy chain comprising a heavy chain variable region having the amino acid sequence from Glu at position 20 to Ala at position 137 of the amino acid sequence of SEQ ID NO:8. Also illustrative examples of light chains having the amino acid sequence of SEQ ID NO:4 as CDR1, the amino acid sequence of SEQ ID NO:5 as CDR2 and the amino acid sequence of SEQ ID NO:6 as CDR3 may include, but not limited to, a light chain comprising a light chain variable region having the amino acid sequence from Asp at position 21 to Lys at position 133 of the amino acid sequence of SEQ ID NO:10.

The introduction of mutation into a polypeptide is a method well known to those skilled in the art for producing a polypeptide that is functionally equivalent to a particular polypeptide. For example, as known to those skilled in the art, antibody that exhibits the activity equivalent to that of an antibody of the present invention can be produced by introducing suitable mutations into the antibody of the present invention using site-specific mutagenesis (Hashimoto-Gotoh, T. et al. (1995) Gene 152, 271-275; Zoller, M.J. and Smith, M. (1983) Methods Enzymol. 100, 468-500; Kramer, W. et al. (1984) Nucleic Acids Res. 12, 9441-9456; Kramer, W. and Fritz, H.J. (1987) Methods Enzymol. 154, 350-367; Kunkel, T.A. (1985) Proc. Natl. Acad. Sci. USA 82, 488-492; and Kunkel (1988) Methods Enzymol. 85, 2763-2766). Amino acid mutations may also be produced by natural mutation. The antibody of the present invention also encompasses antibody that has an amino acid sequence generated by one or more amino acid mutations in the amino acid sequence of an antibody of the present invention and that exhibits the activity equivalent to that of the antibody of the present invention. With regard to the number of amino acids that have been mutated in such a mutant, generally no more than 50 amino acids, preferably no more than 30 amino acids, and more preferably no more than 10 amino acids (for example, no more than 5 amino acids) can be considered.

Preferably, the amino acid residue is mutated to another amino acid residue that conserves the characteristics of the amino acid side chain. For example, the following classification has been established based on the characteristics of the amino acid side chain. hydrophobic amino acids (A, I, L, M, F, P, W, Y, V) hydrophilic amino acids (R, D, N, C, E, Q, G, H, K, S, T) amino acids having an aliphatic side chain (G, A, V, L, I, P) amino acids having a hydroxyl-containing side chain (S, T, Y) amino acids having a sulfur-containing side chain (C, M) amino acids having a carboxyl- or amide-containing side chain (D, N, E, Q) amino acids having a base-containing side chain (R, K, H) amino acids having an aromatic-containing side chain (H, F, Y, W)
(The single letter designation for the amino acids is given in the parentheses.)

In the case of a polypeptide having a modified amino sequence generated by deleting and/or adding one or a plurality of amino acid residues from and/or to a particular amino acid sequence and/or by substituting one or a plurality of amino acid residues in the particular amino sequence with another amino acid, it is already known that such a polypeptide can maintain its biological activity (Mark, D.F. et al., Proc. Natl. Acad. Sci. USA (1984) 81, 5662-5666; Zoller, M.J. and Smith, M., Nucleic Acids Research (1982) 10, 6487-6500; Wang, A. et al., Science 224, 1431-1433; Dalbadie-McFarland, G. et al., Proc. Natl. Acad. Sci. USA (1982) 79, 6409-6413). That is, in general, when, in the amino acid sequence of a particular polypeptide, the amino acids in a particular classification are substituted by other amino acids in that classification, there is a high probability that the activity of the particular polypeptide will be retained. Substitutions between amino acids in the same classification in the amino acid classification provided above are designated in the present invention as conservative substitutions.

In [1], supra, the present invention also provides antibody that binds to an epitope that is the same as the epitope bound by anti-HB-EGF antibody disclosed by the present invention. Such an antibody can be obtained, for example, by the following method.

Whether a test antibody and a particular antibody have a common epitope can be determined by competition by the two for the same epitope. Competition between antibodies can be detected, for example, by a reciprocal blocking assay. For example, a competitive ELISA assay is a preferred reciprocal blocking assay. In specific terms, in a reciprocal blocking assay, HB-EGF protein is coated on the wells of a microtiter plate; pre-incubated in the presence or absence of the candidate competitive antibody; then the anti-HB-EGF antibody of the present invention is added. The amount of anti-HB-EGF antibody of the present invention that has become bound to the HB-EGF protein in the well is indirectly correlated with the binding activity of the candidate competitive antibody (test antibody) competing for binding to the same epitope. That is, the higher the affinity of the test antibody for the same epitope, the less anti-HB-EGF antibody of the present invention that binds to the HB-EGF protein-coated well and the greater the amount of binding by the test antibody to the HB-EGF protein-coated well.

The amount of well-bound antibody can be conveniently measured by labeling the antibody in advance. For example, biotin-labeled antibody can be measured using an avidin-peroxidase conjugate and a suitable substrate. A reciprocal blocking assay based on an enzyme label such as peroxidase is in particular known as a competitive ELISA assay. The antibody can be labeled with some other label that can be detected or measured. In specific terms, radioactive labels and fluorescent labels are also known.

In addition, when the test antibody has a constant region originating from a species different from that for the anti-HB-EGF antibody of the present invention, the amount of well-bound antibody can also be measured using a labeled secondary antibody that recognizes the constant region of the antibody. Or, even when the antibody originates in the same species but the classes are different, the amount of well-bound antibody can be measured using a secondary antibody that discriminates among the individual classes.

When - in comparison to the binding activity obtained in the control test that is carried out in the absence of the candidate competitive antibody - the candidate antibody can block binding of at least 20%, preferably at least 20 to 50%, and even more preferably at least 50% of the anti-HB-EGF antibody, such a candidate competitive antibody is then an antibody that binds to substantially the same epitope as the anti-HB-EGF antibody of the present invention or that competes for binding to the same epitope.

Binding activity by antibody

Known procedures can be used to measure the antigen binding activity of an antibody (Antibodies: A Laboratory Manual. Ed Harlow, David Lane, Cold Spring Harbor Laboratory, 1988). For example, an enzyme-linked immunosorbent assay (ELISA), enzyme immunoassay (EIA), radioimmunoassay (RIA), or immunofluorescence procedure can be used. The method described on pages 359 to 420 of Antibodies: A Laboratory Manual is an example of a procedure for measuring the binding activity by an antibody for antigen expressed in a cell.

In addition, procedures that in particular employ a flow cytometer can be suitably used to measure binding between antigen expressed on the surface of cells suspended in, for example, buffer, and antibody against the antigen. Examples of usable flow cytometers are as follows: FACSCanto™ II, FACSAria™, FACSArray™, FACSVantage™ SE, and FACSCalibur™ (the preceding instruments are from BD Biosciences), and EPICS ALTRA HyPerSort, Cytomics FC 500, EPICS XL-MCL ADC EPICS XL ADC, and Cell Lab Quanta / Cell Lab Quanta SC (the preceding instruments are from Beckman Coulter).

In one example of a convenient method for measuring the binding activity of a test HB-EGF antibody for an antigen, the test antibody is reacted with a cell that expresses HB-EGF, and stained with FITC-labeled secondary antibody that recognizes the test antibody. The fluorescent intensity is measured with FACSCalibur (Becton, Dickinson and Company) and analyzed with CELL QUEST software (Becton, Dickinson and Company) .

Proliferation inhibiting activity

The following methods are conveniently used to evaluate or measure the cell proliferation inhibiting effect due to anti-HB-EGF antibody. In a method that can be used to evaluate or measure the cell proliferation inhibiting activity *in vitro*, the uptake by live cells of [$^3$H]-labeled thymidine added to the medium is measured as an index of the DNA replication ability. Methods that are more convenient include the MTT method and dye exclusion methods in which the ability of cells to exclude a dye (e.g., trypan blue) is measured using a microscope. The MTT method utilizes the fact that live cells have the ability to convert the tetrazolium salt MTT (3-(4,5-dimethylthiazol-2-yl)-2,5-diphenyl tetrazolium bromide) into a blue formazan product. More specifically, the ligand and test antibody are added to the culture fluid of the test cell and, after a specified time has passed, an MTT solution is added to the culture fluid and MTT is incorporated into the cells by standing for a specified period of time. As a result, MTT, which is a yellow compound, is converted into a blue compound by succinate dehydrogenase in the mitochondria within the cells. The blue product is dissolved to provide coloration, and measurement of its absorbance provides an index to the viable cell count. In addition to MTT, reagents such as MTS, XTT, WST-1, WST-8, and so forth are also commercially available (Nacalai Tesque, Inc.) and can be suitably used. In the activity measurement, a control antibody is used in the same way as the anti-HB-EGF antibody; the control antibody is a binding antibody that has the same isotype as the anti-HB-EGF antibody while not having the aforementioned cell proliferation inhibiting activity. The antibody has the cell proliferation inhibiting activity when the anti-HB-EGF antibody exhibits a stronger cell proliferation inhibiting activity than the control antibody.

Tumor-supporting mouse models may also be used as a method for evaluating or measuring the cell proliferation inhibiting activity *in vivo.* For example, cancer cells whose growth is promoted by HB-EGF may be subcutaneously or intracutaneously grafted into a nonhuman test animal, after which the test antibody may be administered intravenously or intraabdominally every day or on a multiday interval beginning on the day of grafting or on the next day. The cell proliferation inhibiting activity can be evaluated by measuring tumor size with elapsed time. Just as with the *in vitro* evaluation, a control antibody having the same isotype is administered, and the antibody has a cell proliferating inhibiting activity when the tumor size in the group receiving the anti-HB-EGF antibody is significantly smaller than the tumor size in the group receiving the control antibody. The nude (nu/nu) mouse is suitably employed when the mouse is used as the nonhuman test animal; the nude (nu/nu) mouse lacks T-lymphocyte function due to the genetic loss of the thymus gland. The use of this type of mouse makes it possible to exclude a contribution by T-lymphocytes in the test animal in the evaluation or measurement of the cell proliferation inhibiting activity due to the administered antibody.

The method of inhibiting cell proliferation

The present invention provides a method of inhibiting the proliferation of HB-EGF-expressing cells by bringing such cells into contact with the antibody of the present invention. The antibody of the present invention, which is present in the cell proliferation inhibitor of the present invention, is an HB-EGF protein-binding antibody as has been described above. There are no particular limitations on the cells that may be brought into contact with the anti-HB-EGF antibody other than that these cells express HB-EGF, but disease-related cells are preferred. Cancer cells are a preferred example of the disease-related cells. The cancer is preferably pancreatic cancer, liver cancer, esophageal cancer, melanoma, colorectal cancer, gastric cancer, ovarian cancer, uterine cervical cancer, breast cancer, bladder cancer, a brain tumor, or a hematological cancer. The hematological cancers include, for example, myelomas, lymphomas, and leukemias.

The delivery method using anti-HB-EGF antibody

The present invention relates to a method of delivering a cytotoxic substance into a cell using anti-HB-EGF antibody. The antibody used in this method is the cytotoxic activity-conjugated anti-HB-EGF antibody that has been described above. Delivery of the cytotoxic substance can be achieved by bringing HB-EGF-expressing cells into contact with the cytotoxic substance-conjugated anti-HB-EGF antibody. There are no particular limitations in the present invention on the cells to which the cytotoxic substance is delivered, but disease-related cells are preferred. Cancer cells are an example of the disease-related cells. The cancer is preferably pancreatic cancer, liver cancer, esophageal cancer, melanoma, colorectal cancer, gastric cancer, ovarian cancer, uterine cervical cancer, breast cancer, bladder cancer, a brain tumor, or a hematological cancer. The hematological cancers include, for example, myelomas, lymphomas, and leukemias.

Contact in the present invention may be carried out *in vitro* or *in vivo.* With regard to the state in which the antibody is added here, for example, a solid obtained by freeze-drying or a solution may suitably be used. In those instances where the antibody is added in the form of the aqueous solution, this may be an aqueous solution that contains only the pure antibody or may be a solution that contains, for example, surfactant, excipient, colorant, flavorant, preservative, stabilizer, buffer, suspending agent, tonicity agent, binder, disintegrant, lubricant, fluidity promoter, taste-masking agent, and so forth. While there are no particular limitations on the concentration of addition, suitable final concentrations in the culture fluid are preferably 1 pg/mL to 1 g/mL, more preferably 1 ng/mL to 1 mg/mL, and even more preferably 1 $\mu$g/mL to 1 mg/mL.

*in vivo* "contact" may also be carried out in the present invention by administration to a non-human animal into which HB-EGF-expressing cells have been implanted, transplanted, or grafted, or by administration to an animal that bears HB-EGF-expressing cancer cells. The mode of administration may be oral administration or parenteral administration. Parenteral administration is particularly preferred, and the corresponding routes of administration may include injection, transnasal administration, transpulmonary administration, transdermal administration, and so forth. With regard to examples of administration by injection, the pharmaceutical composition of the present invention, as a cell proliferation inhibitor or anti-cancer agent, can be administered systemically or locally by, for example, intravenous injection, intramuscular injection, intraperitoneal injection, or subcutaneous injection. The appropriate mode of administration can be selected as a function of the age and symptomatology of the animal subject. In those instances where an aqueous solution is administered, this solution may be an aqueous solution that contains only the pure antibody or may be a solution that contains, for example, surfactant, excipient, colorant, flavorant, preservative, stabilizer, buffer, suspending agent, tonicity agent, binder, disintegrant, lubricant, fluidity promoter, taste-masking agent, and so forth. The dosage, for example, may be selected from the range of 0.0001 mg to 1000 mg per 1 kg body weight per administration. Alternatively, the dosage may be selected from the range of 0.001 to 100000 mg/body per patient. However, the dosage of the antibody of the present invention is not limited to the preceding dosages.

The pharmaceutical composition

In another aspect, a characteristic feature of the present invention is a pharmaceutical composition that comprises an antibody that binds to HB-EGF protein. An additional characteristic feature of the present invention is a cell proliferation inhibitor, and particularly an anti-cancer agent, that comprises an antibody that binds to HB-EGF protein. The cell proliferation inhibitor of the present invention and the anti-cancer agent of the present invention are preferably administered to a subject suffering from cancer or to a subject at risk for cancer.

In the present invention, the cell proliferation inhibitor comprising HB-EGF protein-binding antibody also subsumes a method of inhibiting cell proliferation comprising a step of administering HB-EGF protein-binding antibody to a subject as well as the use of HB-EGF protein-binding antibody for the production of a cell proliferation inhibitor.

Moreover, in the present invention, the anti-cancer agent comprising HB-EGF protein-binding antibody subsumes a method of preventing or treating cancer comprising a step of administering HB-EGF protein-binding antibody to a subject as well as the use of HB-EGF protein-binding antibody for the production of an anti-cancer agent.

There are no particular limitations on the antibody present in the pharmaceutical composition of the present invention (for example, a cell proliferation inhibitor or an anti-cancer agent; this also applies below) other than that this antibody has the ability to bind to HB-EGF protein, and any of the antibodies provided herein as examples may also be used.

The mode of administration of the pharmaceutical composition of the present invention may be oral administration or parenteral administration. Parenteral administration is particularly preferred, and the corresponding routes of administration may include injection, transnasal administration, transpulmonary administration, transdermal administration, and so forth. With regard to examples of administration by injection, the pharmaceutical composition of the present invention can be administered systemically or locally by, for example, intravenous injection, intramuscular injection, intraperitoneal injection, or subcutaneous injection. The appropriate mode of administration can be selected as a function of the age and symptomatology of the patient. The dosage, for example, may be selected from the range of 0.0001 mg to 1000 mg per 1 kg body weight per administration. Alternatively, the dosage may be selected from the range of 0.001 to 100000 mg/body per patient. However, the pharmaceutical composition of the present invention is not limited to the preceding dosages.

The pharmaceutical composition of the present invention can be formulated according to the usual methods (for example, Remington's Pharmaceutical Science, latest edition, Mack Publishing Company, Easton, USA) and may comprise a pharmaceutically acceptable vehicle and pharmaceutically acceptable additives. Examples are surfactants, excipients, colorants, flavorants, preservatives, stabilizers, buffers, suspending agents, tonicity agents, binders, disintegrants, lubricants, fluidity promoters, taste-masking agents, and so forth, but there is no limitation to the preceding and other generally used vehicles can be employed as appropriate. Specific examples are light silicic anhydride, lactic acid, crystalline cellulose, mannitol, starch, carmellose calcium, carmellose sodium, hydroxypropyl cellulose, hydroxypropylmethyl cellulose, polyvinyl acetal diethylamino acetate, polyvinylpyrrolidone, gelatin, medium-chain fatty acid triglycerides, polyoxyethylene hardened castor oil 60, sucrose, carboxymethyl cellulose, corn starch, inorganic salts, and so forth.

The method of producing a pharmaceutical product

The present invention additionally provides a method of producing a pharmaceutical product and particularly an anti-cancer agent, comprising the steps of:

(a) providing anti-HB-EGF antibody;
(b) determining whether the antibody of (a) has an internalizing activity;
(c) selecting antibody that has an internalizing activity; and
(d) attaching a cytotoxic substance to the antibody selected in (c).

The presence/absence of an internalizing activity can be determined by the methods described above. In addition, the anti-HB-EGF antibody and the cytotoxic substance can be the anti-HB-EGF antibody and the cytotoxic substance already described above.

Cancer diagnosis

Based on the fact that HB-EGF expression increases in a broad range of cancers, such as pancreatic cancer, liver cancer, esophageal cancer, melanoma, colorectal cancer, gastric cancer, ovarian cancer, uterine cervical cancer, breast cancer, bladder cancer, brain tumors, and hematological tumors, the present invention provides in another of its aspects a method of diagnosing a disease, and particularly a method of diagnosing cancer, using anti-HB-EGF antibody.
The diagnostic method of the present invention can be carried out through detection of the anti-HB-EGF antibody that has become incorporated within a cell. The anti-HB-EGF antibody used in the present invention preferably has an internalizing activity and is preferably labeled with a labeling substance.
Accordingly, a preferred embodiment of the diagnostic method of the present invention is a diagnostic method that employs anti-HB-EGF antibody that has been labeled with a labeling substance and that has an internalizing activity. The abovementioned anti-HB-EGF antibody can be used for an anti-HB-EGF antibody to be bound to the labeling substance.
The labeling substance attached to the anti-HB-EGF antibody is not particularly limited, and those labeling substances known to those skilled in the art can be used, for example, fluorescent dyes, enzymes, co-enzymes, chemiluminescent substances, radioactive substances, and so forth. Specific examples are radioisotopes (e.g., 32P, 14C, 125I, 3H, 131I, and so forth), fluorescein, rhodamine, dansyl chloride, umbelliferone, luciferase, peroxidase, alkali phosphatase, β-galactosidase, β-glucosidase, horseradish peroxidase, glucoamylase, lysozyme, saccharide oxidase, microperoxidase, biotin, and so forth. When biotin is used as the labeling substance, the addition of the biotin-labeled antibody is preferably followed by the addition of avidin attached to an enzyme such as alkali phosphatase. Known methods can be used to attach the labeling substance to the anti-HB-EGF antibody, for example, the glutaraldehyde method, maleimide method, pyridyl disulfide method, periodic acid method, and so forth. The labeling substance may be attached to the antibody by procedures known to those skilled in the art.
There are no particular limitations on the type of cancer when cancer is the disease being diagnosed by the method of the present invention, but the cancer is preferably pancreatic cancer, liver cancer, esophageal cancer, melanoma, colorectal cancer, gastric cancer, ovarian cancer, uterine cervical cancer, breast cancer, bladder cancer, a brain tumor, or a hematological cancer. The hematological cancers include, for example, myelomas, lymphomas, and leukemias.
Diagnosis in the present invention may be carried out *in vivo* or *in vitro.*
The *in vitro* diagnosis may be carried out, for example, according to a method comprising the steps of:

(a) providing a sample collected from a subject;
(b) bringing the sample from (a) into contact with anti-HB-EGF antibody to which a labeling substance is attached; and
(c) detecting the antibody that has become incorporated within cells.

There are no particular limitations on the sample that is collected, and may include cells collected from the subject and tissue collected from the subject. The sample used in the present invention also encompasses secondary samples that have been obtained from the test sample, for example, a cell culture fluid or a specimen prepared by fixing tissue or cells collected from the body of a living organism.
The *in vivo* diagnosis may be carried out, for example, according to a method comprising the steps of:

(a) administering labeled anti-HB-EGF antibody to a subject; and
(b) detecting the antibody that has become incorporated within cancer cells.

The dosage of the anti-HB-EGF antibody can be set as appropriate by those skilled in the art based on, for example, the type of labeling substance, the disease to be diagnosed, and so forth. The labeled anti-HB-EGF antibody may be formulated using the methods already described above.
The present invention additionally provides a method of producing a diagnostic reagent, and particularly a diagnostic reagent for cancer, comprising the steps of:

(a) providing anti-HB-EGF antibody;
(b) determining whether the antibody of (a) has an internalizing activity;
(c) selecting antibody that has an internalizing activity; and
(d) binding a labeling substance to the antibody selected in (c).

The presence/absence of the internalizing activity can be determined by the methods already described above.

In addition, the anti-HB-EGF antibody and the labeling substance can be the anti-HB-EGF antibody and the labeling substance already described above.

The contents of all the patents and reference literature explicitly cited in the specification are herein incorporated by reference in their entirety.

EXAMPLES

The present invention is described in greater detail by the examples provided below, but the present invention is not limited by these examples.

Immunization

1-1. Immunogen production

1-1-1. Construction of an HB-EGF expression vector

In order to construct an HB-EGF expression vector, an HB-EGF gene was first cloned as described below. Using human heart cDNA (human Marathon Ready cDNA, Clontech Laboratories, Inc.) as template, RT-PCT was carried out using Pyrobest Taq polymerase (Takara Bio Inc.) and the full-length HG-EGF gene was cloned.

EGF-1: ATGAAGCTGCTGCCGTCGGTG (SEQ ID NO: 13)
EGF-2: TCAGTGGGAATTAGTCATGCCC (SEQ ID NO: 14)
(94°C/30 s, 65°C/30 s, 72°C/60 s: 35 cycles)

Using the obtained PCR product as template, PCR was carried out for the second time under the conditions given below and a full-length HB-EGF cDNA fragment was obtained in which SalI and NotI cleavage sequences were added, respectively, at the 5' and 3' terminals.

EGF-3: TAAGTCGACCACCATGAAGCTGCTGCCGTCGGTG (SEQ ID NO: 15)
EGF-4: TTTGCGGCCGCTCACTTGTCATCGTCGTCCTTGTAGTCGTGGGAAT TAGTCATGCCCAAC (SEQ ID NO: 16)
(94°C/30 s, 65°C/30 s, 72°C/60 s: 25 cycles)

The fragment was digested with SalI and NotI and was inserted into an expression vector for use with animal cells (pMCN) that had likewise been digested with SalI and NotI, thus constructing an HB-EGF expression vector (pMCN_HB-EGF).

1-1-2. Construction of an HB-EGF_Fc fusion protein expression vector

A fusion protein (HB-EGF_Fc) between the extracellular domain of HB-EGF and the Fc region of mouse IgG2a was used as the immunogen for acquisition of HB-EGF neutralizing antibody. The structure of the immunizing fusion protein is shown in Figure 1.

The expression vector for the mouse Fc region/HB-EGF fusion protein was constructed as described below. First, using the HB-EGF expression vector (pMCN_HB-EGF) as template, PCR was carried out under the following conditions using Pyrobest Taq polymerase (Takara Bio Inc.).

EGF-5: AAAGAATTCCACCATGAAGCTGCTGCCGTC (SEQ ID NO: 17)
EGF-6: TATCGGTCCGCGAGGTTCGAGGCTCAGCCCATGACACCTC (SEQ ID NO: 18)
(94°C/30 s, 68°C/30 s, 72°C/30 s: 25 cycles)

The obtained PCR product was then digested with EcoRI and CpoI. The resulting DNA fragment was inserted between EcoRI and CpoI in an animal cell expression vector that contained mouse IgG2a_Fc (pMCDN_mIgG2a_Fc) to construct an HB-EGF-Fc expression vector (pMCDN_HB-EGF-Fc).

1-1-3. Creation of an HB-EGF_Fc-producing strain

15 μg of the HB-EGF-Fc expression vector pMCDN_HB-EGF-Fc, which had been linearized by digestion with pvuI, was transfected by electroporation at 1.5 kV, 25 μF (Gene Pulser from Bio-Rad Laboratories, Inc.) into DG44 cells (1 x 10⁷ cells/mL, 800 μL) suspended in PBS(-). After dilution to a suitable cell count with a growth medium (CHO-S-SFM II, Invitrogen Corporation) containing penicillin/streptomycin (PS), the cells were seeded to 96-well plates and 500 μg/mL G418 (geneticin, Invitrogen Corporation) was added the next day. After about 2 weeks, wells having a monoclone were selected under a microscope and SDS-PAGE was run using 10 μL of the culture supernatant from each. Cell lines producing HB-EGF-Fc were screened by Western blotting using a PVDF membrane and goat anti-HB-EGF antibody (AF-259-NA, R&D Systems, Inc.) and HRP-anti-goat antibody (ACI3404, BioSource). The highest producing strain was selected and subjected to expansion culture.

1-1-4. Purification of the HB-EGF_Fc protein

The HB-EGF_Fc protein was purified from the culture supernatant of the obtained HB-EGF_Fc-producing strain using a Hi Trap Protein G HP 1 mL column (Amersham Biosciences #17-0404-01). The culture supernatant was adsorbed at a flow rate of 1 mL/min followed by washing with 20 mL 20 mM phosphate buffer (pH 7.0) and then elution with 3.5 mL 0.1 M glycine-HCl (pH 2.7). The eluate was recovered in 0.5 mL fractions in Eppendorf tubes, each of which already contained 50 $\mu$L 1 M Tris-HCl (pH 9.0). The $OD_{280nm}$ was measured. The fractions containing the target protein were combined and PBS(-) was added to bring to a total of 2.5 mL, then the buffer was replaced with PBS(-) using a PD-10 column (Amersham Biosciences #17-0851-01). The purified protein was passed through a 0.22 $\mu$m filter (Millipore #SLGV033RS) and was stored at 4°C.

1-2. Immunization

An emulsion of the HB-EGF_Fc protein was prepared with Complete Adjuvant (DIFCO DF263810) for the initial immunization and with Incomplete Adjuvant (DIFCO DC263910) for the second and subsequent immunizations. Three animals [(MRL/lpr, male, age: 4 weeks)(balb/c, female, age: 6 weeks), both purchased from Charles River Japan ] were immunized by subcutaneous injection at 50 $\mu$g/mouse (1 mL Thermo syringe, 26-gauge needle). The second immunization was given two weeks after the initial immunization, and a total of 4-5 immunizations were given on a one week interval. For the final immunization, the HB-EGF_Fc (50 $\mu$g) was suspended in 100 $\mu$L PBS and was injected into the tail vein; cell fusion was carried out three days later.

1-3. Hybridoma production

Cell fusion was carried out as follows. The spleen was aseptically removed from the mouse and a single cell suspension was prepared by grinding in medium 1 (RPMI1640 + PS). The suspension was passed through a 70 $\mu$m nylon mesh (Falcon) to remove fatty tissue and so forth and the cells were counted. The obtained B cells were mixed with mouse myeloma cells (P3U1 cells) in a cell count ratio of about 2 : 1; 1 mL 50% PEG (Roche, cat # 783 641) was added; and cell fusion was carried out. The fused cells were suspended in medium 2 (RPMI1640 + PS, 10% FCS, HAT (Sigma, H0262), 5% BM Condimed H1 (Roche #1088947)), and distributed at 200 $\mu$L/well into a suitable number of 96-well plates (10 plates) and cultivated at 37°C. After one week, hybridoma were screened using the culture supernatant and analyzed. The hybridomas originating from two Balb/c mice were designated as the HA series and the HB series, respectively, and the hybridomas originating from one Mrl/lpr mouse were designated as the HC series.

Screening for anti-HB-EGF neutralizing antibody

2-1. Creation of human HB-EGF-expressing cell lines

2-1-1. Creation of the strain HB-EGF_DG44

An HB-EGF-expressing DG44 cell line was established as follows. First, 15 $\mu$g of the HB-EGF expression vector pMCN_HB-EGF constructed as described in 1-1-1 was digested with pvuI and was transfected into DG44 cells by electroporation using the same procedure as in 1-1-3. Then the G418-resistant strains were picked out and the cells were stained with goat anti-HB-EGF antibody (R&D Systems, Inc.) and FITC-labeled anti-goat IgG antibody. The HB-EGF expressed on the cell surface was analyzed with a FACSCalibur (Becton, Dickinson and Company) and the high-expressing clone was selected.

2-1-2. Creation of the strain HB-EGF_Ba/F3

A Ba/F3 cell line that expressed HB-EGF on the cell membrane was established as follows. It is known that the HB-EGF expressed on the cell membrane is processed by protease and cleaved into the culture medium. Therefore, an expression vector for proHB-EGF mutated at the protease cleavage site was first constructed.
Using pMCN-HB-EGF as template, separate PCRs were carried out using the following two sets of conditions and Pyrobest Taq polymerase (Takara Bio Inc.).
PCR reaction 1
EGF-3: TAAGTCGACCACCATGAAGCTGCTGCCGTCGGTG (SEQ ID NO: 15)
EGF-7: CGATTTTCCACTGTGCTGCTCAGCCCATGACACCTCTC (SEQ ID NO: 19)

(94°C/30 s, 68°C/30 s, 72°C/30 s: 20 cycles)

PCR reaction 2

EGF-8: TGGGCTGAGCAGCACAGTGGAAAATCGCTTATATACCTA (SEQ ID NO: 20)

EGF-4: TTTGCGGCCGCTCACTTGTCATCGTCGTCCTTGTAGTCGTGGGAAT TAGTCATGCCCAAC (SEQ ID NO: 16)

(94°C/30 s, 68°C/30 s, 72°C/30 s: 20 cycles)

The two DNA fragments obtained by PCR reactions 1 and 2 were then mixed; a recombination reaction (94°C/30 s, 72°C/60 s: 5 cycles) was run using Pyrobest Taq polymerase (Takara Bio Inc.); followed by PCR under the following conditions using 1 μL of the preceding reaction solution as template.

EGF-3: TAAGTCGACCACCATGAAGCTGCTGCCGTCGGTG (SEQ ID NO: 15)

EGF-4: TTTGCGGCCGCTCACTTGTCATCGTCGTCCTTGTAGTCGTGGGAAT TAGTCATGCCCAAC (SEQ ID NO: 16)

(94°C/30 s, 68°C/30 s, 72°C/60 s: 22 cycles)

The obtained PCR product was digested with SalI and NotI followed by insertion into an expression vector for use in animal cells (pMCN) that had likewise been digested with SalI and NotI, in order to construct a proHB-EGF expression vector (pMCN-MHB-EGF).

A Ba/F3 cell line that expressed proHB-EGF was then created as described in the following. 15 μg of the previously constructed proHB-EGF expression vector pMCN-MHB-EGF was cleaved with pvuI and then transfected by electroporation at 0.33 kV, 950 μF (Gene Pulser from Bio-Rad Laboratories, Inc.) into Ba/F3 cells suspended in PBS(-) (1 x 10^7 cells/mL, 800 μL). These cells were then cultured in 96-well plates on medium (RPMI1640, 10% FCS, PS) containing 1 ng/mL IL-3 and 500 μg/mL G418, and after two weeks the G418-resistant strains were picked out. The cells were stained with goat anti-HB-EGF antibody (R&D Systems, Inc.) and FITC-labeled anti-mouse IgG antibody (Beckman Coulter, PN IM0819) and the clone was selected that presented a high level of expression of cell surface HB-EGF according to FACS (Becton, Dickinson and Company).

2-2. Creation of HB-EGF-expressing SKOV-3 cells

A SKOV-3 cell line that expressed HB-EGF was established as described in the following. SKOV-3 (purchased from ATTC), which is an ovarian cancer cell line, was cultured on a growth medium (McCoy's 5A medium, Invitrogen Corporation) that contained 10% FCS and penicillin/streptomycin (P/S).

15 μg of the HB-EGF expression vector pMCN_HB-EGF constructed in 1-1-1 was digested with pvuI. This was followed by transfection by electroporation at 1.5 kV, 25 μF (Gene Pulser from Bio-Rad Laboratories, Inc.) into SKOV-3 cells suspended in PBS(-) (1 x 10^7 cells/mL, 800 μL). Dilution to a suitable cell count using the growth medium cited above was followed by seeding to 96-well plates. G418 (geneticin, Invitrogen Corporation) was added the next day at 500 μg/mL. After about two weeks the G418-resistant monoclones were selected and screened for HB-EGF-expressing cell lines by Western blotting. The highest producing line was selected and used in subsequent experiments.

2-3. Creation of an EGFR_Ba/F3 cell line that exhibits HB-EGF-dependent growth

2-3-1. Construction of pCV-hEGFR/G-CSFR

In order to evaluate the activity of antibody of the present invention, a vector was constructed that expressed a chimeric receptor (hEGFR/mG-CSFR) composed of the extracellular region of human EGFR and the intracellular region of mouse G-CSFR. The effect on a cell that expresses the chimeric receptor when HB-EGF binds to such a cell is shown schematically in Figure 2a.

In order to clone the gene encoding the extracellular region of the human epidermal growth factor receptor (EGFR), PCR was carried out with human liver cDNA (Marathon Ready cDNA, Clontech Laboratories, Inc.) as a template using the primer set specified below. The base sequence (MN_005228) and the amino acid sequence (NP_005219) of human EGFR are shown, respectively, in SEQ ID NO: 21 and SEQ ID NO: 22.

EGFR-1: ATGCGACCCTCCGGGACGGC (SEQ ID NO: 23)

EGFR-2: CAGTGGCGATGGACGGGATCT (SEQ ID NO: 24)

(94°C/30 s, 65°C/30 s, 72°C/2 min: 35 cycles)

The amplified cDNA (approximately 2 Kb) was excised from the agarose gel and was inserted into the pCR-TOPO vector (Invitrogen Corporation). The base sequence of the fragment inserted into this plasmid was analyzed and confirmed that the obtained EGFR gene had the correct sequence. PCR was then carried out with the plasmid obtained as above as a template using the following primer set.

EGFR-5: TTGCGGCCGCCACCATGCGACCCTCCGGGACGGC (SEQ ID NO: 25)

EGFR-6: ACCAGATCTCCAGGAAAATGTTTAAGTCAGATGGATCGGACGGGATCTTAG GCCCATTCGT (SEQ ID NO: 26)
(94°C/30 s, 68°C/30 s, 72°C/2 min: 25 cycles)

A gene fragment was obtained that encoded the EGFR extracellular region and that had a 5' NotI site and a 3' BglII site. This fragment was digested with NotI-BglII and inserted between NotI-BamHI in pCV_mG-CSFR.

The expression plasmid vector pCV was constructed by replacing the poly(A) addition signal of pCOS1 (International Publication No. WO 98/13388) with the poly(A) addition signal from human G-CSF. pEF-BOS (Mizushima S. et al., Nuc. Acids Res. 18, 5322 (1990)) was digested with EcoRI and XbaI to obtain the poly(A) addition signal fragment originating from human G-CSF. This fragment was inserted into pBacPAK8 (Clontech Laboratories, Inc.) at the EcoRI/XbaI sites. After digested with EcoRI, both terminals were blunted and digested with BamHI, resulted in the production of a fragment containing the poly(A) addition signal of human G-CSF origin having a BamHI site added at the 5' terminal and a blunted 3' terminal. This fragment was exchanged with the poly(A) addition signal of pCOS1 at the BamHI/EcoRV sites, giving the expression plasmid vector designated pCV.

pCV_mG-CSFR comprises the mouse G-CSF receptor from the asparagine residue at position 623 to the C terminal, which is the intracellular region, in pCV. The base sequence (M58288) of the mouse G-CSF receptor is shown in SEQ ID NO: 27 and the amino acid sequence (AAA37673) of the mouse G-CSF receptor is shown in SEQ ID NO: 28. However, the glycine reside at position 632 in SEQ ID NO: 28 is replaced by a glutamic acid residue due to the creation of a BamHI site (restriction enzyme site) in the coding cDNA sequence at the N-terminal region in the insertion sequence of pCV_mG-CSFR.

Construction of the vector pCV_hEGFR/mG-CSFR expressing the chimeric receptor hEGFR/mG-CSFR composed of the extracellular region of human EGFR and the intracellular region of mouse G-CSFR was completed by confirming the base sequence of the gene fragment inserted in pCV_mG-CSFR.

The base sequence and amino acid sequence for the protein expressed by the expression vector, i.e., a human EGFR/mouse G-CSFR chimeric receptor, are shown, respectively, in SEQ ID NO: 29 and SEQ ID NO: 30.

2-3-2. Creation of an HB-EGF-dependent cell line

15 µg of the hEGFR/mG-CSFR chimeric receptor expression vector pCV_ hEGFR/mG-CSFR, linearized by digestion with pvuI, was transfected by electroporation (Gene Pulser, Bio-Rad Laboratories, Inc.) at 0.33 kV, 950 µF into Ba/F3 cells. These cells were cultured for 2 weeks on medium (RPMI1640, 10% FCS, PS) containing 10 ng/mL HB-EGF and 500 µg/mL G418 and the emergent colony was picked up.

It was then determined in the following experiment if the obtained cell line exhibited growth dependent on the HB-EGF concentration. The EGFR_Ba/F3 cells were seeded to 96-well plates at $1 \times 10^3$ cells/well in the presence of 0 to 100 ng/mL HB-EGF (R&D Systems, Inc., 259-HE) followed by incubation for 3 days. Then the cell count was measured using the WST-8 reagent (Cell Counting Kit-8, Dojindo Laboratories) in accordance with the manufacturers instructions.

The results showed that growth of the established EGFR_Ba/F3 cell line was promoted in a manner dependent on the HB-EGF concentration (Figure 2b).

2-4. Hybridoma screening

2-4-1. Screening for HB-EGF-binding antibodies (primary screening)

In order to obtain anti-HB-EGF neutralizing antibodies, HB-EGF-binding antibodies was first screened. ELISA and FACS were used to screen for binding antibodies.

2-4-1-1. ELISA

The hybridoma culture supernatant was reacted by incubation for 1 hour in ELISA plates (NUNC) coated with 1 µg/mL HB-EGF protein (R&D Systems, Inc., 259-HE). This was followed by reaction for 1 hour with alkali phosphatase (AP)-labeled anti-mouse IgG (Zymed Laboratories, Inc., #62-6622), after which color development was brought about by the addition of 1 mg/mL substrate (Sigma, S0942-50TAB). The $OD_{405}$ was measured with a plate reader (Bio-Rad Laboratories, Inc.) and the ELISA-positive wells were selected.

2-4-1-2. FACS

The hybridoma culture supernatant was added to HB-EGF_Ba/F3 cells (approximately $1 \times 10^5$ cells) and incubated for 1 hour at 4°C. FITC-labeled anti-mouse IgG antibody (Beckman Coulter, PN IM0819) was then added and incubated for 30 minutes at 4°C. The binding activity to cell surface HB-EGF was then analyzed for each hybridoma culture supernatant by FACS (Becton, Dickinson and Company).

2-4-1-3. Limit dilution

Limit dilution (LD) was carried out in order to divide the clones exhibiting HB-EGF binding activity according to ELISA or FACS analysis into monoclones. The cell count in positive wells was measured, and seeding to 96-well plates was done so as to provide 3 cells/well. After incubation for approximately 10 days, the binding activity was again analyzed by ELISA or FACS on the culture supernatant in wells in which colonies had emerged. Using this series of procedures, five monoclones exhibiting HB-EGF binding activity were obtained in the HA series, four monoclones exhibiting HB-EGF binding activity were obtained in the HB series, and five monoclones exhibiting HB-EGF binding activity were obtained in the HC series.

2-4-1-4. Subtype determination

The antibody subtype was determined using IsoStrip (Roche #1,493,027). The hybridoma culture supernatant diluted 10 times with PBS (-) was used for subtype determination.

[Table 1]

Characteristics of the isolated antibodies

| mouse strain | clone ID | EXP.1 | | EXP.2 | | isotype |
| --- | --- | --- | --- | --- | --- | --- |
| | | ELISA (OD405) | FACS (GEO-mean) | ELISA (OD405) | FACS (GEO-mean) | |
| | no-mAb | | 19.1 | | 6.9 | |
| balb #1 | HA-1 | 0.40 | 17.1 | | | 2b |
| | HA-3 | 0.42 | 59.0 | | | 2a |
| | HA-9 | 4.00 | 18.1 | | 10.2 | 2b |
| | HA-10 | 2.68 | 17.7 | | | G1 |
| | HA-20 | 4.00 | 18.9 | | | G1 |
| balb #2 | HB-10 | 2.55 | 108.0 | | | 2a |
| | HB-13 | 1.42 | 21.2 | | | G1 |
| | HB-20 | 3.91 | 188.2 | 4.00 | 98.9 | 2a |
| | HB-22 | 1.34 | 450.4 | | | 2b |
| MRL #1 | HC-15 | | 594.1 | 4.00 | 233.8 | 2a |
| | HC-19 | | 65.1 | 0.06 | 41.7 | 2a |
| | HC-26 | | 149.2 | 0.05 | 60.6 | 2a |
| | HC-42 | | 47.5 | 0.05 | 40.5 | 2a |
| | HC-74 | | | 0.05 | 45.2 | 2a |

2-4-2. Antibody purification

The antibody was purified from 80 mL of the culture supernatant for the obtained monoclonal hybridoma using a HiTrap Protein G HP 1 mL column (Amersham Biosciences #17-0404-01). The hybridoma supernatant was adsorbed at a flow rate of 1 mL/min followed by washing with 20 mL 20 mM phosphate buffer (pH 7.0) and then elution with 3.5 mL 0.1 M glycine-HCl (pH 2.7). The eluate was recovered in 0.5 mL fractions in Eppendorf tubes, each of which already contained 50 $\mu$L 1 M Tris-HCL (pH 9.0). The $OD_{280nm}$ was measured. The fractions containing antibody were combined and PBS(-) was added to bring to a total of 2.5 mL, then the buffer was replaced to PBS(-) using a PD-10 column (Amersham Biosciences #17-0851-01). The purified antibody was passed through a 0.22 $\mu$m filter (Millipore #SLGV033RS) and the properties of the individual purified antibodies were investigated in detail as follows.

2-4-3. Analysis of the growth neutralizing activity in EGFR_Ba/F3 cells (secondary screening)

The neutralizing activity on the HB-EGF-dependent growth of EGFR_Ba/F3 cells was analyzed for each of the purified antibodies. EGFR_Ba/F3 cells were seeded to 96-well plates at $2 \times 10^4$ cells/well in the presence of HB-EGF (80 ng/mL) and the particular purified antibody was added at 0 to 200 ng/mL. After incubation for 3 days, the cell count was measured using WST-8 (Cell Counting Kit-8).

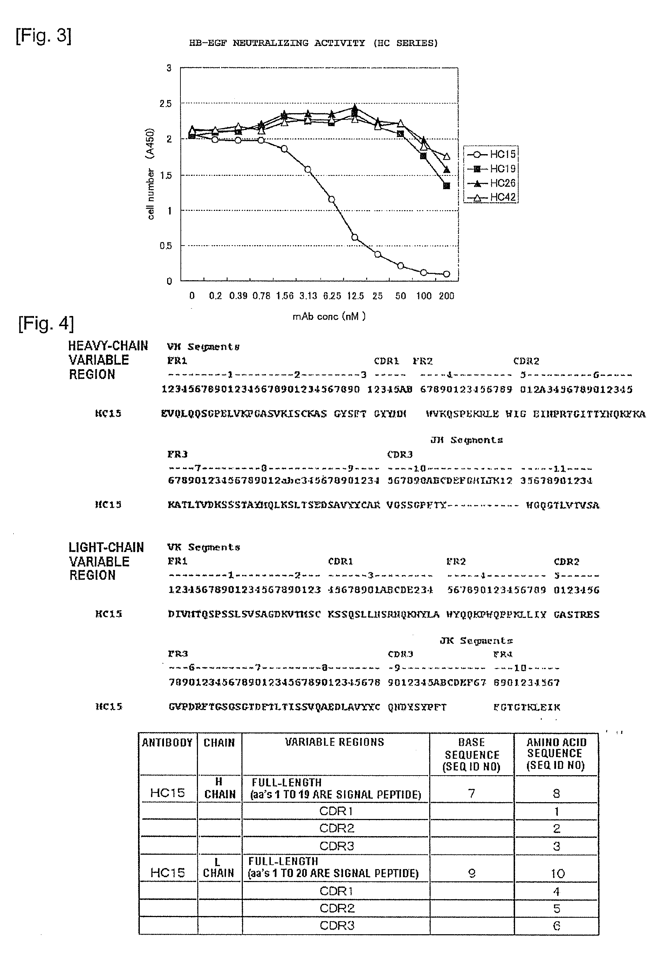

The results showed that HC-15 exhibits a strong neutralizing activity (Figure 3).

Analysis of the properties of HB-EGF neutralizing antibodies (HA-20, HB-20, HC-15)

3-1. Cloning of the variable region and determination of the amino acid sequence for HA-20, HB-20, and HC-15

The total RNA was purified using Trizol (#15596-018, Life Technologies) from approximately $5 \times 10^6$ hybridomas. Using a SMART RACE cDNA Amplification Kit (Clontech Laboratories, Inc., #PT3269-1), full-length cDNA synthesis was carried out according to the manual provided with the kit from 1 μg of the obtained total RNA. For each antibody, the gene encoding the variable region of the heavy chain (VH) and the variable region of the light chain (VL) was amplified using the obtained cDNA as template and an Advantage 2 PCR Enzyme System (Clontech Laboratories, Inc. #PT3281-1).
cloning primers for the light chain variable region UPM - k(VL-k)
UPM: provided with the kit
VL-k: GCT CAC TGG ATG GTG GGA AGA TG (SEQ ID NO: 31)
cloning primers for the heavy chain variable region HA-20: UPM - VH-G1
HB-20, HC-15: UPM - VH-2a
UPM: provided with the kit
VH-G1: GGG CCA GTG GAT AGA CAG ATG (SEQ ID NO: 32)
VH-2a: CAG GGG CCA GTG GAT AGA CCG ATG (SEQ ID NO: 33)
94°C/5 s, 72°C/2 min, 5 cycles
94°C/5 s, 70°C/10 s, 72°C/2 min, 5 cycles
94°C/5 s, 68°C/10 s, 72°C/2 min, 27 cycles
The gene fragments amplified in the preceding procedures were TA-cloned into pCRII-TOPO (Invitrogen TOPO TA-cloning Kit, #45-0640) and the base sequence for each insert was identified. The identified variable region sequences are shown in Figure 4.

3-2. Analysis of the binding activity for the active form of HB-EGF

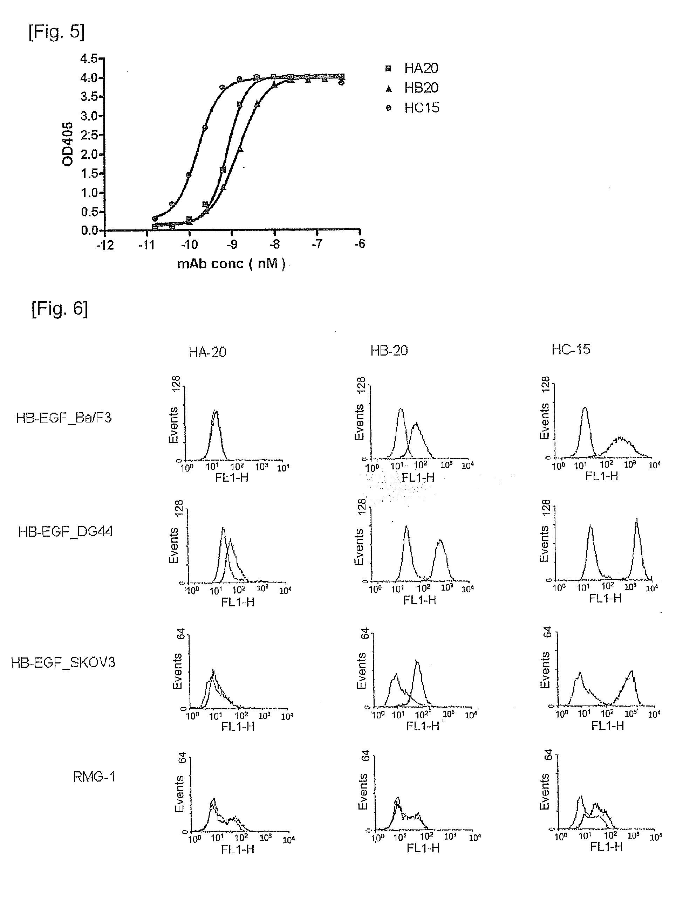

The following experiment was run in order to compare the ability of the thus obtained three antibodies (HA-20, HB-20, HC-15) to bind to active-form HB-EGF protein. The HA-20, HB-20, or HC-15 antibody was reacted at various concentrations in ELISA plates (NUNC) coated with 1 μg/mL HB-EGF protein (R&D Systems, Inc., 259-HE). This was followed by reaction for 1 hour with alkali phosphatase (AP)-labeled anti-mouse IgG (Zymed Laboratories, Inc., #62-6622), and addition of 1 mg/mL substrate (Sigma, S0942-50TAB) for color development. The OD405 was measured with a plate reader and the antibody concentration that gave 50% binding ($ED_{50}$) was calculated based on the binding curve obtained for the particular antibody. With regard to the binding activity for active-form HB-EGF, $ED_{50}$ values of 0.2 to 1.4 nM were observed and a strong binding activity was thus found to be present in all instances (Figure 5).
[Table 2]

$ED_{50}$ value for binding to HB-EGF for the antibodies HA-20, HB- 20, and HC-15

| mAb | HB-EGF binding | ($ED_{50}$, nmol/L) |
|---|---|---|
| HA-20 | | 0.8 |
| HB-20 | | 1.4 |
| HC-15 | | 0.2 |

3-3. Analysis of the binding activity for proHB-EGF

The binding activity for proHB-EGF was then analyzed for the obtained three antibodies. RMG1 cells (ovarian cancer cell line, purchased from the Japan Health Sciences Foundation), which are known to intrinsically express HB-EGF, were cultured on a growth medium (Ham's F12 medium, Invitrogen Corporation) containing 10% FCS. Each of the antibodies (10 μg/mL) was reacted for 1 hour at 4°C with the RMG1 cells, which intrinsically expressed HB-EGF, and the Ba/F3 cells (HB-EGF_Ba/F3), HB-EGF-expressing DG44 cells (HB-EGF_DG44), and SKOV-3 cells (HB-EGF_ SKOV-3), which were cells overexpressing HB-EGF, followed by staining with FITC-labeled anti-mouse IgG antibody (Beckman Coulter, PN IM0819). Binding to the cell surface HB-EGF was then analyzed by FACS (Becton, Dickinson and Company) for each antibody.
The histograms shown in Figure 6 compare the binding activity of the HA-20, HB-20, and HC-15 antibodies according to FACS analysis to the proHB-EGF intrinsically expressed in RMG1 cells and the proHB-EGF overexpressed in the Ba/F3, DG44, and SKOV-3 cells. The grey waveform shows the staining pattern in the absence of the primary antibody (control), while the staining pattern in the presence of the particular antibody is shown with a solid line. The horizontal axis shows the staining intensity and the vertical axis shows the number of cells. As shown in Figure 6, HB-20 and HC-15 recognized the HB-EGF overexpressed on the cell membrane and the HB-EGF intrinsically expressed on the cell membrane by the ovarian cancer line, while the HA-20 either did not bind at all or was bound only very weakly. These results showed that HA-20 was an antibody that, while strongly binding to active-form HB-EGF, did not recognize proHB-EGF.

3-4. Analysis of the neutralizing activity

3-4-1. Solid-phase analysis of the ability to inhibit EGFR/HB-EGF binding

3-4-1-1. Production of EGFR-Fc protein

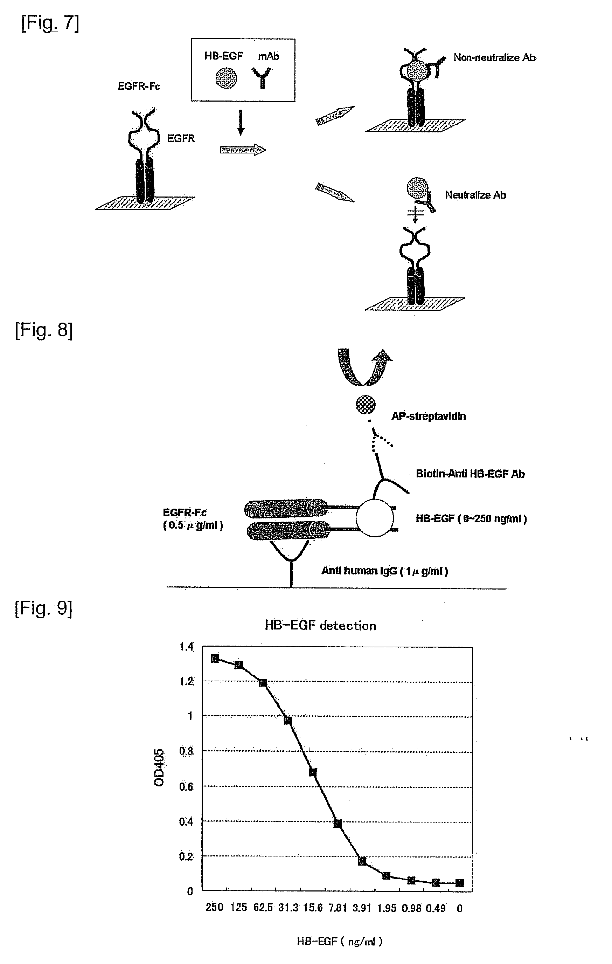

In order to construct an ELISA system that could check binding between HB-EGF and its receptor (EGFR) under solid phase conditions, a fusion protein (EGFR-Fc) from the extracellular region of EGFR and the Fc region of human IgG1 was first prepared to serve as the receptor protein. The mode of inhibition of binding between HB-EGF and EGFR by HB-EGF antibody on the solid phase are schematically illustrated in Figure 7.

An EGFR-Fc expression vector was first constructed. PCR was carried out using the following primers and using the pCV_hEGFR/mG-CSFR constructed in example 2-3-1 as the template.
EGFR-7: GTTAAGCTTCCACCATGCGACCCTCCGGGAC (SEQ ID NO: 34)
EGFR-8: GTTGGTGACCGACGGGATCTTAGGCCCATTCGTTG (SEQ ID NO: 35)
(94°C/30 s, 72°C/30 s: 25 cycles)
The amplified gene fragment coding for the extracellular region of EGFR was cleaved with BstEII and HindIII and was inserted between BstEII-HindIII in pMCDN2-Fc. The base sequence of the inserted gene fragment was confirmed to complete construction of a vector (pMCDN2_EGFR-Fc) expressing a fusion protein (EGFR-Fc) of the extracellular region of human EGFR and the Fc region of human IgG1. The base sequence and the amino acid sequence of the protein expressed by the expression vector, i.e., EGFR-Fc, are shown, respectively, in SEQ ID NO: 36 and SEQ ID NO: 37.

An EGFR-Fc protein-producing cell line was then established as follows. 15 $\mu$g of the EGFR-Fc expression vector pMCDN2_EGFR-Fc was first digested with pvuI and was then transfected by electroporation into DG44 cells. The EGFR-Fc protein produced in the culture supernatant of the G418-resistant strains was subsequently analyzed by Western blotting. Thus, 10 $\mu$L of the particular culture supernatant was separated by SDS-PAGE; blotted to a PVDF membrane; and the target protein was detected with HRP-labeled anti-human IgG antibody (Amersham, NA933V). The clone providing the highest production level was selected and run through expansion culture and the culture supernatant was recovered.

Purification of the EGFR-F protein was carried out as follows. The culture supernatant from the obtained EGFR-Fc-producing strain was adsorbed at a flow rate of 1 mL/min on a HiTrap Protein G HP 1 mL column (Amersham Biosciences #17-0404-01). After washing with 20 mL 20 mM phosphate buffer (pH 7.0), the protein was eluted with 3.5 mL 0.1 M glycine-HC1 (pH 2.7). To identify the fraction containing the target protein, 10 $\mu$L of each of the recovered fractions was separated by SDS-PAGE followed by Western blotting and staining with Coomassie Brilliant Blue. The buffer was replaced to PBS(-) using a PD-10 column (Amersham Biosciences #17-0851-01). The purified protein was passed through a 0.22 $\mu$m filter (Millipore #SLGV033RS) and was stored at 4°C.

3-4-1-2. Analysis of binding between HB-EGF and EGFR using ELISA

The purified EGFR-Fc was reacted at 0.5 $\mu$g/mL for 1 hour in ELISA plates coated with anti-human IgG antibody. 0 to 250 ng/mL HB-EGF (R&D Systems, Inc., 259-HE) was reacted for 1 hour, followed by detection of the HB-EGF protein bound to the EGFR-Fc with biotin-labeled anti-HB-EGF antibody (R&D Systems, Inc., BAF259) and AP-labeled streptavidin (Zymed, #43-8322). The model for analyzing the EGFR/HB-EGF binding mode using ELISA is shown in Figure 8. The results showed that HB-EGF binding to EGFR could be detected with the solid-phase system beginning at a concentration of about 4 ng/mL (Figure 9).

3-4-1-3. Analysis of the antibody-mediated inhibitory activity on HB-EGF/EGFR binding

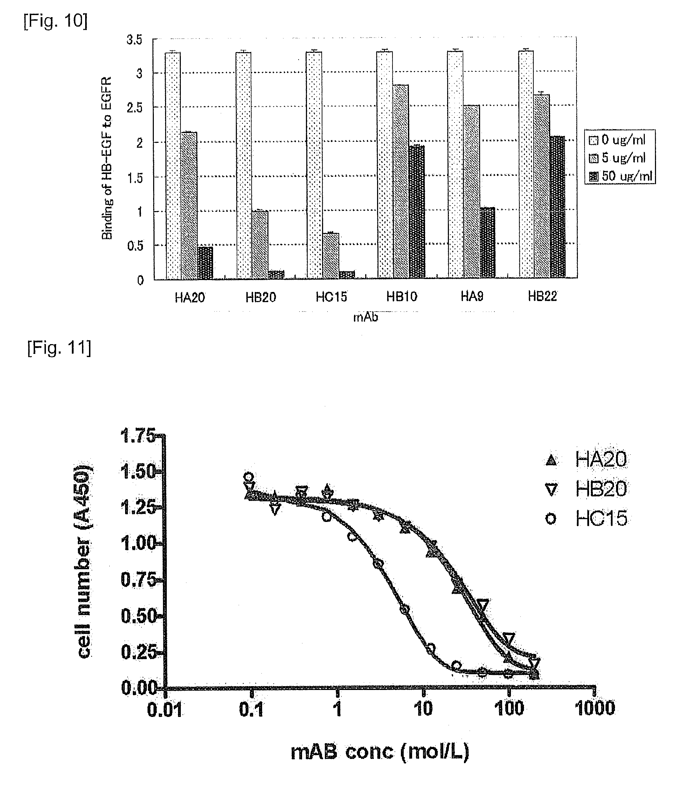

The solid-phase system described in the preceding was used to analyze the inhibitory activity on HB-EGF/EGFR binding by the antibodies obtained in 2-4-2. The individual antibody and HB-EGF (50 ng/mL) were added to ELISA plates on which EGFR-Fc had been immobilized and a reacted for one hour at room temperature. The plates were washed with TBS-T and the HB-EGF bound to the EGFR was detected by the previously described procedure (Figure 10).

A concentration-dependent activity to inhibit binding was observed for all the antibodies, and a particularly strong binding inhibition was recognized for HA-20, HB-20, and HC-15.

3-4-2. Growth inhibiting activity on EGFR_Ba/F3 cells

The neutralizing activity on the HB-EGF-dependent growth of EGFR_Ba/F3 cells was compared for HA-20, HB-20, and HC-15. As above, the EGFR_Ba/F3 cells were seeded to 96-well plates at $2 \times 10^4$ cells/well in the presence of HB-EGF (80 ng/mL) and the particular purified antibody was added. After cultivation for 3 days, the cell count was measured using WST-8 (Cell Counting Kit-8) and a growth curve was constructed. The antibody concentration at 50% of the maximum inhibitory effect ($EC_{50}$ value) was calculated based on the obtained results.

According to the results, the strongest growth inhibiting effect on EGFR_Ba/F3 cells was exhibited by HC-15 ($EC_{50}$ = 3.8 nM) followed by HA-20 ($EC_{50}$ = 32.6 nM) and HB-20 ($EC_{50}$ = 40.3 nM) (Figure 11).

[Table 3]

$ED_{50}$ values exhibited by HA-20, HB-20, and HC-15 antibodies for the growth-inhibiting effect on EGFR_Ba/F3 cells

|  | HA-20 | HB-20 | HC-15 |
|---|---|---|---|
| EC 50 (nM) | 32.6 | 40.3 | 3.8 |

3-4-3. Growth inhibiting activity for RMG-1 cells

The neutralizing activity on RMG-1 cells was analyzed as follows. RMG-1 cells ($6 \times 10^3$ cells/well) were seeded into Ham's F12 medium containing 8% or 2% FCS in 96-well plates and the particular antibody was then added. After cultivation for one week, the cell count was measured using the WST-8 reagent.

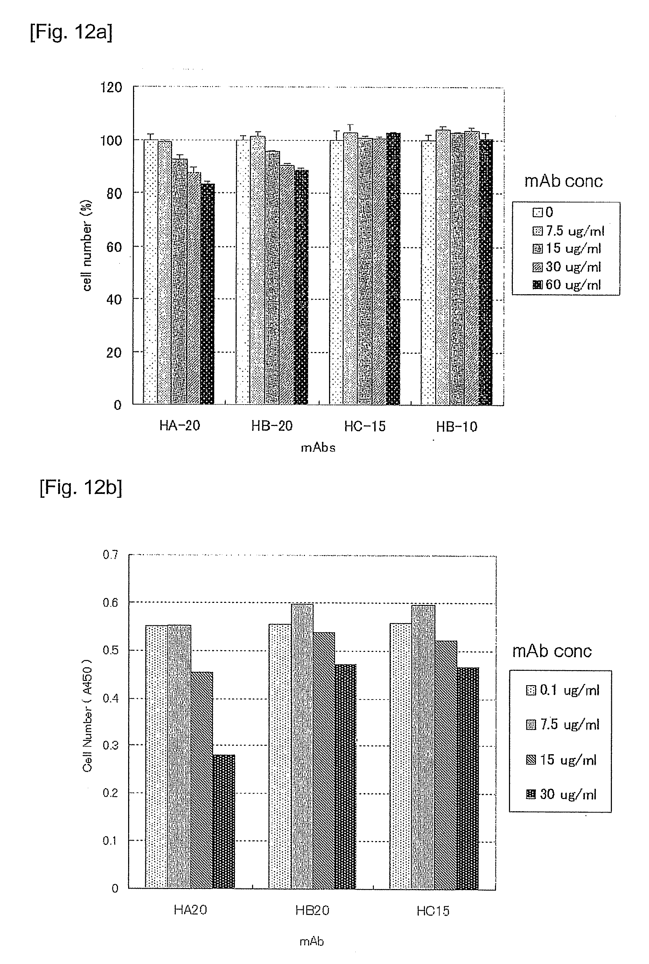

According to the result, HA-20 inhibited the growth of RMG-1 cells in an antibody concentration-dependent manner (Figure 12). The growth inhibiting activity was particularly significant at a 2% FCS concentration.

3-5. Analysis of the cytotoxicity mediated by the antibody's internalizing activity

3-5-1. System for evaluating the internalizing activity-mediated induction of cell death

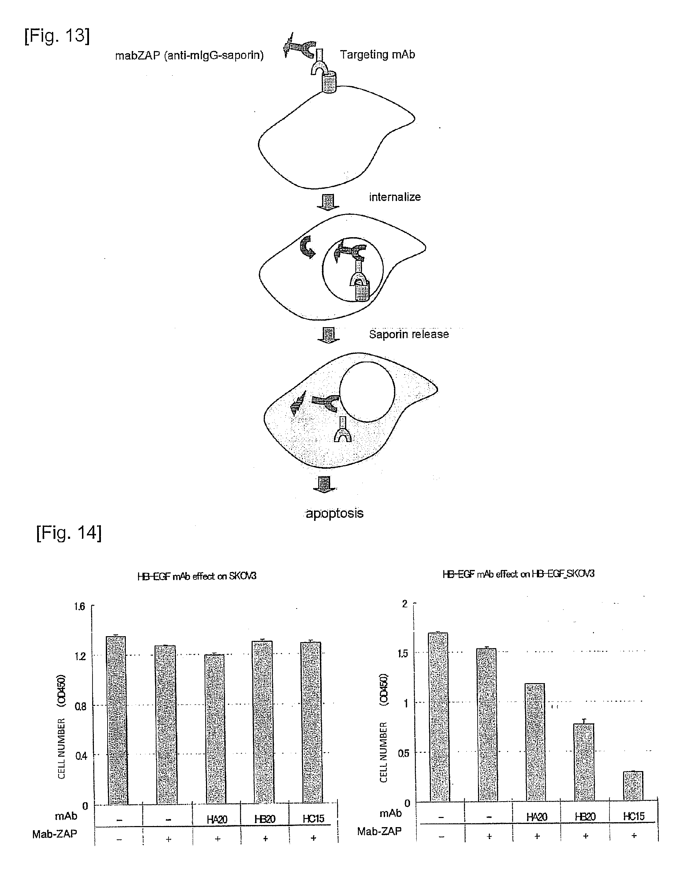

The activity to induce cell death through antibody internalization was evaluated using a saporin (toxin)-labeled anti-mouse IgG antibody (Mab-ZAP, Advanced Targeting Systems). An indirectly toxin-labeled antibody was first prepared by mixing the primary antibody and Mab-ZAP and reacting for 15 minutes at room temperature, and added to the target cells. When the added antibody was internalized into the cells, the Mab-ZAP was then also incorporated into the cells along with the primary antibody, resulted in the induction of cell death by the saporin released within the cell. This is shown schematically in Figure 13.

3-5-2. Internalization-mediated induction of cell death in a high HB-EGF-expressing cell line

The antibody was examined using HC-15 for its ability to induce cell death by the internalizing activity. SKOV-3 cells and HB-EGF_SKOV3 cells (SKOV-3 cells that overexpress HB-EGF) were seeded to 96-well plates at $2 \times 10^3$ cells/well. After culture overnight, Mab-ZAP was reacted, at 100 ng/well, with the obtained anti-HB-EGF antibody (100 ng/well) and added to the cells. A viable cell count was taken using WST-8 four days after antibody addition. In the case of the original SKOV-3 cells, which only weakly expressed HB-EGF, an ability to induce cell death was not seen for any of the antibodies; however, for the SKOV-3 cells that overexpressing HB-EGF, a cell death inducing activity in the presence of Mab-ZAP was seen for each antibody. In particular, a strong cell death inducing activity was seen for HB-20 and HC-15, which bind to proHB-EGF (Figure 14).

3-5-3. Internalization-mediated cell death induction in an ovarian cancer line

3-5-3-1. Analysis of cytotoxicity for an ovarian cancer line (ES-2)

3-5-3-1-1. Binding activity for ES-2 by individual antibodies

The ability to induce cell death in an ovarian cell line that intrinsically expresses HB-EGF (ES-2) was then investigated. ES-2 cells (ovarian cancer cell line, purchased from the ATCC) were cultured in a growth medium (McCoy's 5A medium, Invitrogen Corporation) containing 10% FCS and penicillin/streptomycin (P/S).

The ability of each antibody to bind to the cell surface of ES-2 cells was first analyzed using FACS. The cells were detached with 1 mM EDTA; the cells and the particular antibody (10 μg/mL) were reacted for 1 hour at 4°C in FACS buffer (PBS containing 2% FCS and 0.05% NaN$_3$); and stained with FITC-labeled anti-mouse IgG antibody (Beckman Coulter, PN IM0819) for 30 minutes at 4°C. The antibody binding to the HB-EGF expressed on the cell surface was analyzed using FACS (Becton, Dickinson and Company).

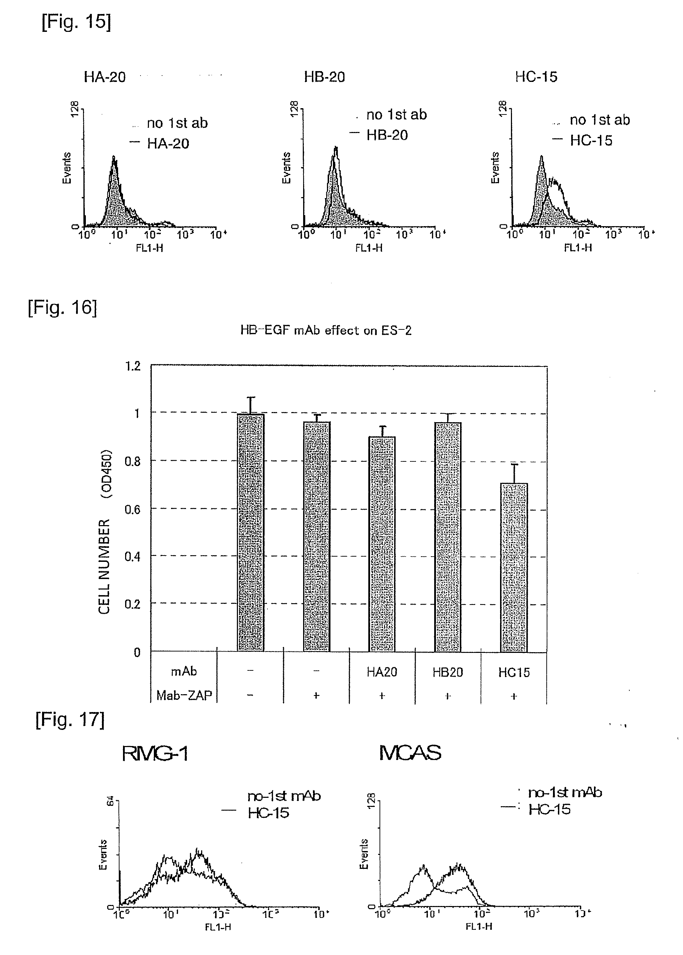

Figure 15 provides histograms that compare, via FACS analysis, the binding activity of the HA-20, HB-20, and HC-15 antibodies for ES-2 cells. The grey waveform shows the staining pattern in the absence of the primary antibody (control), while the staining pattern in the presence of the particular antibody is shown with a solid line. The horizontal axis shows the staining intensity and the vertical axis shows the number of cells. As shown in Figure 15, binding to the HB-EGF expressed on the cell membrane of ES-2 cells was detected in particular for HC-15.

3-5-3-1-2. Ability to induce cell death in ES-2

Internalization-mediated cytotoxicity of each antibody for ES-2 cells was investigated. ES-2 cells were seeded at 2 x 10$^3$ cells/well to 96-well plates. After culture overnight, the particular antibody (100 ng/well) and Mab-ZAP (100 ng/well) were reacted and added to the cells. After three days, the viable cell count was measured using WST-8. According to the results shown in Figure 16, a cell death inducing activity in the presence of Mab-ZAP was seen for HC-15, which exhibited the strongest HB-EGF binding activity.

3-5-3-2. Analysis of the ability to inhibit the growth of ovarian cancer lines (RMG-1, MCAS)

3-5-3-2-1. Binding activity of HC-15 for MCAS and RMG-1

The ability of the antibodies to inhibit growth was then investigated using separate ovarian cancer cell lines (RMG-1, MCAS). MCAS cells (purchased from JCRB) were cultured on a growth medium (Eagle's Minimal Essential Medium, Invitrogen Corporation) that contained 20% FCS.

In order to investigate whether and to what degree MCAS and RMG-1 express HB-EGF on the cell surface, FACS analysis was carried out using the HC-15 antibody. The cells were detached with 1 mM EDTA; the cells and the HC-15 antibody (10 μg/mL) were reacted for 1 hour at 4°C in FACS buffer (PBS containing 2% FCS and 0.05% NaN$_3$); and stained with FITC-labeled anti-mouse IgG antibody (Beckman Coulter, PN IM0819) for 30 minutes at 4°C. The antibody binding to the HB-EGF expressed on the cell surface was analyzed using FACS (Becton, Dickinson and Company).

Histograms are provided in Figure 17 that compare, via FACS analysis, the binding activity by the HC-15 antibody for RMG-1 and MCAS cells. It was revealed that HB-EGF was expressed on the cell surface of both the RMG-1 cells and the MCAS cells.

3-5-3-2-2. Analysis using the soft agar colony formation assay of the ability of the antibodies to inhibit the proliferation of RMG-1 and MCAS

The activity of the antibodies on the anchorage-independent proliferation of RMG-1 and MCAS cells was then investigated using the soft agar colony formation assay. The soft agar colony formation assay was carried out as described in the following.

MEM medium containing 0.6% agar (3:1 NuSieve, Cambrex) was added at 100 μL/well to each well in 96-well plates in order to prepare agar bottoms. The cells were then suspended at 8000 cells/well in medium containing 0.3% agar. Each test substance (antibody, Mab-ZAP) was mixed together with the cells into the agar; the preparation was dripped at 100 μL/well onto the agar bottoms. After cultivation at 37°C for from 3 weeks to 1 month, the emerged colonies were stained with 1% iodonitrotetrazolium chloride (Sigma, 18377) and the colonies were examined under a microscope.

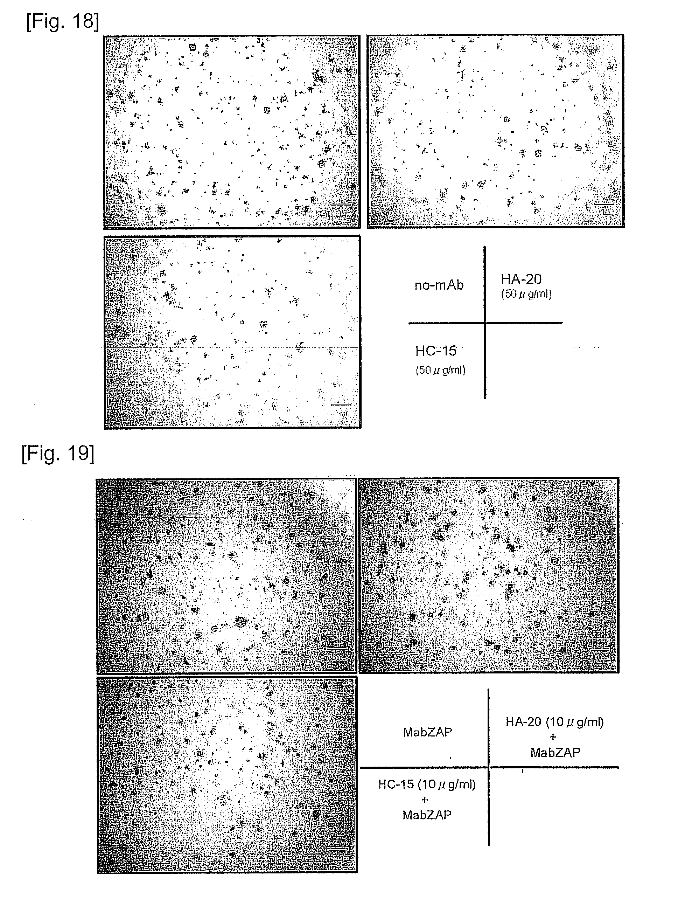

The action of individual antibodies (HA-20, HC-15) on colony formation by RMG-1 cells was first analyzed. The HA-20 or HC-15 antibody was mixed into the RMG-1 cells so as to provide 50 μg/mL, and the colonies formed in the agar were observed after approximately 3 weeks. According to the results, colony formation by the RMG-1 cells had been inhibited in the HC-15 antibody addition group in comparison to the non-addition group (Figure 18). It was thus found that the HC-15 antibody, just through its neutralizing activity alone, could inhibit anchorage-independent colony formation by RMG-1 cells.

The toxin-mediated activity to inhibit colony formation was then analyzed for the HA-20 and HC-15 antibodies. Mab-ZAP (1 μg/mL) was mixed into the agar together with the RMG-1 cells and MCAS cells and HA-20 or HC-15 antibody (10 μg/mL), and cultured for approximately 3 weeks to 1 month. The colonies formed was stained and observed by microscopy.

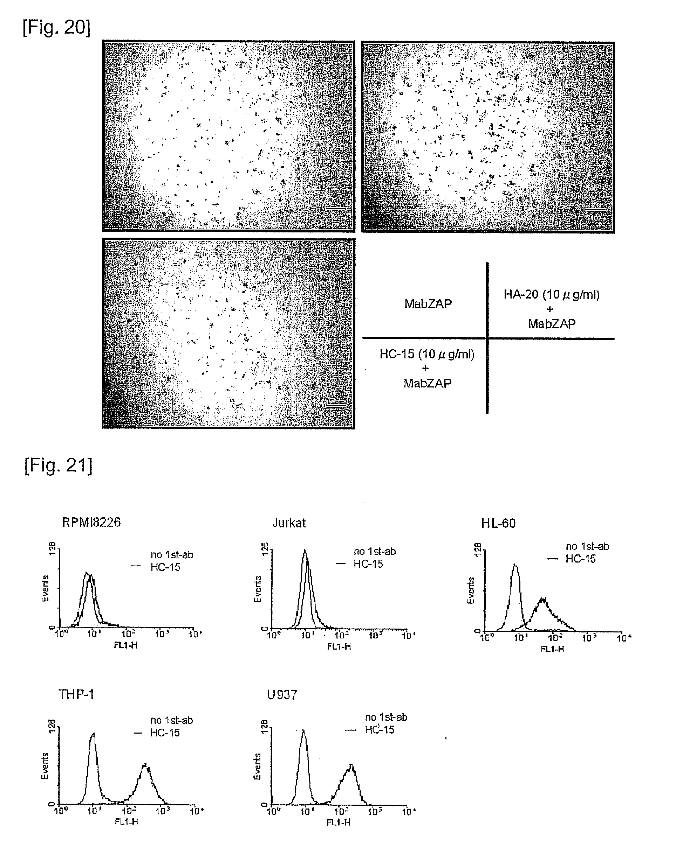

According to the results, an inhibition of colony formation was observed due to the simultaneous addition of HC-15 antibody and Mab-ZAP in both the RMG-1 cells (Figure 19) and the MCAS cells (Figure 20). Based on the preceding, it was demonstrated that the HC-15 antibody can inhibit the ability of ovarian cancer cells to form colonies not only through the exhibition of its neutralizing activity but also through the exhibition of an internalization activity.

3-5-4. Internalization-mediated cell death induction in hematological cancer lines

3-5-4-1. Analysis of HB-EGF expression by hematological cancer lines

It was then examined whether the internalization-mediated antitumor effect of HC-15 is also seen with hematological cancers. The following were cultured on RPMI1640 (Invitrogen Corporation) containing 10% FCS: RPMI8226 (multiple myeloma, purchased from the ATCC), Jurkat (acute T-cell leukemia, purchased from the ATCC), HL-60 (acute myeloid leukemia, purchased from JCRB), THP-1 (acute monocytic leukemia, purchased from JCRB), and U937 (monocytic leukemia, purchased from JCRB).

FACS analysis was carried out to investigate the expression of HB-EGF by these cells. The HC-15 (10 μg/mL) antibody was reacted with the particular cell line (2 x $10^5$ cells) for 60 minutes on ice, and stained with FITC-labeled anti-mouse IgG antibody (Beckman Coulter, PN IM0819). Binding by the antibody to the HB-EGF expressed on the cell surface was then analyzed by FACS (Becton, Dickinson and Company).

Histograms are given in Figure 21 that compare, based on FACS analysis, the expression of HB-EGF by the individual hematological cancer cell lines. THP-1 and U937 were shown to exhibit a particularly strong HB-EGF expression. In contrast, almost no expression was seen with Jurkat and RPMI8226.

3-5-4-2. Analysis of cytotoxicity for hematological cell lines

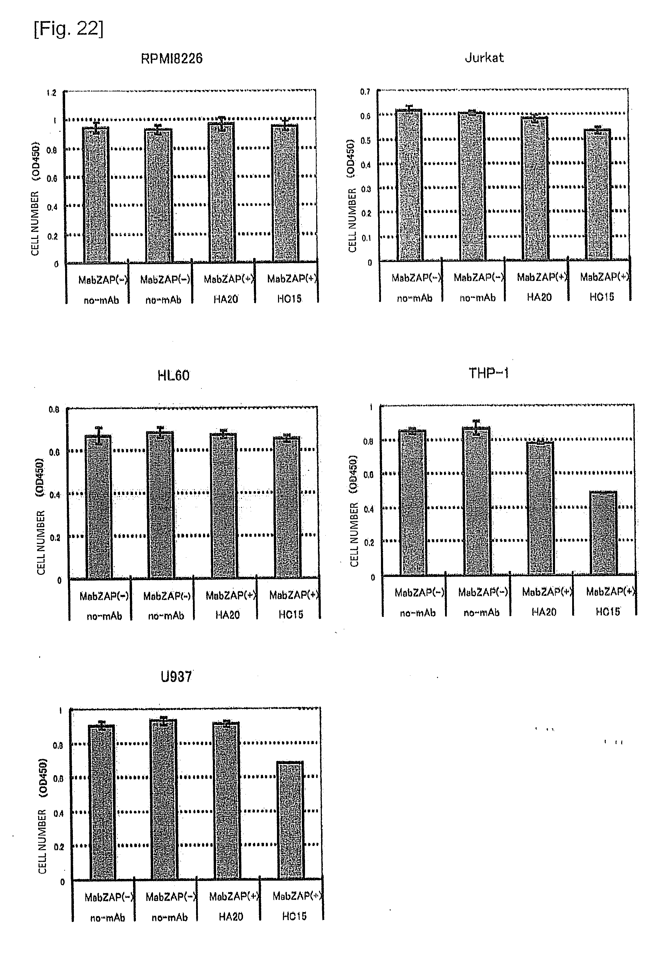

The particular hematological cell line was seeded to 96-well plates at 1 to 2 x $10^4$ cells/well. Mab-ZAP was then reacted, at 100 ng/well, with the particular anti-HB-EGF antibody (100 ng/well) and added to the cells. Five days after antibody addition, the viable cell count was measured using WST-8. An inhibition of proliferation was seen for the simultaneous addition of HC-15 antibody and Mab-ZAP to U937 cells and THP-1 cells (Figure 22). Based on these results, the internalization activity of the HC-15 antibody was shown to be effective as an antitumor agent against several hematological cancers.

Analysis of cell death induction by saporin-labeled antibodies

4-1. Saporin labeling of the antibodies

Cytotoxicity mediated by the antibody's internalization activity was investigated using HA-20 antibody directly labeled with toxin (HA-SAP) and HC-15 antibody directly labeled with toxin (HC-SAP).

Saporin labeling of the purified HA-20 antibody and purified HC-15 antibody was outsourced to Advanced Targeting Systems. Antibodies were thus obtained consisting of HA-20 labeled with an average of 3 saporin molecules and HC-15 labeled with an average of 2.4 saporin molecules (respectively designated HA-SAP and HC-SAP). These were employed in an investigation of the ability to induce cell death in cancer cells.

4-2. Analysis of the cytotoxicity of saporin-labeled antibodies

4-2-1. Analysis of the cytotoxicity of saporin-labeled antibodies for solid cancer cell lines

The following cancer cells were used in the analysis: ES-2, MCAS (ovarian cancer), Capan-2 (pancreatic cancer, purchased from the Japan Health Sciences Foundation), BxPC-3, 22Rvl (prostate cancer, purchased from the ATCC), and HUVEC (human endothelial cells, purchased from Takara Bio Inc.). These cells were in each case cultured using the culture conditions indicated in the instructions supplied by the vendor.

The cytotoxicity was analyzed as follows. Each of the cell lines was seeded to 96-well plates at 1 to 5 x $10^3$ cells/well and cultured overnight. The next day, HA-SAP, HC-SAP, or the control antibody (saporin-labeled mouse IgG (IgG-SAP), Advanced Targeting Systems) was added so as to provide from approximately 100 nM to 1 fM and cultured for 3 to 5 days. The viable cell count was measured using WST-8.

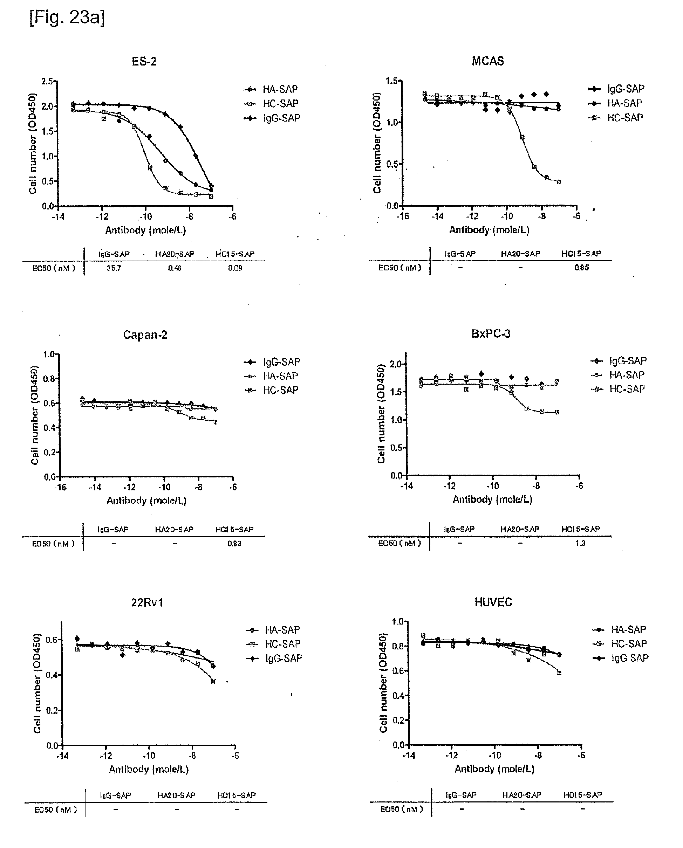

According to the results shown in Figure 23a, HC-SAP strongly induced cell death in ES-2 and MCAS, which are ovarian cancer cell lines. The HC-SAP activity was as follows: $EC_{50}$ = 0.09 nM in ES-2 cells, $EC_{50}$ = 0.86 nM in MCAS cells. On the other hand, no effect at all was shown against HUVEC (normal human endothelial cells).

4-2-2. Analysis of the cytotoxicity exhibited by saporin-labeled antibodies on hematological cancer cell lines

The following cell lines were cultured on RPMI1640 (Invitrogen Corporation) containing 10% FCS: RPMI8226 (multiple myeloma, purchased from the ATCC), HL-60 (acute myeloid leukemia, purchased from JCRB), SKM-1 and THP-1 (acute monocytic leukemia, purchased from JCRB), and U937 (monocytic leukemia, purchased from JCRB).

The cytotoxicity exhibited by HA-SAP and HC-SAP on these hematological cancer cell lines was examined as follows. Each of the cell lines was seeded to 96-well plates at 1 to 5 ξ $10^3$ cells/well followed by the addition of HA-SAP or HC-SAP at from approximately 100 nM to 1 fM and cultivation for 3 to 5 days. The viable cell count was then measured using WST-8.

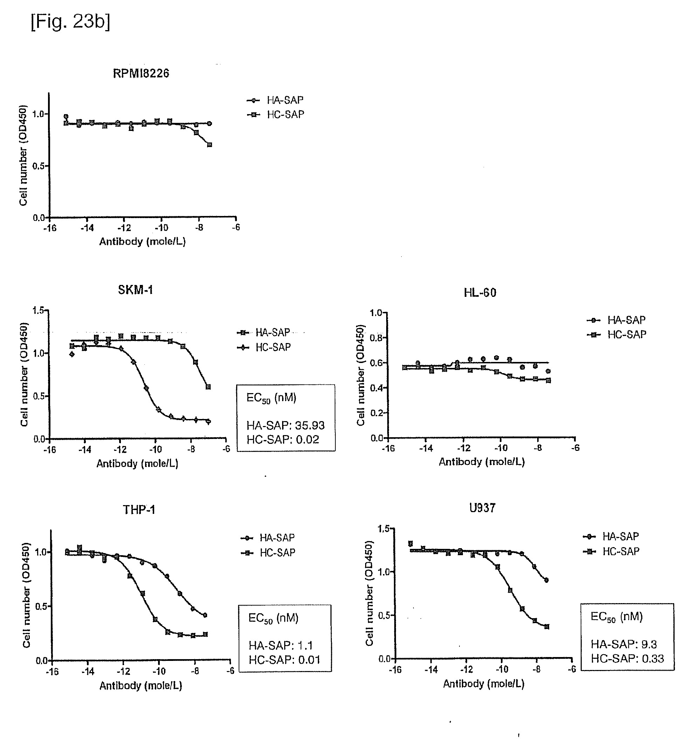

According to the results shown in Figure 23b, cell death was substantially induced by HC-SAP in the U937, SKM-1, and THP-1 cells. The HC-SAP activity was as follows: $EC_{50}$ = 0.33 nM for U937 cells, $EC_{50}$ = 0.02 nM for SKM-1 cells, and $EC_{50}$ = 0.01 nM for THP-1 cells. These results showed that an antibody labeled with, for example, toxin, and targeted to HB-EGF was also effective on hematological cancers.

Analysis of the ADCC activity mediated by the anti-HB-EGF antibodies

10-1. Analysis of the binding activity exhibited by the individual antibodies on membrane-expressed HB-EGF

The binding activity for membrane-expressed HB-EGF was compared, via FACS analysis, for the antibodies obtained so far. The particular antibody (10 μg/mL) was reacted for 1 hour at 4°C with HB-EGF_Ba/F3 cells (Ba/F3 cells that overexpress HB-EGF), followed by staining with FITC-labeled anti-mouse IgG antibody (Beckman Coulter, PN IM0819). Binding by the particular antibody to the cell surface HB-EGF was analyzed by FACS (Becton, Dickinson and Company).

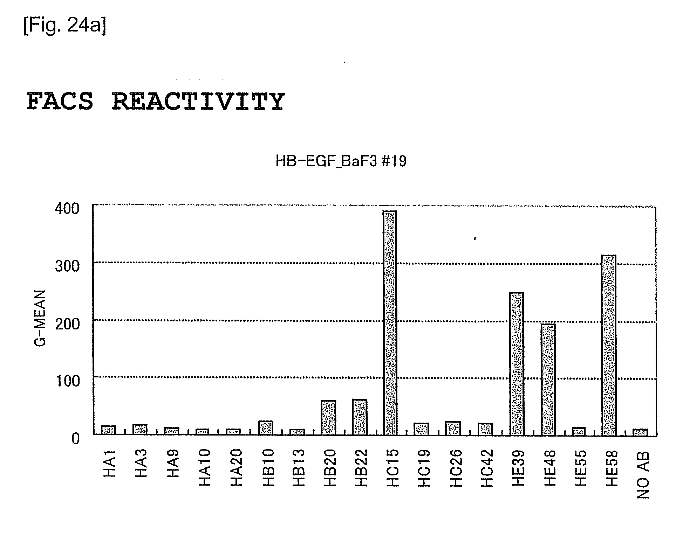

The binding activity for HB-EGF_Ba/F3 measured by FACS analysis for each antibody is represented graphically in Figure 24a. The G-mean value (GEO-mean) on the vertical axis is a value obtained by converting the antibody-induced fluorescence intensity of the cells into numerical values. The results of the analysis showed that HC-15 was the antibody with the strongest binding activity to HB-EGF on the cell membrane, and that HE-39, HE-48, and HE-58 also had strong binding activities.

10-2. Analysis of the antibody-dependent cellular cytotoxicity (ADCC) of the anti-HB-EGF antibodies

The ADCC activity on HB-EGF_Ba/F3 cells was analyzed using the chromium release method and the antibodies (HB-10, HB-20, HB-22, HC-15, HE-39, HE-48, HE-58) that had exhibited binding activity to HB-EGF_Ba/F3 cells.

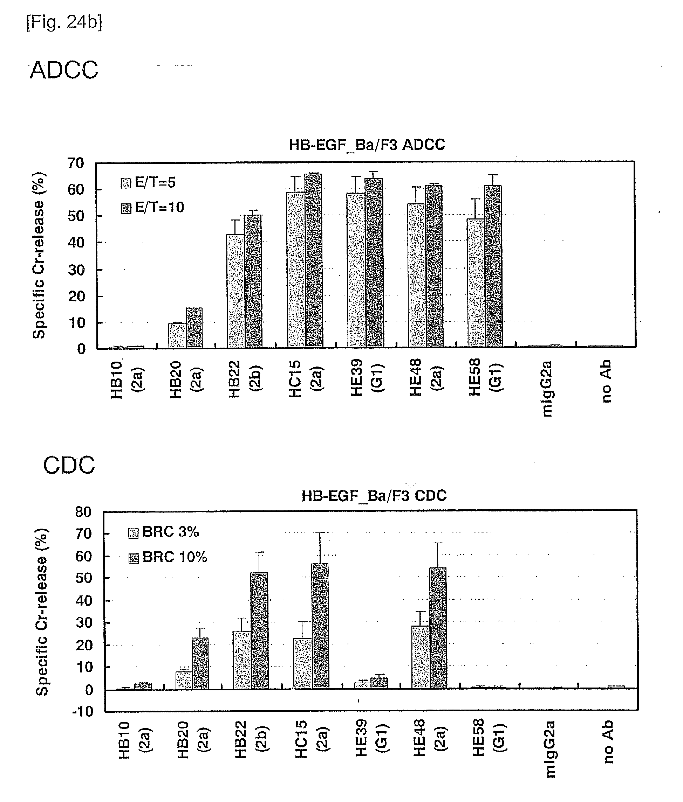

Chromium-51 was added to HB-EGF_Ba/F3 cells propagated in 96-well plates and cultivation was continued for several hours. The culture medium was removed; the cells were washed with culture medium; and fresh culture medium was then added. The antibody was added to give a final concentration of 10 μg/mL; effector cells (recombinant cells obtained by inducing the forced expression of the mouse Fc-gamma receptor (NM_010188) in NK-92 (ATCC, CRL-2407)) were also added to each well at approximately 5X or 10X with reference to the target cells; and the plates were allowed to stand for 4 hours at 37°C in a 5% $CO_2$ incubator. After standing, the plates were centrifuged and a constant amount of supernatant was recovered from each well; the radioactivity was measured using a Wallac 1480 gamma counter; and the specific chromium release (%) was determined. According to the results, which are shown in the upper graph in Figure 24b, HB-22, HC-15, HE-39, HE-48, and HE-58 in particular induced a very strong ADCC activity among the anti-HB-EGF monoclonal antibodies used in the test. These results are results that show that anti-tumor antibody therapy targeted to HB-EGF is very useful.

The specific chromium release rate was calculated using the following formula.

```
specific chromium release (%) = (A-C) x 100/(B-C)
```

where: A is the radioactivity in a particular well; B is the average value of the radioactivity released into the medium by cell lysis with Nonidet P-40 at a final concentration of 1%; and C is the average value of the radioactivity for the addition of only medium.

10-3. Measurement of the complement-dependent cytotoxicity (CDC) of the anti-HB-EGF antibodies

HB-EGF_Ba/F3 cells were recovered by centrifugal separation (1000 rpm, 5 minutes, 4°C); the cell pellet was suspended in approximately 200 μL medium and 3.7 MBq chromium-51 (Code No. CJS4, Amersham Pharmacia Biotech); and cultured for 1 hour at 37°C in a 5% $CO_2$ incubator. The cells were then washed three times with medium; the cell density was subsequently adjusted to 1 x $10^4$/mL with medium; and 100 μL was added to each well in 96-well flat-bottom plates.

The anti-HB-EGF monoclonal antibody (HB-10, HB-20, HB-22, HC-15, HE-39, HE-48, HE-58) and the control mouse IgG2a antibody (Cat. No. 553453, BD Biosciences Pharmingen) were diluted with medium and then added at 50 μL per well. The antibody was adjusted to a final concentration of 10 μg/mL. Baby rabbit complement (Cat. No. CL3441, Cedarlane) was then added to each plate well so as to provide a concentration of 3% or 10%, followed by standing for 1.5 hours at 37°C in a 5% $CO_2$ incubator. After standing, the plates were centrifuged (1000 rpm, 5 minutes, 4°C); 100 μL supernatant was recovered from each well; and the radioactivity in the recovered supernatant was measured with a gamma counter (1480 WIZARD 3", Wallace) .

According to the results as shown in the lower graph in Figure 24b, HB-20, HB-22, HC-15, and HE-48 exhibited a CDC activity among the anti-HB-EGF monoclonal antibodies used in this test. On the other, the mouse IgG2a antibody used as a control did not exhibit CDC activity at the same concentration.

Preparation and Characteristics of Human Chimeric Antibodies from HB-EGF-Neutralizing Antibodies (HC15 and HE39)

11-1. Preparation of Human Chimeric Antibodies from HC15 and HE39

A human chimeric antibody (chHC15) derived from HC15 antibody was prepared as follows. A gene fragment comprising a polynucleotide (SEQ ID NO:7) coding for the H chain variable region (SEQ ID NO:8) of mouse HC15 antibody linked to a polynucleotide (SEQ ID NO:39) coding for the H chain constant region (SEQ ID NO:38) of human IgG1 was introduced into an animal cell expression vector. Also, a gene fragment comprising a polynucleotide (SEQ ID NO:9) coding for the L chain variable region (SEQ ID NO:10) of mouse HC15 antibody linked to a polynucleotide (SEQ ID NO:41) coding for the L chain constant region (SEQ ID NO:40) of human IgG1 was introduced into an animal cell expression vector.

The human chimeric antibody (chHE39) derived from HE39 antibody was obtained as follows. Preparation, characteristics and the amino acid sequence of HE39 antibody are described in WO 2008/047925. A gene fragment comprising a polynucleotide (SEQ ID NO:43) coding for the H chain variable region (SEQ ID NO:42) of mouse HE39 antibody linked to a polynucleotide (SEQ ID NO:39) coding for the H chain constant region (SEQ ID NO:38) of human IgG1 was introduced into an animal cell expression vector. Also, a gene fragment comprising a polynucleotide (SEQ ID NO:45) coding for the L chain variable region (SEQ ID NO:44) of mouse HE39 antibody linked to a polynucleotide (SEQ ID NO:41) coding for the L chain constant region (SEQ ID NO:40) of human IgG1 was introduced into an animal cell expression vector. The nucleotide sequence of the resulting expression vector was determined by a method known to those skilled in the art.

11-2. Expression and Purification of chHC15 and chHE39

Antibody expression and purification were carried out by the following method. The respective antibody expression vectors cleaved with pvuI were introduced in an amount of 15 μg by electroporation into a DG44 cell line (Invitrogen). The transformed DG44 cells were seeded onto 96-well plates. The cells were then cultured in a CHO-S-SFM-II (Invitrogen) medium containing 500 μg/mL of G418 (Invitrogen). Antibody production in the culture supernatants from those wells exhibiting resistance to G418 were detected by ELISA. The clones that were found to produce antibody were expanded, and culture supernatants of the recovered clones were subjected to antibody purification. The cells were removed by centrifugation (approx. 2,000g, 5 min, room temperature) and the culture supernatant was filtered through a 0.22 μm filter (MILLEX(R)-GV (MILLIPORE)). Using a Hi Trap Protein G HP column (Amersham Biosciences #17-0404-01), the antibody was purified from the culture supernatant by a method known to those skilled in the art. The purified antibody solution was additionally filtered through a 0.22 μm filter (MILLIPORE #SLGV033RS), and used in subsequent analysis.

11-3. Comparison of chHC15 and chHE39 Binding Activities to sHB-EGF

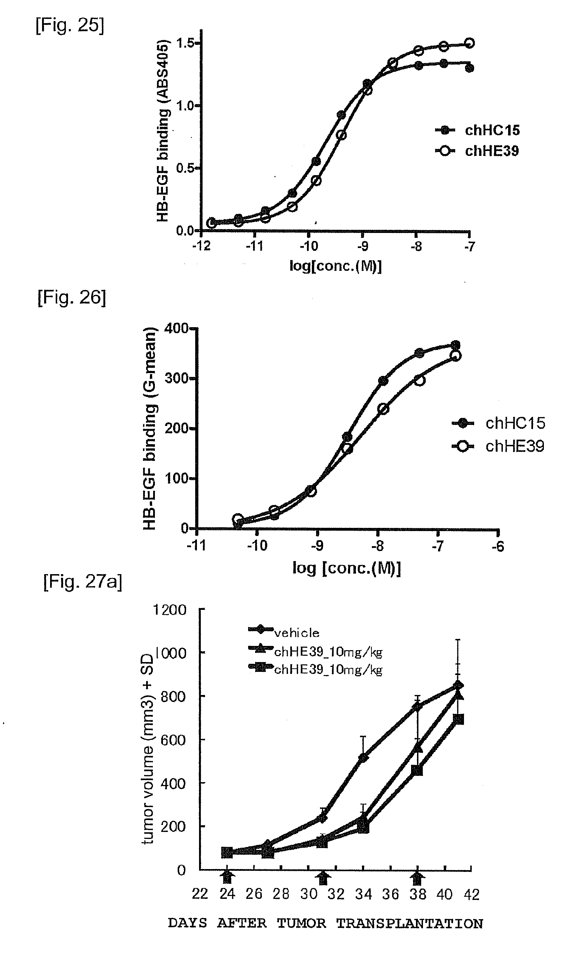

The binding activities of the chimeric antibodies chHC15 and chHE39 prepared as above to active HB-EGF protein were analyzed by the following experiment. The antibodies chHC15 and chHE39 were reacted at various concentrations in individual wells on an ELISA plate (NUNC) coated with HB-EGF protein (R&D, 259-HE) at a concentration of 1 $\mu$g/mL. Next, the plate was reacted with alkali phosphatase (AP)-labeled anti-human IgG for 1 hour, and then 1 mg/mL of substrate was added. Color development from the substrate was quantified by measuring the OD405 absorbance using a plate reader. Based on the binding curve of each of the antibodies, the antibody concentration which exhibits 50% binding ($ED_{50}$) was calculated. The binding activities of chHC15 and chHE39 to active HB-EGF, expressed as $ED_{50}$ values, were found to be about 0.21 nM and 0.42 nM, respectively (Figure 25).

11-4. Analysis of Binding Activities of chHC15 and chHE39 to HB-EGF Expressed on Cell Membrane

Next, the binding activities of the chimeric antibodies chHC15 and chHE39 prepared as above to the HB-EGF expressed on the cell membrane were analyzed by the following experiment.
HB-EGF-expressing DG44 cells (HB-EGF_DG44) and various concentrations of chHC15 or chHE39 were reacted at 4°C for 1 hour. Next, the cells were labeled with FITC-labeled anti-human IgG antibody. The binding of chHC15 and chHE39 to the HB-EGF expressed on the cell membrane was analyzed using FACS (Becton Dickinson).
The labeling intensity (Geo-Mean value) at the respective concentrations of chHC15 and chHE39 were plotted on a graph, and the antibody concentration which exhibits 50% binding ($ED_{50}$) was calculated. The antibodies chHC15 and chHE39 were shown to have the binding activity to HB-EGF with the $ED_{50}$ values of about 3.3 nM and 5.3 nM, respectively (Figure 26).
The binding activities of chHC15 and chHE39 to HB-EGF are summarized in Table 4.

Table 4. Binding activities of chHC15 and chHE39 to HB-EGF (EC50 values)

|  | ELISA (EC50, nmol/L) | FACS (EC50, nmol/L) |
| --- | --- | --- |
| chHC15 | 0.21 | 3.3 |
| chHE39 | 0.42 | 5.3 |

11-5. Comparison of in vivo Antitumor Activities of chHC15 and chHE39

The HB-EGF-high expressing SKOV3 cell line or ovarian cancer cell line (MCAS) was suspended in HBSS (SIGMA H9269) to prepare a cell suspension (0.2 mL) of $5 \times 10^7$ cells. The suspension was transplanted subcutaneously in the ventral area of SCID mice (male, 7 week old (C.B-17/lcr-scid Jcl, CLEA Japan, Inc.). After the tumors have been engrafted, a plurality of the nude mice were divided into five groups based on the tumor volume and weight as the indicators (one control group and four dosing groups for each cell line).
Starting from the day on which the animals were divided into groups, the control group was given PBS (Dulbecco's PBS GIBCO 14190), and the dosing groups were given chHC15 or chHE39, with the doses of 10 mg/kg or 50 mg/kg for the groups to which the HB-EGF-high expressing SKOV3 cell line had been transplanted, and 2 mg/kg or 10 mg/kg for the groups to which the ovarian cancer cell line (MCAS) had been transplanted. In each case, administration was carried instantaneously via the caudal vein every other week, and the tumor volume was measured over time. The therapeutic effects of the antibody were evaluated by measurement of the tumor volume.

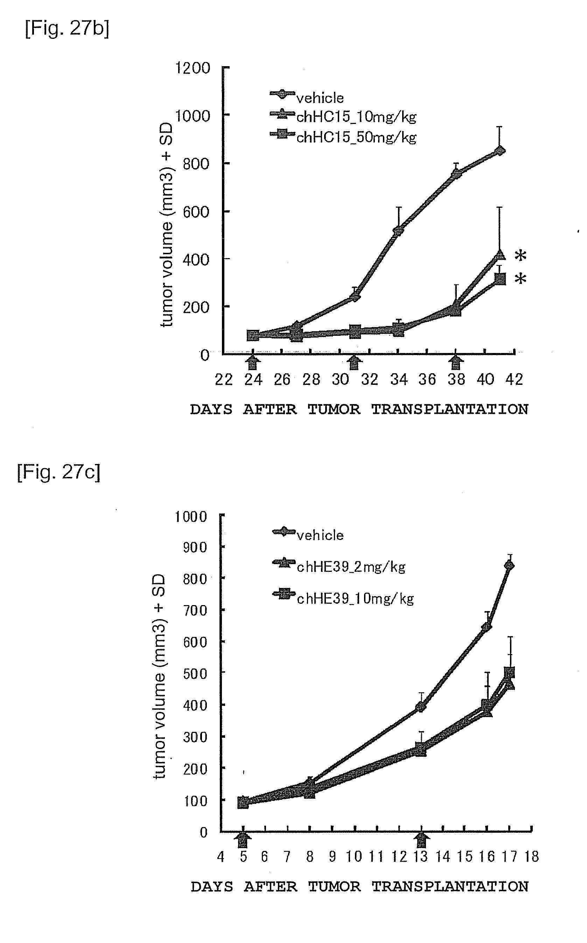

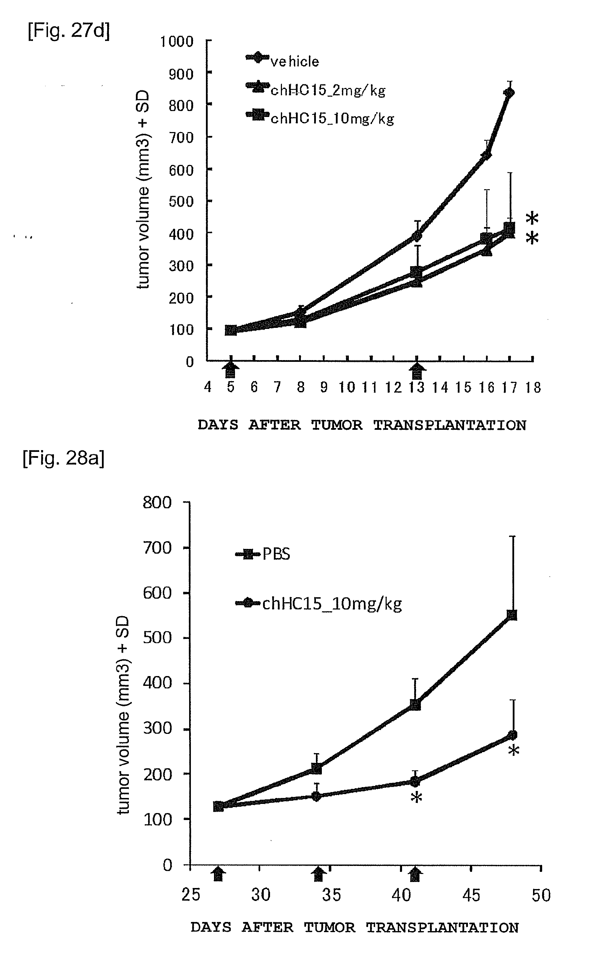

Figures 27a and 27b show the results of a HB-EGF-high-expressing SKOV-3 cell line transplantation model, and Figures 27c and 27d shows the results of a MCAS transplantation mouse model. In both the HB-EGF-high expressing SKOV-3 line and the MCAS transplantation models, the tumor volume of the chHC15 dosing groups was suppressed compared with the tumor volume of the chHE39 dosing groups. That is, chHC15 exhibited a stronger antitumor activity than chHE39. The tumor volume data were analyzed by non-parametric Dunnett multiple comparison. Administration of chHC15 was shown to exhibit a significant tumor growth suppressing effect in both the HB-EGF-high expressing SKOV-3 line transplanted mice and the MCAS transplanted mice.

11-6. Analysis of chHC15 in vivo Antitumor Activities in Other Cancer Cell Lines

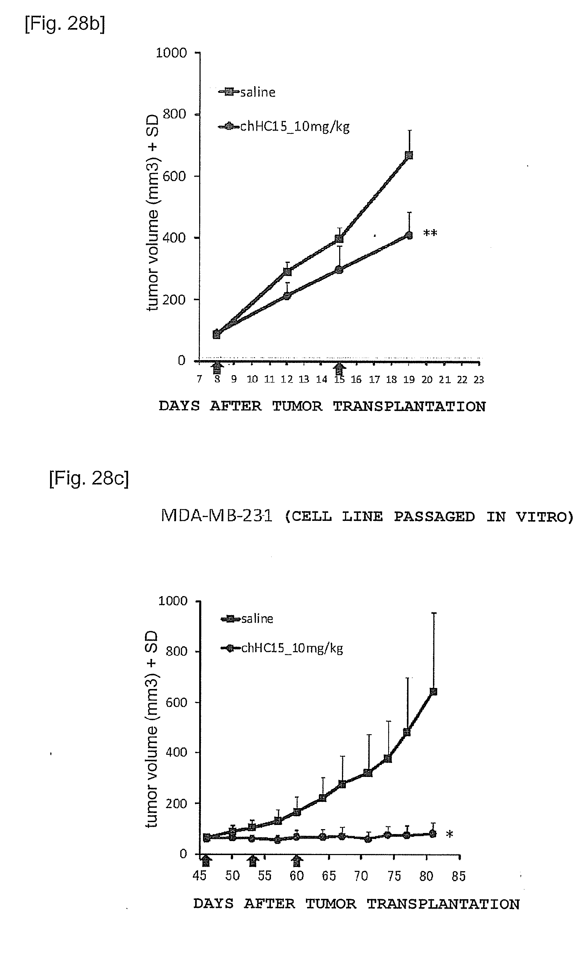

The antitumor activities of chHC15 was analyzed using transplantation mouse models of pancreatic cancer cell line BxPC-3, breast cancer cell line MDA-MB-231 (maintained in vitro) and breast cancer cell line MDA-MB-231 (maintained in vivo).
In general accordance with the method described above in section 11-5, the various cancer cell lines were transplanted into mice, and the mice were divided into groups. Mice in the drug dosing groups were administered with a dose of 10 mg/kg chHC15 once per week. Figure 28a shows the evaluation of drug efficacy of chHC15 in the BxPC-3 line transplantation model, and Figures 28b and 28c show the evaluation of chHC15 drug efficacy in the respective MDA-MB-231 (maintained in vitro) and MDA-MB-231 (maintained in vivo) transplantation models. The data were analyzed by non-parametric Dunnett multiple comparison. Administration of the HC15 human chimeric antibody in a dose of 10 mg/kg exhibited significant tumor growth-suppressing effects in all of the transplantation models.

INDUSTRIAL APPLICABILITY

The antibody of the present invention and the pharmaceutical composition comprising the antibody of the invention are useful for the treatment and diagnosis of cancer.

**Claims**

1. An anti-HB-EGF antibody selected from [1] and [2]:

   [1] an antibody comprising
   a heavy chain comprising the amino acid sequence of SEQ ID N0:1 as CDR1, the amino acid sequence of SEQ ID NO:2 as CDR2, and the amino acid sequence of SEQ ID NO:3 as CDR3, and
   a light chain comprising the amino acid sequence of SEQ ID NO:4 as CDR1, the amino acid sequence of SEQ ID NO:5 as CDR2, and the amino acid sequence of SEQ ID NO:6 as CDR3; and
   [2] an antibody which binds to the same epitope as an epitope to which the antibody set forth in [1] binds.

2. The anti-HB-EGF antibody according to claim 1 having an internalizing activity.

3. The anti-HB-EGF antibody according to claim 1 or 2 to which a cytotoxic substance is attached.

4. The anti-HB-EGF antibody according to claim 1 or 2 having an ADCC activity or a CDC activity.

5. The anti-HB-EGF antibody according to any one of claims 1 to 4, further having a neutralizing activity.

6. The anti-HB-EGF antibody according to any one of claims 1 to 5, which comprises a marker attached.

7. A pharmaceutical composition comprising the antibody according to any one of claims 1 to 6.

8. A pharmaceutical composition comprising a cytotoxic substance attached to the antibody according to any one of claims 1 to 6.

9. The pharmaceutical composition according to claim 7 or 8, which is a cell proliferation inhibitor.

10. The pharmaceutical composition according to claim 9, which is an anti-cancer agent.

11. The pharmaceutical composition according to claim 10, wherein the cancer is pancreatic cancer, liver cancer, esophageal cancer, melanoma, colorectal cancer, gastric cancer, ovarian cancer, uterine cervical cancer, breast cancer, bladder cancer, a brain tumor, or a hematological cancer.

12. A method of delivering a cytotoxic substance into a cell by means of the anti-HB-EGF antibody according to any one of claims 1 to 6.

13. A method of inhibiting cell proliferation with a cytotoxic substance attached to the anti-HB-EGF antibody according to any one of claims 1 to 6.

14. The method according to claim 13, wherein the cell is a cancer cell.

15. The method according to any one of claims 12 to 14, wherein the cytotoxic substance is a chemotherapeutic agent, a radioactive substance, or a toxic peptide.

16. Use of the anti-HB-EGF antibody according to any one of claims 1 to 6 for transporting a cytotoxic substance into a cell.

17. Use of the anti-HB-EGF antibody according to any one of claims 2 to 6 having an internalizing activity for inhibiting cell proliferation.

18. The use according to claim 16 or 17, wherein the cell is a cancer cell.

19. The use according to any one of claims 16 to 18, wherein a cytotoxic substance is attached to the anti-HB-EGF antibody.

20. A method of producing a pharmaceutical composition comprising the steps of:

(a) providing the anti-HB-EGF antibody according to any one of claims 1 to 6;
(b) determining whether the antibody of (a) has an internalizing activity;
(c) selecting an antibody that has an internalizing activity; and
(d) attaching a cytotoxic substance to the antibody selected in (c).

21. The method according to claim 20, wherein the pharmaceutical composition is an anti-cancer agent.

22. A method of diagnosing cancer using the anti-HB-EGF antibody according to any one of claims 1 to 6.

23. The method according to claim 22, comprising using an anti-HB-EGF antibody to which a labeling substance is attached.

24. The method according to claim 22 or 23, wherein an intracellularly incorporated anti-HB-EGF antibody is detected.

[Fig. 1]

MEMBRANE-ANCHORED HB-EGF     SECRETORY (ACTIVE) HB-EGF     HB-EGF_Fc (FOR IMMUNIZATION)

[Fig. 2a]

EGFR_BaF3

[Fig. 2b]

HB-EGF dependency

[Fig. 3]

HB-EGF NEUTRALIZING ACTIVITY (HC SERIES)

[Fig. 4]

HEAVY-CHAIN
VARIABLE
REGION

VH Segments

```
FR1                                          CDR1   FR2              CDR2
--------1---------2----------3 ----- ----4--------- 5---------6-----
12345678901234567890123456789 0 12345AB 67890123456789 012A345678901234S
```

HC15    EVQLQQSGPELVKPGASVKISCKAS GYSFT GYYIH WVKQSPEKRLE WIG EIHPRTGITTYNQKFKA

JH Segments

```
FR3                                          CDR3
---7---------8-----------9---- ----10----------- -----11----
67890123456789012abc345678901234 567890ABCDEFGHIJK12 35678901234
```

HC15    KATLTVDKSSSTAYMQLKSLTSEDSAVYYCAR VGSSGPFTY----------- WGQGTLVTVSA

LIGHT-CHAIN
VARIABLE
REGION

VK Segments

```
FR1                             CDR1        FR2          CDR2
--------1---------2--- ------3--------- ----4--------- 5------
12345678901234567890123 45678901ABCDE234 567890123456789 0123456
```

HC15    DIVMTQSPSSLSVSAGDKVTMSC KSSQSLLHSRHQKNYLA WYQQKPWQPPKLLIY GASTRES

JK Segments

```
FR3                             CDR3        FR4
---6---------7----------8-------- -9------------- ---10-----
78901234567890123456789012345678 9012345ABCDEFG7 8901234567
```

HC15    GVPDRFTGSGSGTDFTLTISSVQAEDLAVYYC QNDYSYPFT FGTGTKLEIK

| ANTIBODY | CHAIN | VARIABLE REGIONS | BASE SEQUENCE (SEQ ID NO) | AMINO ACID SEQUENCE (SEQ ID NO) |
|---|---|---|---|---|
| HC15 | H CHAIN | FULL-LENGTH (aa's 1 TO 19 ARE SIGNAL PEPTIDE) | 7 | 8 |
| | | CDR1 | | 1 |
| | | CDR2 | | 2 |
| | | CDR3 | | 3 |
| HC15 | L CHAIN | FULL-LENGTH (aa's 1 TO 20 ARE SIGNAL PEPTIDE) | 9 | 10 |
| | | CDR1 | | 4 |
| | | CDR2 | | 5 |
| | | CDR3 | | 6 |

[Fig. 5]

[Fig. 6]

[Fig. 7]

[Fig. 8]

[Fig. 9]

[Fig. 10]

[Fig. 11]

[Fig. 12a]

[Fig. 12b]

[Fig. 13]

mabZAP (anti-mIgG-saporin)     Targeting mAb

internalize

Saporin release

apoptosis

[Fig. 14]

HB-EGF mAb effect on SKOV3

HB-EGF mAb effect on HB-EGF_SKOV3

[Fig. 15]

HA-20

no 1st ab
— HA-20

Events
128
0
$10^0$  $10^1$  $10^2$  $10^3$  $10^4$
FL1-H

HB-20

no 1st ab
— HB-20

Events
128
0
$10^0$  $10^1$  $10^2$  $10^3$  $10^4$
FL1-H

HC-15

no 1st ab
— HC-15

Events
128
0
$10^0$  $10^1$  $10^2$  $10^3$  $10^4$
FL1-H

[Fig. 16]

HB-EGF mAb effect on ES-2

| mAb | – | – | HA20 | HB20 | HC15 |
| Mab-ZAP | – | + | + | + | + |

[Fig. 17]

RMG-1

no-1st mAb
— HC-15

Events
64
0
$10^0$  $10^1$  $10^2$  $10^3$  $10^4$
FL1-H

MCAS

no-1st mAb
— HC-15

Events
128
0
$10^0$  $10^1$  $10^2$  $10^3$  $10^4$
FL1-H

[Fig. 18]

|          |                      |
|----------|----------------------|
| no-mAb   | HA-20 (50 μg/ml)     |
| HC-15 (50 μg/ml) |              |

[Fig. 19]

|          |                                 |
|----------|---------------------------------|
| MabZAP   | HA-20 (10 μg/ml) + MabZAP       |
| HC-15 (10 μg/ml) + MabZAP |            |

[Fig. 20]

|  MabZAP | HA-20 (10 μg/ml)<br>+<br>MabZAP |
| HC-15 (10 μg/ml)<br>+<br>MabZAP |  |

[Fig. 21]

[Fig. 22]

RPMI8226

Jurkat

HL60

THP-1

U937

[Fig. 23a]

**ES-2**

| | IgG-SAP | HA20-SAP | HC15-SAP |
|---|---|---|---|
| EC50 (nM) | 35.7 | 0.48 | 0.09 |

**MCAS**

| | IgG-SAP | HA20-SAP | HC15-SAP |
|---|---|---|---|
| EC50 (nM) | – | – | 0.86 |

**Capan-2**

| | IgG-SAP | HA20-SAP | HC15-SAP |
|---|---|---|---|
| EC50 (nM) | – | – | 0.83 |

**BxPC-3**

| | IgG-SAP | HA20-SAP | HC15-SAP |
|---|---|---|---|
| EC50 (nM) | – | – | 1.3 |

**22Rv1**

| | IgG-SAP | HA20-SAP | HC15-SAP |
|---|---|---|---|
| EC50 (nM) | – | – | – |

**HUVEC**

| | IgG-SAP | HA20-SAP | HC15-SAP |
|---|---|---|---|
| EC50 (nM) | – | – | – |

[Fig. 23b]

[Fig. 24a]

# FACS REACTIVITY

HB-EGF_BaF3 #19

[Fig. 24b]

## ADCC

**HB-EGF_Ba/F3 ADCC**

## CDC

**HB-EGF_Ba/F3 CDC**

[Fig. 25]

[Fig. 26]

[Fig. 27a]

[Fig. 27b]

[Fig. 27c]

[Fig. 27d]

DAYS AFTER TUMOR TRANSPLANTATION

[Fig. 28a]

DAYS AFTER TUMOR TRANSPLANTATION

[Fig. 28b]

[Fig. 28c]

MDA-MB-231 (CELL LINE PASSAGED IN VITRO)

## INTERNATIONAL SEARCH REPORT

| International application No. |
|---|
| PCT/JP2010/005074 |

A. CLASSIFICATION OF SUBJECT MATTER
*C07K16/22*(2006.01)i, *A61K39/395*(2006.01)i, *A61P35/00*(2006.01)i, *C12Q1/02* (2006.01)i

According to International Patent Classification (IPC) or to both national classification and IPC

B. FIELDS SEARCHED

Minimum documentation searched (classification system followed by classification symbols)
C07K16/22, A61K39/395, A61P35/00, C12Q1/02

Documentation searched other than minimum documentation to the extent that such documents are included in the fields searched

Electronic data base consulted during the international search (name of data base and, where practicable, search terms used)
CA/BIOSIS/MEDLINE/WPIDS(STN), GenBank/EMBL/DDBJ/GeneSeq, UniProt/GeneSeq, JSTPlus(JDreamII)

C. DOCUMENTS CONSIDERED TO BE RELEVANT

| Category* | Citation of document, with indication, where appropriate, of the relevant passages | Relevant to claim No. |
|---|---|---|
| X | WO 2008/047925 A1 (FORERUNNER PHARM RES CO., LTD.), 24 April 2008 (24.04.2008), abstract; claims; paragraphs [0170] to [0295]; fig. 1 to 33, sequence no.46, 45 & EP 2078731 A1 | 1-21 |
| X/Y | WO 2009/072628 A1 (KYOWA HAKKO KIRIN CO., LTD.), 11 June 2009 (11.06.2009), abstract; claims; examples 1 to 10; fig. 2, 9, 10 to 12, 14, 16, 17 & AU 2008332296 A | 1,3-11,13-15 /2,12,16-21 |
| Y | POUL, M.A., et al., Selection of tumor-specific internalizing human antibodies from phage libraries. Journal of Molecular Biology, 2000, Vol.301, pp.1149-1161 | 2,12,16-21 |

☒ Further documents are listed in the continuation of Box C.     ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 03 September, 2010 (03.09.10) | 28 September, 2010 (28.09.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.

PCT/JP2010/005074

C (Continuation). DOCUMENTS CONSIDERED TO BE RELEVANT

| Category* | Citation of document, with indication, where appropriate, of the relevant passages | Relevant to claim No. |
|---|---|---|
| A | WO 2007/142277 A1 (KYOWA HAKKO KIRIN CO., LTD.), 13 December 2007 (13.12.2007), & EP 2039704 A1 | 1-21 |
| A | WO 2008/047914 A1 (FORERUNNER PHARM RES CO., LTD.), 24 April 2008 (24.04.2008), & EP 2093237 A1 | 1-21 |
| A | MIYAMOTO, S., et al., Potential for molecularly targeted therapy against epidermal growth factor receptor ligands. Anticancer Research, Mar. 2009, Vol.29, No.3, pp.823-830 | 1-21 |

Form PCT/ISA/210 (continuation of second sheet) (July 2009)

## INTERNATIONAL SEARCH REPORT

| International application No. |
|---|
| PCT/JP2010/005074 |

| Box No. II | Observations where certain claims were found unsearchable (Continuation of item 2 of first sheet) |
|---|---|

This international search report has not been established in respect of certain claims under Article 17(2)(a) for the following reasons:

1. ☒ Claims Nos.: 22-24
   because they relate to subject matter not required to be searched by this Authority, namely:
   Claims 22 to 24 pertain to diagnostic methods and thus relate to a subject matter on which the International Searching Authority is not required to carry out a search.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

| Box No. III | Observations where unity of invention is lacking (Continuation of item 3 of first sheet) |
|---|---|

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
   ☐ The additional search fees were accompanied by the applicant's protest and, where applicable, the payment of a protest fee.
   ☐ The additional search fees were accompanied by the applicant's protest but the applicable protest fee was not paid within the time limit specified in the invitation.
   ☐ No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2009003915 W
- WO 2008047914 A
- WO 2008047925 A
- WO 03104453 A
- JP H159878 B
- WO 9425585 A
- WO 9312227 A
- WO 9203918 A
- WO 9402602 A
- WO 9411523 A
- EP 239400 A
- WO 9602576 A
- WO 9634096 A
- WO 9633735 A
- WO 9201047 A
- WO 9220791 A
- WO 9306213 A
- WO 9311236 A
- WO 9319172 A
- WO 9501438 A
- WO 9515388 A
- EP 404097 A
- WO 9311161 A
- WO 9954342 A
- WO 0061739 A
- WO 023140 A
- WO 2006067847 A
- WO 2006067913 A
- WO 0279255 A
- WO 0231140 A
- WO 03035835 A
- WO 9813388 A

### Non-patent literature cited in the description

- **Iwamoto R ; Yamazaki S ; Asakura M et al.** Heparin-binding EGF-like growth factor and ErbB signaling is essential for heart function. *Proc. Natl. Acad. Sci. USA,* 2003, vol. 100, 3221-6
- **Abraham JA ; Damm D ; Bajardi A ; Miller J ; Klagsbrun M ; Ezekowitz RA.** Heparin-binding EGF-like growth factor: characterization of rat and mouse cDNA clones, protein domain conservation across species, and transcript expression in tissues. *Biochem Biophys Res Commun,* 1993, vol. 190, 125-33
- **Karen M.** *Frontiers in Bioscience,* 1998, vol. 3, 288-299
- **Raab G ; Klagsbrun M.** Heparin-binding EGF-like growth factor. *Biochim Biophys Acta,* 1997, vol. 1333, F179-99
- **Yamazaki S ; Iwamoto R ; Saeki K et al.** Mice with defects in HB-EGF ectodomain shedding show severe developmental abnormalities. *J Cell Biol,* 2003, vol. 163, 469-75
- **Ongusaha P.** *Cancer Res,* 2004, vol. 64, 5283-5290
- **Iwamoto R. ; Higashiyama S.** *EMBO J.,* 1994, vol. 13, 2322-2330
- **Naglich JG. ; Metherall JE.** *Cell,* 1992, vol. 69, 1051-1061
- **Iwamoto R ; Handa K ; Mekada E.** Contact-dependent growth inhibition and apoptosis of epidermal growth factor (EGF) receptor-expressing cells by the membrane-anchored form of heparin-binding EGF-like growth factor. *J. Biol. Chem.,* 1999, vol. 274, 25906-12
- **Miyamoto S.** *Cancer Sci.,* vol. 97, 341-347
- **Blotnick S.** *Proc. Natl. Acad. Sci. USA,* 1994, vol. 91, 2890-2894
- **Hashimoto K.** *J. Biol. Chem.,* 1994, vol. 269, 20060-20066
- **Mishima K.** *Act Neuropathol.,* 1998, vol. 96, 322-328
- **Wang YD.** *Oncogene,* 2002, vol. 21, 2584-2592
- **Miyamoto S.** *Cancer Res.,* 2004, vol. 64, 5720
- **Buzzi S.** *Cancer Immunol Immunother,* 2004, vol. 53, 1041-1048
- **Langone J.J. et al.** *Methods in Enzymology,* 1983, vol. 93, 307-308
- *Mature Medicine,* 1996, vol. 2, 350-353
- **Fulton R.J. et al.** *J. Biol. Chem.,* vol. 261, 5314-5319
- **Sivam G. et al.** *Cancer Res.,* 1987, vol. 47, 3169-3173
- **Cumber A.J. et al.** *J. Immunol. Methods,* vol. 135, 15-24

- **Wawrzynczak E.J. et al.** *Cancer Res.,* 1990, vol. 50, 7519-7562
- **Gheeite V. et al.** *J. Immunol. Methods,* 1991, vol. 142, 223-230
- **Thorpe P.E. et al.** *Cancer Res.,* 1987, vol. 47, 5924-5931
- **Wawrzynczak E.J. et al.** *Br. J. Cancer,* 1992, vol. 66, 361-366
- **Cumber A.J. et al.** *J. Immunol. Methods,* 1990, vol. 135, 15-24
- **Bolognesi A. et al.** *Clin. Exp.Immunol.,* 1992, vol. 89, 341-346
- **Bolognesi A. et al.** *Clin. Exp. Immunol.,* 1992, vol. 89, 341-346
- **Stirpe F. ; Barbieri L.** *FEBS Letter,* 1986, vol. 195, 1-8
- **Casellas P. et al.** *Eur. J. Biochem.,* 1988, vol. 176, 581-588
- **Rowland G.F. et al.** *Nature,* 1975, vol. 255, 487-488
- **Hurwitz E. ; Haimovich J.** *Method in Enzymology,* 1986, vol. 178, 369-375
- **Schechter B. et al.** *Int. J. Cancer,* 1991, vol. 48, 167-172
- **Ota Y. et al.** *Asia-Oceania J. Obstet. Gynaecol.,* 1993, vol. 19, 449-457
- **Noguchi A. et al.** *Bioconjugate Chem.,* 1992, vol. 3, 132-137
- **Shih L.B. et al.** *Cancer Res.,* 1991, vol. 51, 4192-4198
- **Zhu Z. et al.** *Cancer Immunol. Immunother.,* 1995, vol. 40, 257-267
- **Trail P.A. et al.** *Science,* 1993, vol. 261, 212-215
- **Kondo Y. et al.** *Jpn. J. Cancer Res.,* 1995, vol. 86, 1072-1079
- **Zhu Z. et al.** *Cancer Immunol. Immunother.,* vol. 40, 257-267
- **Dillman R.O. et al.** *Cancer Res.,* 1988, vol. 48, 6097-6102
- **Hudecz F. et al.** *Bioconjugate Chem.,* 1990, vol. 1, 197-204
- **Tukada Y. et al.** *J. Natl. Cancer Inst.,* 1984, vol. 75, 721-729
- **Manabe Y. et al.** *Biochem. Biophys. Res. Commun.,* 1983, vol. 115, 1009-1014
- **Kitamura K. et al.** *Cancer Immunol. Immunother.,* 1993, vol. 36, 177-184
- **Yamaguchi T. et al.** *Jpn. J. Cancer Res.,* 1994, vol. 85, 167-171
- **Kralovec J. et al.** *Cancer Immunol. Immunother.,* 1989, vol. 29, 293-302
- **Kulkarni P.N. et al.** *Cancer Res.,* 1981, vol. 41, 2700-2706
- **Shin L.B. et al.** *Int. J. Cancer,* 1988, vol. 41, 832-839
- **Gamett M.C. et al.** *Int. J. Cancer,* 1983, vol. 31, 661-670
- **Shin L.B.** *Int. J. Cancer,* 1990, vol. 46, 1101-1106
- **Goerlach A. et al.** *Bioconjugate Chem.,* 1991, vol. 2, 96-101
- **Hurwitz E. et al.** *J. Med. Chem.,* 1985, vol. 28, 137-140
- **Kanellos J. et al.** *Immunol. Cell. Biol.,* 1987, vol. 65, 483-493
- **Johnson J.R. et al.** *Br. J. Cancer,* 1980, vol. 42, 17
- **Johnson J.R. et al.** *Br. J. Cancer,* 1981, vol. 44, 472-475
- **Hermanson G.T.** *BIOCONJUGATE Techniques,* 1996, 230-232
- **Hermanson G.T.** *BIOCONJUGATE Techniques,* 1996, 232-235
- **Hermanson G.T.** *BIOCONJUGATE Techniques,* 1996, 242-243
- **Hermanson G.T.** *BIOCONJUGATE Techniques,* 1996, 237-238
- Current Protocols in Immunology. Immunologic Studies in Humans. John Wiley & Sons, Inc, 1993
- Molecular Cloning. **Sambrook, J. et al.** Molecular Cloning. Cold Spring Harbor Laboratory Press, 1989
- *J. Immunol.,* 1979, vol. 123, 1548-1550
- *Current Topics in Microbiology and Immunology,* 1978, vol. 81, 1-7
- **Kohler, G. ; Milstein, C.** *Eur. J. Immunol.,* 1976, vol. 6, 511-519
- **Margulies, D.H. et al.** *Cell,* 1976, vol. 8, 405-415
- **Shulman, M. et al.** *Nature,* 1978, vol. 276, 269-270
- **de St. Groth, S. F. et al.** *J. Immunol. Methods,* 1980, vol. 35, 1-21
- **Trowbridge, I.S.** *J. Exp. Med.,* 1978, vol. 148, 313-323
- **Galfre, G. et al.** *Nature,* 1979, vol. 277, 131-133
- **Kohler, G. ; Milstein, C.** *Methods Enzymol.,* 1981, vol. 73, 3-46
- **Vandamme, A.M. et al.** *Eur. J. Biochem.,* 1990, vol. 192, 767-775
- **Chirgwin, J. M. et al.** *Biochemistry,* 1979, vol. 18, 5294-5299
- **Chomczynski, P. et al.** *Anal. Biochem.,* 1987, vol. 162, 156-159
- **Frohman, M.A. et al.** *Proc. Natl. Acad. Sci. USA,* 1988, vol. 85, 8998-9002
- **Belyavsky, A. et al.** *Nucleic Acids Res.,* 1989, vol. 17, 2919-2932
- *J. Mol. Biol.,* 1991, vol. 222, 581-597
- **Mulligan et al.** *Nature,* 1979, vol. 277, 108
- **Mizushima et al.** *Nucleic Acids Res.,* 1990, vol. 18, 5322
- **Ward et al.** *Nature,* 1989, vol. 341, 544-546
- *FASEBJ.,* 1992, vol. 6, 2422-2427

- **Better et al.** *Science,* 1988, vol. 240, 1041-1043
- **Lei, S.P. et al.** *J. Bacteriol.,* 1987, vol. 169, 4379
- **Ed Harlow ; David Lane.** Antibodies: A Laboratory Manual. Cold Spring Harbor Laboratory, 1988
- **Ebert, K.M. et al.** *Bio/Technology,* 1994, vol. 12, 699-702
- **Sato, K. et al.** *Cancer Res.,* 1993, vol. 53, 851-856
- **Co, M.S. et al.** *J. Immunol.,* 1994, vol. 152, 2968-2976
- **Better, M. ; Horwitz, A.H.** *Methods in Enzymology,* 1989, vol. 178, 476-496
- **Plueckthun, A. ; Skerra, A.** *Methods in Enzymology,* 1989, vol. 178, 476-496
- **Lamoyi, E.** *Methods in Enzymology,* 1989, vol. 121, 652-663
- **Rousseaux, J. et al.** *Methods in Enzymology,* 1989, vol. 121, 663-669
- **Bird, R.E. et al.** *TIBTECH,* 1991, vol. 9, 132-137
- **Holliger, P. et al.** *Proc. Natl. Acad. Sci. USA,* 1993, vol. 90, 6444-6448
- **Huston, J.S. et al.** *Proc. Natl. Acad. Sci. USA,* 1988, vol. 85, 5879-5883
- **Hudson et al.** *J. Immunol. Methods,* 1999, vol. 231, 177-189
- *Protein Engineering,* 1996, vol. 9 (3), 299-305
- **Better, M. ; Horwitz, A.H.** *Methods Enzymol.,* 1989, vol. 178, 476-496
- **Plueckthun, A. ; Skerra, A.** *Methods Enzymol.,* 1989, vol. 178, 497-515
- **Lamoyi, E.** *Methods Enzymol.,* 1986, vol. 121, 652-663
- **Rousseaux, J. et al.** *Methods Enzymol.,* 1986, vol. 121, 663-669
- **Bird, R.E. ; Walker, B.W.** *Trends Biotechnol.,* 1991, vol. 9, 132-137
- **Shimizu Y. et al.** *Carbohydrate Research,* 2001, vol. 332, 381-388
- **Kondo A. et al.** *Agricultural and Biological Chemistry,* 1990, vol. 54 (8), 2169-2170
- **Hashimoto-Gotoh, T. et al.** *Gene,* 1995, vol. 152, 271-275
- **Zoller, M.J. ; Smith, M.** *Methods Enzymol.,* 1983, vol. 100, 468-500
- **Kramer, W. et al.** *Nucleic Acids Res.,* 1984, vol. 12, 9441-9456
- **Kramer, W. ; Fritz, H.J.** *Methods Enzymol.,* 1987, vol. 154, 350-367
- **Kunkel, T.A.** *Proc. Natl. Acad. Sci. USA,* 1985, vol. 82, 488-492
- **Kunkel.** *Methods Enzymol.,* 1988, vol. 85, 2763-2766
- **Mark, D.F. et al.** *Proc. Natl. Acad. Sci. USA,* 1984, vol. 81, 5662-5666
- **Zoller, M.J. ; Smith, M.** *Nucleic Acids Research,* 1982, vol. 10, 6487-6500
- **Wang, A. et al.** *Science,* vol. 224, 1431-1433
- **Dalbadie-McFarland, G. et al.** *Proc. Natl. Acad. Sci. USA,* 1982, vol. 79, 6409-6413
- Antibodies: A Laboratory Manual. 359-420
- Remington's Pharmaceutical Science. Mack Publishing Company
- **Mizushima S. et al.** *Nuc. Acids Res.,* 1990, vol. 18, 5322